# EUROPEAN PATENT APPLICATION

(11) **EP 1 889 632 A1**
(43) Date of publication of application: **20.02.2008**
(21) Application number: 06745533.7
(22) Date of filing: 21.04.2006
(51) Int. Cl.: A61K 45/00

(54) **REMEDY FOR XANTHOMA**

(30) Priority: 22.04.2005 JP 2005124781
(71) Applicant: National University Corp. Kobe University, Kobe-shi, Hyogo 6578501 (JP); Takeda Pharmaceutical Company Limited, Osaka-shi, Osaka 541-0045 (JP)
(72) Inventor: SHIOMI, Masashi,Nat. Uni. Corp. Kobe University, Kobe-shi, Hyogo 657-8501 (JP); ITO, Takashi,Nat. Uni. Corp. Kobe University, Kobe-shi, Hyogo 657-8501 (JP); TOZAWA, Ryuichi Takeda Pharmaceutical Co., Ltd., Osaka-shi, Osaka 532-8686 (JP); AMANO, Yuichiro Takeda Pharmaceutical Co., Ltd., Osaka-shi, Osaka 528-8686 (JP)
(74) Representative: Dossmann, Gérard
(86) International application number: PCT/JP2006/308402
(87) International publication number: WO 2006/115193

(57) **Abstract**

A preventive/remedy for xanthoma which contains a compound having an inhibitory effect on squalene synthase, its prodrug or its salt.

## Description

### Technical field

The present invention relates to a remedy for xanthoma which comprises a compound having an inhibitory effect on squalene synthase, its prodrug or its salt, and the like.

### Background technique

Xanthomatosis is a clinical finding in which lipids are deposited in the skin or connective tissues, and sites of the deposition are found in the skin of eyelids, palms, tendons and articular extension sides, and the like. It is known that patients with xanthoma palpebrarum, which is said to most frequently appear, have a high coronary disease morbidity. Thus the therapy of xanthomatosis is significant. As a mechanism of xanthoma formation, it is known that lipoproteins in plasma leak out of a blood vessel due to mechanical stimulation or inflammation, and then the lipoproteins are ingested by macrophages, thereby cells in which a large amount of lipids are accumulated (foamy cells) are localized. In addition, it is believed that one of factors for promotion or exacerbation of the xanthoma formation process is hyperlipidemia (hyperlipoproteinemia), and the type of a formed xanthoma depends on the type of hyperlipoproteinemia.

That is, in the case of hypercholesterolemia, particularly familial hypercholesterolemia, xanthoma tuberosum, tendon xanthoma and xanthoma palpebrarum are specifically observed. In the case of hypertriglyceridemia, eruptive xanthoma is specifically observed. In the case of type III hyperlipidemia in which remnant lipoprotein is increased, eruptive tuberous xanthoma, palmar xanthoma and xanthoma striatum palmare are specifically observed. On the other hand, it is reported that xanthoma palpebrarum is not accompanied by hypercholesterolemia in some cases, rather in many cases, the level of high density lipoprotein (HDL)-cholesterol is relatively lowered (see nonpatent document 1).

Thus, all factors contributing to plasma lipid abnormality including hypercholesterolemia, change in blood vessel function, activation of macrophage and the like are important as a xanthomatosis promoting factor, and hyperlipidemia dose not necessarily lead to development of xanthoma. For example, a patient in whom a factor contributing to infiltration, activation or the like of a macrophage preferentially acts is more likely to develop xanthomatosis even if the lipid level in plasma is not so high. For the above reason, in the medical field, in addition to a therapy depending on classification of hyperlipemia, therapies in which other promotion factors of xanthoma formation are focused are considered.

Therefore, a drug to be used usually varies depending on the type of a xanthoma. For example, improvement of the serum lipid level is effective for treatment of hyperlipemic xanthomatosis, and a dietary therapy or an administration of a fibrate drug is selected for treatment of hypertriglyceridemic xanthomatosis and type III hyperlipemic xanthomatosis. For treatment of hypercholestrolemic xanthomatosis, an administration of an HMG-CoA reductase inhibitor is selected. However, in the case of treatment of familial hypercholesterolemic xanthomatosis, the effect of an HMG-CoA reductase inhibitor is insufficient and it is difficult to induce the regression of xanthoma tuberosum and tendon xanthoma. For treatment of xanthoma palpebrarum, an HMG-CoA reductase inhibitor is expected to be ineffective, and probcol is effective (see nonpatent document 2).

However, it is reported that an HMG-CoA reductase inhibitor has insufficient efficacy (see nonpatent document 2) and that probcol may lead to an abnormal cardiogram (QT extension) or a reduction in the HDL-cholesterol level (see nonpatent document 3), although an HMG-CoA reductase inhibitor and probcol which are cholesterol lowering drugs inhibit the formation of xanthomas or induce the regression of xanthomas. In the case of using a fibrate drug which is a therapeutic drug for hypertriglyceridemia, there is a fear that hepatotoxicity or myotoxicity may be induced. Therefore, there is no satisfactory therapy for xanthomatosis.

Although it has been known that a compound having an inhibitory effect on squalene synthase is useful as a preventive or a remedy for hyperlipemia, atherosclerosis and the like, a triglyceride lowering agent, an HDL-cholesterol increasing agent, an agent for increasing ubiquinone which has an antioxidant effect, and the like (e.g. see patent documents 1 to 5), it has never been reported that a compound having an inhibitory effect on squalene synthase exhibits an improving effect on xanthomatosis both in vitro and in vivo. A person skilled in the art can not clearly anticipate what kind of an effect on xanthomatosis is produced if these effects are exerted in combination.

Further, a compound having an inhibitory effect on squalene synthase can be safely used for treating xanthomatosis in a patient who is hardly treated with an existing drug (particularly, a hyperlipidemia therapeutic drug) such as an HMG-CoA reductase inhibitor or probcol as mentioned above. Therefore, a compound having an inhibitory effect on squalene synthase is useful as a new remedy for xanthomatosis which responds to such medical needs.
Patent Document 1: JP-A 6-239843
Patent Document 2: JP-A 8-157369
Patent Document 3: JP-A 9-136880
Patent Document 4: JP-A 2002-080468
Patent Document 5: JP-A 2002-205956
Nonpatent Document 1: Bergman R, J Am Acad Dermatol vol.30, 236-242 (1994)
Nonpatent Document 2: Japan Clinic vol.59, Supplemental Volume 2, p72-725, 2001
Nonpatent Document 3: Chong PH and Bachenheimer, Drugs vol.60,55-93 (2000)

### Disclosure of the invention

### Problems to be solved by the invention

As described above, existing drugs which are currently used for treating xanthomatosis have insufficient therapeutic effect or may have severe side effect, and therefore they are not necessarily satisfactory. Thus, a new remedy for xanthomatosis which is superior in therapeutic effect and safety has been demanded.

### Means for Solving the Problem

In view of the above circumstances, the present inventors intensively studied and, as a result, found for the first time that a compound having an inhibitory effect on squalene synthase has an activity for suppressing the xanthoma formation and an activity for inducing the xanthoma regression, resulting in completion of the present invention.

That is, the present invention relates to:
(1) an agent for preventing and/or treating xanthomatosis; or physical dysfunction or cosmetic disorder resulting from xanthoma formation, which comprises a compound having an inhibitory effect on squalene synthase, its prodrug or its salt;
(2) the agent according to the above (1), which is a preventing and/or treating agent for xanthomatosis;
(3) the agent according to the above (1), wherein the compound having an inhibitory effect on squalene synthase is a compound represented by the formula: 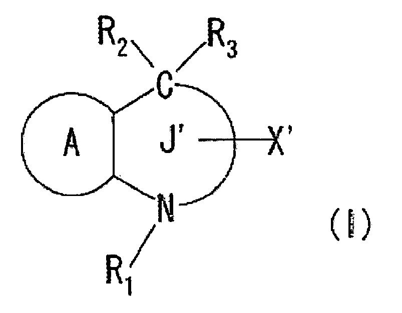 wherein R₁ represents a hydrogen atom or an optionally substituted hydrocarbon group; R₂ and R₃ are the same or different, and represent a hydrogen atom, an optionally substituted hydrocarbon group or an optionally substituted heterocyclic group; X' represents an optionally esterified carboxyl group, an optionally substituted carbamoyl group, an optionally substituted hydroxyl group, an optionally substituted amino group, or a group composed of an optionally substituted heterocyclic residue having a hydrogen atom which can be deprotonated; a ring A represents an optionally substituted benzene ring or an optionally substituted heterocycle; a ring J' represents a 7- or 8-membered heterocycle containing three or less heteroatoms as ring constituting atoms, and a ring J' may have a further substituent in addition to R₁, R₂ ,R₃ and X';
(4) the agent according to the above (1), wherein the compound having an inhibitory effect on squalene synthase is a compound represented by the formula: 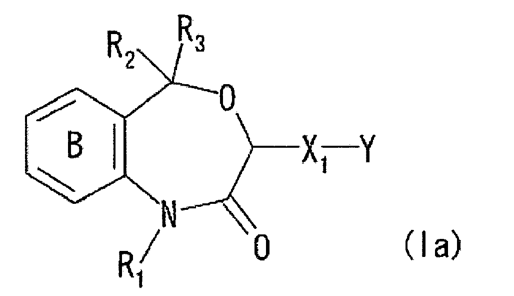 wherein R₁ represents an optionally substituted hydrocarbon group; R₂ and R₃ are the same or different, and represent a hydrogen atom or an optionally substituted hydrocarbon group; X₁ represents a divalent atom chain; Y represents an optionally substituted carbamoyl group; and a ring B represents an optionally substituted benzene ring;
(5) the agent according to the above (1), wherein the compound having an inhibitory effect on squalene synthase is N-[[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl]piperidine-4-acetic acid;
(6) the agent according to the above (1), wherein the compound having an inhibitory effect on squalene synthase is a compound represented by the formula: 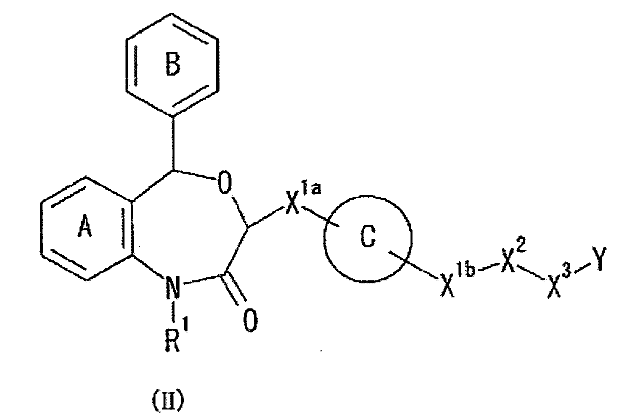 wherein a ring A and a ring B each represent an optionally substituted benzene ring; a ring C represents an optionally further substituted aromatic ring; R¹ represents a lower alkyl group optionally substituted with an optionally substituted hydroxyl group; X^{1a} represents a bond or optionally substituted lower alkylene; X^{1b} represents a bond or optionally substituted lower alkylene; X² represents a bond, -O- or -S-; X³ represents a bond or an optionally substituted divalent hydrocarbon group; and Y represents an optionally esterified or amidated carboxyl group;
(7) the agent according to the above (1), wherein the compound having an inhibitory effect on squalene synthase is 3-(2-{3-[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]propyl}-1,3-thiazol-5-yl)propionic acid, 3-(2-{2-[(3R,5S)-7-chloro-5-(2,3-dimethoxyphenyl)-1-(2,2-dimethylpropyl)-2-oxo-1,2,3,5-tetrahydro-4,1-benzoxazepin-3-yl]ethyl}-1,3-thiazol-4-yl)propionic acid, (2-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-1,3-oxazol-5-yl)propionic acid, or (2-{[(3R,5S)-7-chloro-5-(2,3-dimethoxyphenyl)-1-isobutyl-2-oxo-1,2,3,5-tetrahydro-4,1-benzoxazepin-3-yl]methyl}-1,3-oxazol-5-yl)acetic acid;
(8) the agent according to the above (1), wherein the compound having an inhibitory effect on squalene synthase is 5-(3-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-1,2,4-oxadiazol-5-yl)pentanoic acid, 5-(3-{[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]methyl}-1,2,4-oxadiazol-4-yl)pentanoic acid, 5-(3-{[(3R,5S)-7-chloro-5-(2,3-dimethoxyphenyl)-1-(2,2-dimethylpropyl)-2-oxo-1,2,3,5-tetrahydro-4,1-benzoxazepin-3-yl]methyl}-1,2,4-oxadiazol-5-yl)pentanoic acid, or (4-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl}phenyl)acetic acid;
(9) a method for preventing and/or treating xanthomatosis; or physical dysfunction or cosmetic disorder resulting from xanthoma formation, which comprises administering an effective amount of a compound having an inhibitory effect on squalene synthase, its prodrug or its salt to a mammal;
(10) use of a compound having an inhibitory effect on squalene synthase, its prodrug or its salt for production of a preventing and/or treating agent for xanthomatosis; or physical dysfunction or cosmetic disorder resulting from xanthoma formation; and the like.

### Effect of the invention

Since a compound having an inhibitory effect on squalene synthase used in the present invention has an excellent xanthomatosis improving effect, the present invention can provide a preventive and/or a remedy effective for a patient whom a conventional remedy is ineffective for and who was previously subjected to a physical therapy such as extirpative surgery or a freezing method.

The "xanthomatosis improving effect" in the present invention refers to an effect of regressing or reducing the level of a symptom of xanthoma formed in a living body due to various factors as compared with the level before administration, or an effect of suppressing the progression of a symptom of the xanthoma. The clinical meaning thereof is to exhibit an effect of treating or preventing xanthomatosis itself, as well as an effect of treating or preventing physical dysfunction or cosmetic disorder resulting from xanthoma formation.

Examples of the "xanthoma" in the present invention include xanthoma tuberosum, tendon xanthoma, xanthoma palpebrarum, eruptive xanthoma, eruptive tuberous xanthoma, palmar xanthoma or xanthoma striatum palmare, and the like.

Examples of the "physical dysfunction resulting from xanthoma formation" in the present invention include deterioration in motor function, deterioration in workability, deterioration in visual cognition, and the like.

### Best mode for carrying out the invention

The "compound having an inhibitory effect on squalene synthase" used in the present invention may be any compound having a squalene synthase inhibitory effect, and examples thereof include squalestatins (e.g. US Patent No.5506262, US Patent No.5430055, US Patent No.5409950, US Patent No.5369125, JP-A 7-173166, JP-A 9-124655, JP-A 9-227566, Annual Review of Microbiology, Vol.49, pp.607-639, 1995, Journal of Medicinal Chemistry, Vol.38, pp.3502-3513, 1995, Journal of Medicinal Chemistry, Vol.39, pp.207-216, 1996, Journal of Medicinal Chemistry, Vol.39, pp.1413-1422, 1996 etc.), phosphoric acid compounds and carboxylic acid compounds which are substrate analogous (e.g. US Patent No.5374628, US Patent No.5441946, US Patent No.5428028, JP-A 7-041554, WO 9504025, Journal of Medicinal Chemistry, Vol.38, pp.2596-2605, 1995, Arzniemittel-Forschung Drug Research, Vol.46, pp.759-762, 1996, Journal of Medicinal Chemistry, Vol.31, pp.1869-1871, 1988, Journal of Medicinal Chemistry, Vol.39, pp.657-660, 1996, Journal of Medicinal Chemistry, Vol.39, pp.661-664, 1996 etc.), carboxylic acid derivatives (e.g. WO 9740006, WO 9633159, WO 9521834, WO 9748701, European Patent No. 645377, European Patent No. 645378, European Patent No. 814080, European Patent No. 790235, JP-A 7-173120, JP-A 10-316634, JP-A 10-298134, JP-A 10-298177, JP-A 10-316617, JP-A 9-136880, WO 2000-00458, WO 2001-98282, WO 98-29380, Bioorganic Medicinal Chemistry Letters, Vol.5, pp.1989-1994, Bioorganic Medicinal Chemistry Letters, Vol.6, pp.463-466, 1996, Journal of Medicinal Chemistry, Vol.40, pp.2123-2125, 1997, etc.), amine compounds such as quinuclidine derivatives (e.g. US Patent No. 5385912, US Patent No. 5494918, US Patent No. 5395846, US Patent No. 5451596, JP-A 8-134067, JP-A 2000-169474, JP-A 10-152453, JP-A 2000-502716, WO 9403541, WO 9405660, WO 9535295, WO 9626938, WO 9531458, WO 9500146, WO 9725043, WO 9812170 etc.), Zaragozic acids, and equivalents thereof. Inter alia, preferred are a compound represented by the formula: 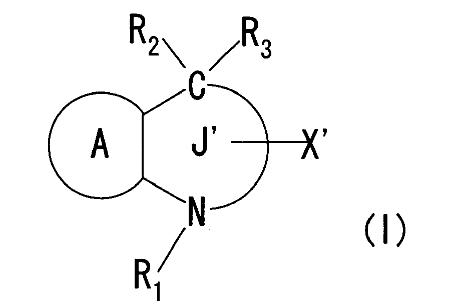 wherein R₁ represents a hydrogen atom or an optionally substituted hydrocarbon group; R₂ and R₃ are the same or different, and represent a hydrogen atom, an optionally substituted hydrocarbon group or an optionally substituted heterocyclic group; X' represents an optionally esterified carboxyl group, an optionally substituted carbamoyl group, an optionally substituted hydroxyl group, an optionally substituted amino group, or a group composed of an optionally substituted heterocyclic residue having a hydrogen atom which can be deprotonated; a ring A represents an optionally substituted benzene ring or an optionally substituted heterocycle; a ring J' represents a 7- or 8-membered heterocycle containing three or less heteroatoms as ring constituting atoms, and a ring J' may have a further substituent in addition to R₁, R₂, R₃ and X'; a compound represented by the formula: 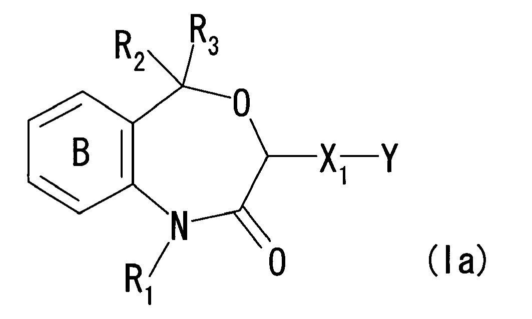 wherein R₁ represents a hydrogen atom or an optionally substituted hydrocarbon group; R₂ and R₃ are the same or different, and represent a hydrogen atom, an optionally substituted hydrocarbon group or an optionally substituted heterocyclic group; X₁ represents a bond or a divalent atom chain; Y represents an optionally esterified carboxyl group, an optionally substituted carbamoyl group, an optionally substituted hydroxyl group, an optionally substituted amino group, or a group composed of an optionally substituted heterocyclic residue having a hydrogen atom which can be deprotonated; and a ring B represents an optionally substituted benzene ring; and
a compound represented by the formula: 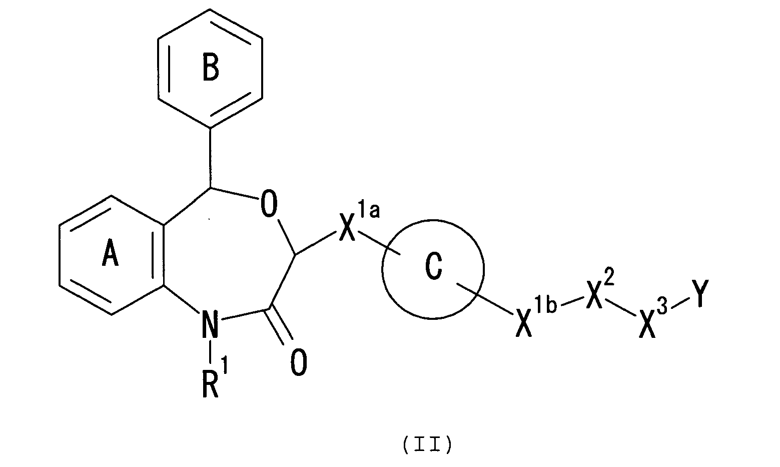 wherein a ring A and a ring B each represent an optionally substituted benzene ring; a ring C represents an optionally further substituted aromatic ring; R¹ represents a lower alkyl group optionally substituted with an optionally substituted hydroxyl group; X^{1a} represents a bond or an optionally substituted lower alkylene; X^{1b} represents a bond or optionally substituted alkylene; X² represents a bond, -O- or -S-; X³ represents a bond or an optionally substituted divalent hydrocarbon group; and Y represents an optionally esterified or amidated carboxyl group.

Examples of other squalene synthase inhibitors include A-104109 (Abott Laboratories): 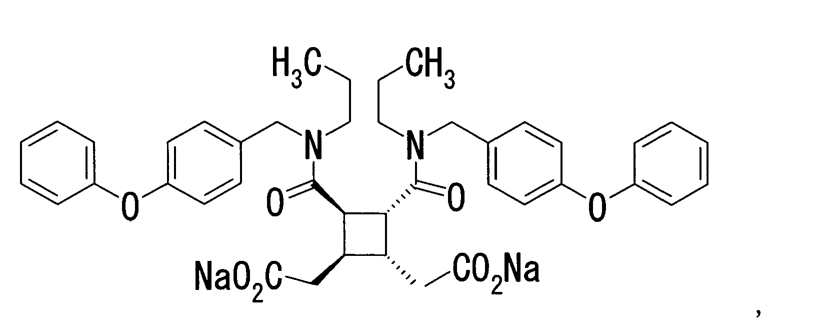 F-10863-A (Zaragozic acid D3, Sankyo), bisphosphonic acid derivatives such as ER-28448 and ER-27856 (ER-28448 prodrug) and quinuclidine derivatives such as ER-119884 and ER-132781 (Eisai): 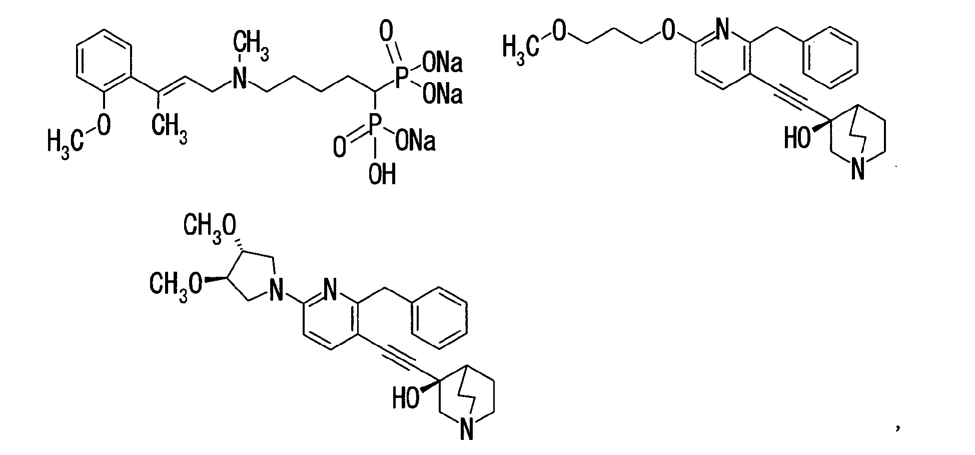 RPR-107393 and RPR-101821 (Aventis): 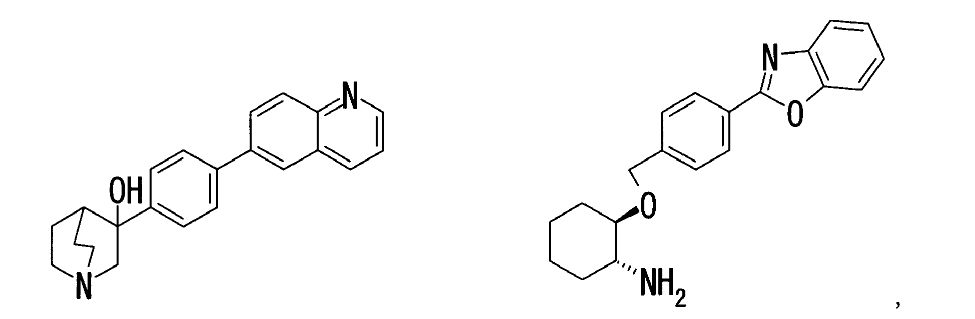 thiadiazole derivatives (Novonordisk): 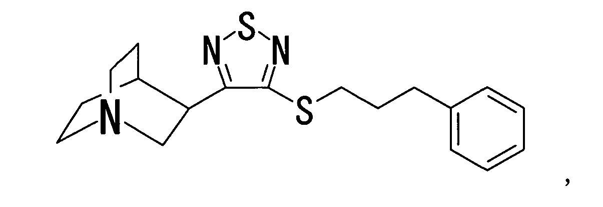 isopropylamine derivatives and quinuclidine derivatives (Yamanouchi): 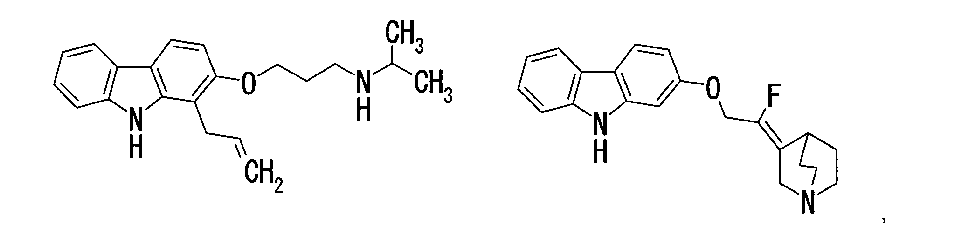 isoquinuclidine derivatives (Kotobuki): 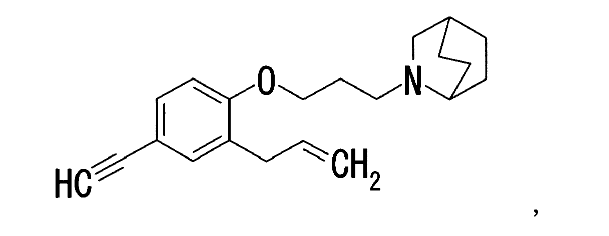 malonic acid derivatives (Nippon Kayaku Co., Ltd.): 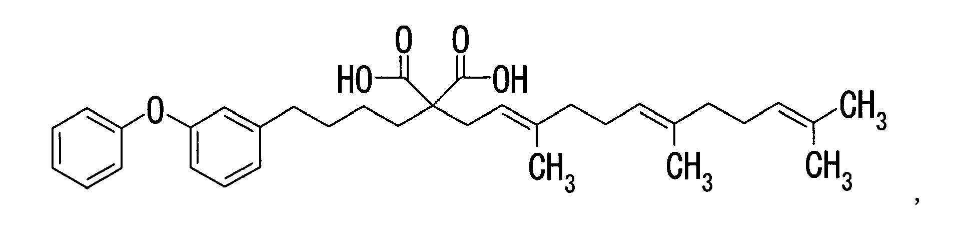 propionyl derivatives (Daiichi Pharmaceutical Co., Ltd.): 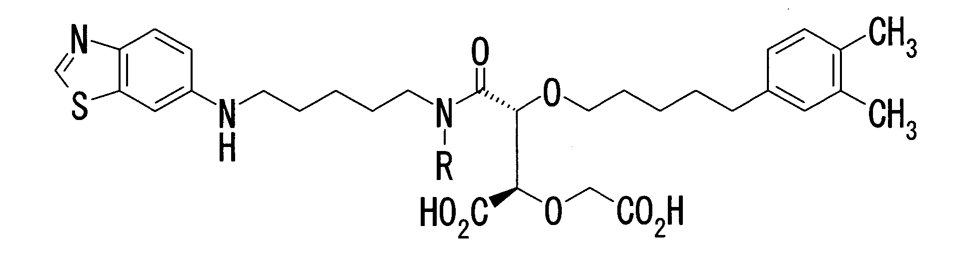 (wherein R is a hydrogen atom or a methyl group), SQ-34919, SQ-32709, BMS-187745, BMS-188494 (Bristol Meyers Squibb): 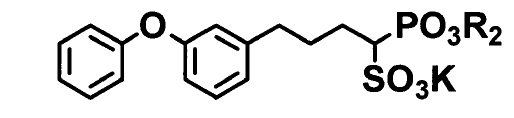 (wherein R is a potassium atom or -CH₂OCOC(CH₃)₃), J-104118 (Merck): 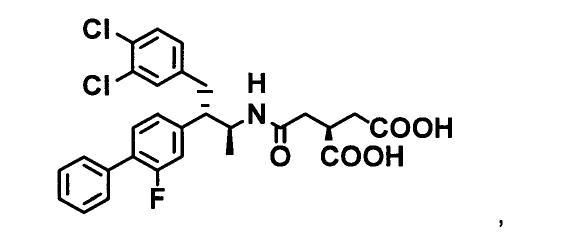 quinuclidine derivatives (Astrazeneka): 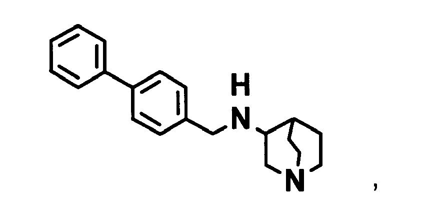 SDZ-266-806 (Novaltis): 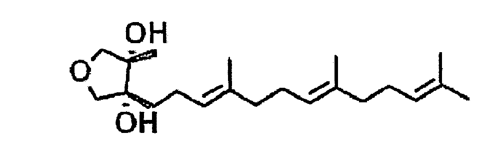 and the like, and these squalene synthase inhibitors can be also used for the agent of the present invention.
The "compound having an inhibitory effect on squalene synthase" used in the present invention may be used in the form of a salt or a prodrug.
The "salt" of the compound having an inhibitory effect on squalene synthase used in the present invention is preferably a pharmaceutically acceptable salt or a physiologically acceptable acid addition salt. Examples of such a salt include salts with inorganic acids (e.g. hydrochloric acid, phosphoric acid, hydrobromic acid, sulfuric acid etc.) and salts with organic acids (e.g. acetic acid, formic acid, propionic acid, fumaric acid, maleic acid, succinic acid, tartaric acid, citric acid, malic acid, oxalic acid, benzoic acid, methanesulfonic acid, benzenesulfonic acid etc.). Further, when the "compound having an inhibitory effect on squalene synthase" used in the present invention has an acidic group such as carboxylic acid, the "compound having an inhibitory effect on squalene synthase" may form a salt with an inorganic base (e.g. alkali metal or alkaline earth metal such as sodium, potassium, calcium or magnesium, or ammonia, or the like) or an organic base (e.g. tri-C₁₋₃alkylamine such as triethylamine or the like).

The "prodrug" of the compound having an inhibitory effect on squalene synthase used in the present invention or a salt thereof [hereinafter, referred to as the SSI compound in some cases] refers to a compound which is converted to the SSI compound by a reaction with an enzyme or gastric acid under the physiological condition, that is, a compound which is converted to the SSI compound by enzymatic oxidation, reduction, hydrolysis or the like, a compound which is converted to the SSI compound by hydrolysis with gastric acid, or the like. Examples of the prodrug of the SSI compound include a compound in which an amino group of the SSI compound is acylated, alkylated or phosphorylated (e.g. a compound in which an amino group of the SSI compound is eicosanoylated, alanylated, pentylaminocarbonylated, (5-methyl-2-oxo-1,3-dioxolen-4-yl)methoxycarbonylated, tetrahydrofuranylated, pyrrolidylmethylated, pivaloyloxymethylated, or tert-butylated), a compound in which a hydroxyl group of the SSI compound is acylated, alkylated, phosphorylated or borated (e.g. a compound in which a hydroxyl group of the SSI compound is acetylated, palmitoylated, propanoyled, pivaloylated, succinylated, fumarylated, alanylated, or dimethylaminomethylcarbonylated), a compound in which a carboxyl group of the SSI compound is esterified or amidated (e.g. a compound in which a carboxyl group of the SSI compound is ethylesterified, phenylesterified, carboxymethylesterified, dimethylaminomethylesterified, pivaloyloxymethylesterified, ethoxycarbonyloxyethylesterified, phthalidylesterified, (5-methyl-2-oxo-1,3-dioxolen-4-yl)methylesterified, cyclohexyloxycarbonylethylesterified, or methylamidated), and the like. These compounds can be produced from the SSI compound by a per se known method.

Alternatively, the prodrug of the SSI compound may be a compound which is changed into the SSI compound under the physiological condition as described in "Pharmaceutical Research and Development" published by Hirokawa Publishing Company in 1990, Vol.7, Drug Design, pp.163-198.

Alternatively, the SSI compound may be a hydrate.

When an optically active form of the SSI compound is needed, it can be obtained, for example, by using an optically active starting material, or resolution of a racemic form of the compound by a conventional method. The SSI compound may have an asymmetric carbon in its molecule, and when there are two kinds of R conformation or S conformation of steric isomers, each of them and a mixture of them are included in the present invention.

Examples of the hydrocarbon group of the "optionally substituted hydrocarbon group" represented by R₁ in the formulas (I) and (Ia) include an aliphatic chain (acyclic) hydrocarbon group, an alicyclic hydrocarbon group and an aryl group, and among them, an aliphatic chain hydrocarbon group is preferable.

Examples of the aliphatic chain hydrocarbon group of the hydrocarbon group include a straight or branched aliphatic hydrocarbon group, for example, an alkyl group, an alkenyl group, an alkynyl group and the like. Among them, a branched alkyl group is preferable. Example of the alkyl include C₁₋₇ alkyl such as methyl, ethyl, n-propyl, isopropyl, n-butyl, isobutyl, sec-butyl, tert-butyl, n-pentyl, isopentyl, neopentyl, 1-methylpropyl, n-hexyl, isohexyl, 1,1-dimethylbutyl, 2,2-dimethylbutyl, 3,3-dimethylbutyl, 3,3-dimethylpropyl, 2-ethylbutyl, n-heptyl and the like, and among them, C₃₋₅ alkyl such as n-propyl, isopropyl, isobutyl and neopentyl is preferable, and isobutyl and neopentyl are particularly preferable. Examples of the alkenyl group include C₂₋₆ alkenyl such as vinyl, allyl, isopropenyl, 2-methylallyl, 1-propenyl, 2-methyl-1-propenyl, 2-methyl-2-propenyl, 1-butenyl, 2-butenyl, 3-butenyl, 2-ethyl-1-butenyl, 2-methyl-2-butenyl, 3-methyl-2-butenyl, 1-pentenyl, 2-pentenyl, 3-pentenyl, 4-pentenyl, 4-methyl-3-pentenyl, 1-hexenyl, 2-hexenyl, 3-hexenyl, 4-hexenyl, 5-hexenyl and the like, and among them, vinyl, allyl, isopropenyl, 2-methylallyl, 2-methyl-1-propenyl, 2-methyl-2-propenyl, 3-methyl-2-butenyl and the like are particularly preferable. Examples of the alkynyl group include C₂₋₆ alkynyl such as ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, 5-hexynyl and the like, and among them, ethynyl, 1-propnyl, 2-propynyl and the like are particularly preferable.

Examples of the alicyclic hydrocarbon group of the hydrocarbon group include a saturated or unsaturated alicyclic hydrocarbon group, for example, a cycloalkyl group, a cycloalkenyl group, a cycloalkadienyl group and the like. As the cycloalkyl group, a cycloalkyl group of 3 to 9 carbon atoms is preferable. Examples of the cycloalkyl group include cyclopropyl, cyclobutyl, cyclopentyl, cyclohexyl, cycloheptyl, cyclooctyl, cyclononyl and the like, and among them, a C₃₋₆ cycloalkyl group such as cyclopropyl, cyclobutyl, cyclopentyl and cyclohexyl is preferable. Examples of the cycloalkenyl group include a C₅₋₆ cycloalkenyl group such as 2-cyclopenten-1-yl, 3-cyclopenten-1-yl, 2-cyclohexen-1-yl, 3-cyclohexen-1-yl, 1-cyclobuten-1-yl, 1-cyclopenten-1-yl and the like. Examples of the cycloalkadienyl group include a C₅₋₆cycloalkadienyl group such as 2,4-cyclopentadien-1-yl, 2,4-cyclohexadien-1-yl, and 2,5-cyclohexadien-1-yl.

Examples of the aryl group of the hydrocarbon group include a monocyclic or fused polycyclic aromatic hydrocarbon group of 6 to 16 carbon atoms, for example, phenyl, naphthyl, anthryl, phenanthryl, acenaphthylenyl and the like, and among them, a C₆₋₁₀ aryl group such as phenyl, 1-naphthyl, 2-naphthyl and the like is particularly preferable.

Examples of a substituent for the "optionally substituted hydrocarbon group" represented by R₁ include an optionally substituted aryl group, an optionally substituted cycloalkyl group, an optionally substituted cycloalkenyl group, an optionally substituted heterocyclic group, an optionally substituted amino group, an optionally substituted hydroxyl group, an optionally substituted thiol group, a halogen (e.g. fluorine, chlorine, bromine, or iodine), oxo and the like. The hydrocarbon group may be substituted with 1 to 5 (preferably 1 to 3) of these arbitrary substituents at replaceable positions. Examples of the aryl group of the optionally substituted aryl group include a C₆₋₁₆ aryl group such as phenyl, naphthyl, anthryl, phenanthryl, acenaphthylenyl and the like, and among them, a C₆₋₁₀ aryl group such as phenyl, 1-naphthyl, 2-naphtyl and the like is preferable. Examples of a substituent for the optionally substituted aryl group include an alkoxy group of 1 to 3 carbon atoms (e.g. methoxy, ethoxy, propoxy etc.), a halogen atom (e.g. fluorine, chlorine, bromine, iodine), an alkyl group of 1 to 3 carbon atoms (e.g. methyl, ethyl, propyl etc.) and the like, and the aryl group may be substituted with 1 to 2 of these arbitrary substituents. Examples of the cycloalkyl group of the optionally substituted cycloalkyl group include a C₃₋₇ cycloalkyl group such as cyclopropyl, cyclobutyl, cyclopentyl, cyclohexyl, cycloheptyl and the like. Examples of a substituent for the optionally substituted cycloalkyl group and a substitution number thereof are the same as those of a substituent for the optionally substituted aryl group described above. Examples of the cycloalkenyl group of the optionally substituted cycloalkenyl group include a C₃₋₆ cycloalkenyl group such as cyclopropenyl, cyclobutenyl, cyclopentenyl, cyclohexenyl and the like. Examples of a substituent for the optionally substituted cycloalkenyl group and a substitution number thereof are the same as those of a substituent for the optionally substituted aryl group described above. Examples of the heterocyclic group of the optionally substituted heterocyclic group include an aromatic heterocyclic group and a saturated or unsaturated non-aromatic heterocyclic group (aliphatic heterocyclic group) which have at least one, preferably 1 to 4 heteroatoms selected from oxygen, sulfur and nitrogen as atoms constituting a ring system (ring atoms), and preferred is an aromatic heterocyclic group. Examples of the aromatic heterocyclic group include a 5- to 6-membered aromatic monocyclic heterocyclic group (e.g. furyl, thienyl, pyrrolyl, oxazolyl, isoxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thidiazolyl, 1,2,4-thiadiazolyl, 1,3,4-thiadiazolyl, 1,2,3-triazolyl, 1,2,4-triazolyl, tetrazolyl, pyridyl, pyridazinyl, pyrimidinyl, pyrazinyl, triazinyl etc.) and an aromatic fused heterocyclic group in which two or three 5- to 6-membered rings are fused (e.g. benzofuranyl, isobenzofuranyl, benzo[b]thienyl, indolyl, isoindolyl, 1H-indazolyl, benzimidazolyl, benzoxazolyl, 1,2-benzisoxazolyl, benzothiazolyl, 1,2-benzoisothiazolyl, 1H-benzotriazolyl, quinolyl, isoquinolyl, cinnolinyl, quinazolinyl, quinoxalinyl, phthalazinyl, naphthyridinyl, purinyl, pteridinyl, carbazolyl, α-carbolinyl, β-carbolinyl, γ-carbolinyl, acridinyl, phenoxazinyl, phenothiazinyl, phenazinyl, phenoxathiinyl, thianthrenyl, phenanthridinyl, phenanthrolinyl, indolidinyl, pyrrolo[1,2-b]pyridazinyl, pyrazolo[1,5-a]pyridyl, imidazo[1,2-a]pyridyl, imidazo[1,5-a]pyridyl, imidazo[1,2-b]pyridazinyl, imidazo[1,2-a]pyrimidinyl, 1,2,4-triazolo[4,3-a]pyridyl, 1,2,4-triazolo[4,3-b]pyridazinyl etc.), and among them, a 5- to 6-membered aromatic monocyclic heterocyclic group such as furyl, thienyl, indolyl, isoindolyl, pyrazinyl, pyridyl and pyrimidinyl is preferable. Examples of the non-aromatic heterocyclic group include a 4- to 8-membered non-aromatic heterocyclic group such as oxyranyl, azetidinyl, oxetanyl, thietanyl, pyrrolidinyl, tetrahydrofuryl, thiolanyl, piperidyl, tetrahydropyranyl, morpholinyl, thiomorpholinyl, piperazinyl and the like. The optionally substituted heterocyclic group may have 1 to 4, preferably 1 to 2 substituents, and examples of such substituent include an alkyl group of 1 to 3 carbon atoms (e.g. methyl, ethyl, propyl etc.) and the like. Examples of a substituent for the optionally substituted amino group (including an amino group, a mono- or di-substituted amino group), the optionally substituted hydroxyl group and the optionally substituted thiol group include lower (C₁₋₃)alkyl (e.g. methyl, ethyl, propyl etc.) and the like. When the hydrocarbon group of the optionally substituted hydrocarbon group represented by R₁ is an alicyclic hydrocarbon group or aryl group, a substituent for the optionally substituted hydrocarbon group may be further an alkyl group of 1 to 3 carbon atoms (e.g. methyl, ethyl, propyl etc.).

Further, as described above, R₁ may have an oxo group as a substituent, and R₁ includes a carboxylic acid acyl group which is such an oxo-substituted hydrocarbon group. Examples of such a carboxylic acid acyl group include an optionally substituted acyl group of 1 to 6 carbon atoms (e.g. formyl, acetyl, propionyl, butyryl, isobutyryl, valeryl, isovaleryl, pivaloyl, hexanoyl, dimethylacetyl, trimethylacetyl etc.). The acyl group may have 1 to 5 substituents at replaceable positions, and examples of the substituent include halogen (e.g. fluorine, chlorine, bromine).

In the formulas (I) and (Ia), examples of the "optionally substituted hydrocarbon group" represented by R₂ and R₃ are the same as examples of the "optionally substituted hydrocarbon group" represented by R₁ as described above, provided that examples of the alkyl group, the aryl group and the substituent thereof may include the following. That is, examples of the alkyl group of the "optionally substituted alkyl group" include a lower alkyl group of 1 to 6 carbon atoms (e.g. methyl, ethyl, n-propyl, isopropyl, butyl, isobutyl, sec-butyl, tert-butyl, pentyl, isopentyl, neopentyl, hexyl, isohexyl etc.), and preferable examples thereof include a C₁₋₄ alkyl group such as methyl, ethyl, propyl, isopropyl, butyl, tert-butyl and the like. For example, such optionally substituted alkyl group may have 1 to 4 substituents, and examples of such a substituent include halogen (e.g. fluorine, chlorine, bromine, iodine), a lower alkoxy group of 1 to 4 carbon atoms (e.g. methoxy, ethoxy, propoxy, isopropoxy, butoxy, tert-butoxy etc.) and the like.

Examples of the "optionally substituted aryl group" include a monocyclic or fused polycyclic hydrocarbon group, for example, phenyl, naphthyl, anthryl, phenanthryl, acenaphthylenyl and the like, and among them, phenyl is particularly preferable. Examples of a substituent for the "optionally substituted aryl group" include halogen (e.g. fluorine, chlorine, bromine, iodine), optionally substituted lower alkyl, optionally substituted lower alkoxy, optionally substituted hydroxyl group, nitro, cyano and the like, and the optionally substituted aryl group may be substituted with 1 to 3 (preferably 1 to 2) same or different substituents of these substituents. Examples of the lower alkyl include an alkyl group of 1 to 4 carbon atoms such as methyl, ethyl, n-propyl, isopropyl, n-butyl, isobutyl, sec-butyl, tert-butyl and the like, particularly preferably methyl and ethyl. Examples of the lower alkoxy include an alkoxy group of 1 to 4 carbon atoms such as methoxy, ethoxy, n-propoxy, isopropoxy, n-butoxy, isobutoxy, sec-butoxy, tert-butoxy and the like, particularly preferably methoxy and ethoxy. Examples of a substituent for the optionally substituted lower alkyl group or the optionally substituted lower alkoxy group include a halogen atom (e.g. fluorine, chlorine, bromine, iodine etc.), and the optionally substituted lower alkyl group or the optionally substituted lower alkoxy group may be substituted at 1 to 5 replaceable positions. Examples of a substituent for the optionally substituted hydroxyl group include a lower (C₁₋₄)alkyl group (e.g. methyl, ethyl, propyl, isopropyl, butyl, tert-butyl etc.), a C₃₋₆ cycloalkyl group (cyclopropyl, cyclobutyl, cyclopentyl, cyclohexyl etc.), a C₆₋₁₀ aryl group (e.g. phenyl, 1-naphthyl, 2-naphtyl etc.), a C₇₋₁₂ aralkyl group (e.g. benzyl, phenethyl etc.) and the like. In addition, adjacent substituents among these substituents may form a ring. When the aryl group of the "optionally substituted aryl group" represented by R₂ or R₃ is a phenyl group, for example, groups represented by: 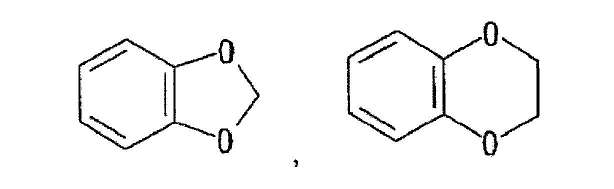 may be used, and such groups may be substituted with 1 to 4 lower(C₁₋₃)alkyl groups (e.g. methyl, ethyl, propyl, isopropyl etc.).

Examples of the heterocyclic group of the "optionally substituted heterocyclic group" represented by R₂ and R₃ include the heterocyclic groups described above concerning the "optionally substituted heterocyclic group" mentioned above as an example of a substituent for the "optionally substituted hydrocarbon group" represented by R₁, and inter alia, particularly preferred is a 5- to 6-membered aromatic monocyclic heterocycle such as furyl, thienyl, indolyl, isoindolyl, pyrazinyl, pyridyl, pyrimidyl, imidazolyl and the like. Examples of a substituent for the heterocyclic group include alkyl of 1 to 3 carbon atoms (e.g. methyl, ethyl, propyl etc.), and the heterocyclic group may have 1 to 4 of these substituents.

Among the aforementioned groups, the group represented by R₂ and R₃ is preferably an optionally substituted phenyl group, more preferably a substituted phenyl group, particularly preferably a phenyl group substituted with 1 to 3, preferably 1 to 2 substituents selected from halogen such as chlorine, bromine and the like and lower(C₁₋₃)alkoxy and the like. It is preferable that one of R₂ and R₃ is hydrogen.

In the formula (I), examples of the "group composed of an optionally esterified carboxyl group" represented by X' include an optionally esterified carboxyl group, and a group having an optionally esterified carboxyl group. Examples of the optionally esterified carboxyl group are the same as those of the optionally esterified carboxyl group represented by Y as defined below.

Examples of the "group composed of an optionally substituted carbamoyl group" represented by X' include an optionally substituted carbamoyl group, and a group having an optionally substituted carbamoyl group. Examples of the optionally substituted carbamoyl group are the same as those of the optionally substituted carbamoyl group represented by Y as defined below.

Examples of the "group composed of an optionally substituted hydroxyl group" represented by X' include an optionally substituted hydroxyl group, and a group having an optionally substituted hydroxyl group. Examples of the optionally substituted hydroxyl group are the same as those of the optionally substituted hydroxyl group represented by Y as defined below.

Examples of the "group composed of an optionally substituted amino group" represented by X' include an optionally substituted amino group, and a group having an optionally substituted amino group. Examples of the optionally substituted amino group are the same as those of the optionally substituted amino group represented by Y as defined below.

Examples of the "group composed of an optionally substituted heterocyclic residue having a hydrogen atom which can be deprotonated" represented by X' include an optionally substituted hetrocyclic residue having a hydrogen atom which can be deprotonated (i.e., having an active proton), and a group having an optionally substituted hetrocyclic residue having a hydrogen atom which can be deprotonated. Examples of the optionally substituted heterocyclic residue are the same as those of the "optionally substituted heterocyclic residue having a hydrogen atom which can be deprotonated" represented by Y as defined below.

Examples of X' include a group represented by the formula (a): 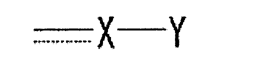 [wherein X represents a bond or a divalent or trivalent atom chain, Y represents an optionally esterified carboxyl group, an optionally substituted carbamoyl group, an optionally substituted hydroxyl group, an optionally substituted amino group, or an optionally substituted heterocyclic residue having a hydrogen atom which can be deprotonated, and a broken line represents a single bond or a double bond].

In the formula (a), the "divalent atom chain" represented by X may be any divalent chain of which straight part is composed of preferably 1 to 7, more preferably 1 to 4 atoms, and may have a side chain.

Examples of the divalent atom chain include a chain represented by: 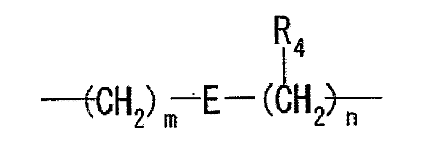 wherein m and n independently represent 0, 1, 2 or 3, E represents a bond, an oxygen atom, a sulfur atom, sulfoxide, sulfone, -N(R₅)-, -NHCO-, -CON(R₆)- or -NHCONH-, R₄ and R₆ represent a hydrogen atom, an optionally substituted lower alkyl group, an optionally substituted aralkyl group or an optionally substituted phenyl group, and R₅ represents a hydrogen atom, a lower alkyl group, an aralkyl group or an acyl group.

Examples of the alkyl group of the "optionally substituted lower alkyl group" represented by R₄ and R₆ include a straight or branched lower alkyl group of 1 to 6 carbon atoms (e.g. methyl, ethyl, n-propyl, isopropyl, n-butyl, isobutyl, tert-butyl, n-pentyl, isopentyl, neopentyl etc.). The optionally substituted lower alkyl group may have 1 to 4, preferably 1 to 2 substituents, and examples of such a substituent include an aromatic heterocyclic group (e.g. a 5- to 6-membered aromatic heterocycle containing 1 to 4 heteroatoms selected from N, O and S, such as furyl, thienyl, indolyl, isoindolyl, pyrazinyl, pyridyl, pyrimidinyl, imidazolyl etc.), an optionally substituted amino group, an optionally substituted hydroxyl group, an optionally substituted thiol group, an optionally esterified carboxyl group, a halogen atom (e.g. fluorine, chlorine, bromine, iodine), and the like. Examples of a substituent for the optionally substituted amino group (amino group or mono- or di-substituted amino group), the optionally substituted hydroxyl group and the optionally substituted thiol group include lower (C₁₋₃)alkyl (e.g. methyl, ethyl, propyl) and the like. Examples of the optionally esterified carboxyl group include C₂₋₅ alkoxycarbonyl and C₇₋₁₁ aryloxycarbonyl, such as methoxycarbonyl, ethoxycarbonyl, propoxycarbonyl, phenoxycarbonyl, 1-naphthoxycarbonyl and the like, preferably methoxycarbonyl, ethoxycarbonyl and propoxycarbonyl.

Examples of the aralkyl group of the "optionally substituted aralkyl group" represented by R₄ and R₆ include a C₇₋₁₅ aralkyl group such as benzyl, naphthylmethyl, phenylpropyl, phenylbutyl and the like. The optionally substituted aralkyl group may have 1 to 4, preferably 1 to 2 substituents, and examples of such a substituent include a halogen atom (e.g. fluorine, chlorine, bromine, iodine), an alkoxy group of 1 to 3 carbon atoms (e.g. methoxy, ethoxy, propoxy group), a hydroxyl group, an amino group, a carboxyl group, a sulfhydryl group and the like.

Examples of a substituent for the "optionally substituted phenyl group" represented by R₄ and R₆ include a halogen atom (e.g. fluorine, chlorine, bromine, iodine), C₁₋₃alkoxy (e.g. methoxy, ethoxy, propoxy etc.), C₁₋₃alkyl (e.g. methyl, ethyl, propyl) and the like.

R₄ may be different on each methylene chain.

Examples of the "lower alkyl group" and the "aralkyl group" represented by R₅ include a lower alkyl group of 1 to 4 carbon atoms (e.g. methyl, ethyl, propyl, butyl, tert-butyl etc.), and an aralkyl group of 7 to 15 carbon atoms (e.g. benzyl, phenethyl, phenylpropyl, phenylbutyl, naphtylmethyl etc.), respectively.

Examples of the "acyl group" represented by R₅ include a lower (C₁₋₆)alkanoyl group (e.g. formyl, acetyl, propionyl, butyryl, isobutyryl, valeryl, isovaleryl, pivaloyl, hexanoyl etc.), a lower (C₃₋₇)alkenoyl group (e.g. acryloyl, methacryloyl, crotonoyl, isocrotonoyl etc.), a C₄₋₇ cycloalkanecarbonyl group (e.g. cyclopropanecarbonyl group, a cylobutanecarbonyl group, a cyclopentanecarbonyl group, a cyclohexanecarbonyl group etc.), a lower (C₁₋₄) alkanesulfonyl group (e.g. mesyl, ethanesulfonyl, propanesulfonyl etc.), a C₇₋₁₄ aroyl group (e.g. benzoyl, p-toluoyl, 1-naphthoyl, 2-naphthoyl etc.), a C₆₋₁₀ aryl lower (C₂₋₄)alkanoyl group (e.g. phenylacetyl, phenylpropionyl, hydroatropoyl, phenylbutyryl etc.), a C₆₋₁₀ aryl lower (C₃₋₅) alkenoyl group (e.g. cinnamoyl, atropoyl etc.), and a C₆₋₁₀ arenesulfonyl group (e.g. benzenesulfonyl, p-toluenesulfonyl etc.) and the like.

Further, X may be a carbon chain containing a double bond, or -L-CH(OH)- (wherein L represents a bond, or a straight or branched alkylene chain). Examples of the "carbon chain containing a double bond" include a chain of which straight chain is composed of preferably 1 to 7, further preferably 1 to 4 carbon atoms, and the chain may have a side chain. The carbon chain has a double bond on its straight part, its branched part, or both of them, and preferably a double bond is on the straight part of the carbon chain. The number of double bonds contained in the carbon chain is not particularly limited, but preferably 1 to 2.

Examples of the carbon chain containing a double bond include methine, vinylene, propenylene, butenylene, butadienylene, methylpropenylene, ethylpropenylene, propylpropenylene, methylbutenylene, ethylbutenylene, propylbutenylene, methylbutadienylene, ethylbutadienylene, propylbutadienylene, pentenylene, hexenylene, heptenylene, pentadienylene, hexadienylene, heptadienylene and the like, preferably methine, vinylene, propenylene, butenylene and butadienylene. When the carbon chain is trivalent, the carbon chain is connected with a replaceable carbon atom on the ring J' via a double bond.

Examples of the "straight or branched alkylene chain" represented by L include a straight or branched alkylene chain of 1 to 6 carbon atoms, for example, divalent groups such as methylene, ethylene, trimethylene, tetramethylene, pentamethylene, hexamethylene, heptamethylene, propylene, ethylmethylene, ethylethylene, propylethylene, butylethylene, methyltetramethylene, methyltrimethylene and the like, preferably groups of 1 to 3 carbon atoms such as methylene, ethylene, trimethylene, propylene and the like.

Among the above groups, X' is preferably a group represented by the formula (b): 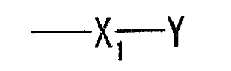 [wherein X₁ represents a bond or a divalent atom chain, Y represents an optionally esterified carboxyl group, an optionally substituted carbamoyl group, an optionally substituted hydroxyl group, an optionally substituted amino group, or an optionally substituted heterocyclic residue having a hydrogen atom which can be deprotonated].

In the formula (b), examples of the divalent atom chain represented by X₁ are the same as those of the divalent atom chains represented by X as defined above.

In the formulas (a) and (b), examples of the "divalent atom chain" represented by X or X₁ preferably include a straight or branched alkylene chain of which straight part is composed of 1 to 7 (more preferably 1 to 4) carbon atoms. Examples of the alkylene chain include a divalent group such as methylene, ethylene, trimethylene, tetramethylene, pentamethylene, hexamethylene, heptamethylene, propylene, ethylmethylene, ethylethylene, propylethylene, butylethylene, methyltetramethylene, methyltrimethylene and the like, preferably groups of 1 to 4 carbon atoms such as methylene, ethylene, trimethylene, propylene and the like.

In the formulas (a) and (b), examples of the "optionally esterified carboxyl group" represented by Y include lower alkoxycarbonyl of 2 to 7 carbon atoms (e.g. methoxycarbonyl, ethoxycarbonly, propoxycarbonyl, isopropoxycarbonyl, butoxycarbonyl, tert-butoxycarbonyl, sec-butoxycarbonyl, pentyloxycarbonyl, isopentyloxycarbonyl, neopentyloxycarbonyl etc.), C₇₋₁₄ aryloxycarbonyl (e.g. phenoxycarbonyl, 1-naphthoxycarbonyl), C₈₋₁₂ aralkyloxycarbonyl (e.g. benzyloxycarbonyl etc.) and the like. Among them, a carboxyl group, methoxycarbonyl and ethoxycarbonyl are preferable.

Examples of a substituent for the "optionally substituted carbamoyl group" represented by Y include optionally substituted lower (C₁₋₆)alkyl (e.g. methyl, ethyl, n-propyl, isopropyl, butyl, isobutyl, sec-butyl, tert-butyl, pentyl, isopentyl, neopentyl, hexyl, isohexyl etc.), optionally substituted C₃₋₆ cycloalkyl (e.g. cyclopropyl, cyclobutyl, cyclopentyl, cyclohexyl etc.), optionally substituted C₆₋₁₄ aryl (e.g. phenyl, 1-naphthyl, 2-naphtyl etc.), optionally substituted C₇₋₁₁ aralkyl (e.g. benzyl, phenethyl etc.) and the like, and the carbamoyl group may be substituted with same or different 1 or 2 of these substituents. Examples of a substituent for the optionally substituted lower (C₁₋₆)alkyl and the optionally substituted C₃₋₆ cycloalkyl include a carboxyl group optionally esterified with lower (C₁₋₅)alkyl (e.g. methyl, ethyl, propyl, isopropyl, butyl, tert-butyl, pentyl, isopentyl, neopentyl), a 5- to 6-membered aromatic heterocyclic group containing 1 to 4 heteroatoms (e.g. furyl, thienyl, indolyl, isoindolyl, pyrazinyl, pyridyl, pyrimidyl, imidazolyl etc.), an amino group, a hydroxyl group, a phenyl group and the like, and they may be substituted with same or different 1 to 3 of these substituents. Examples of a substituent for the optionally substituted aryl group and the optionally substituted aralkyl group include a halogen atom (e.g. fluorine, chlorine, bromine, iodine), and a carboxyl group optionally esterified with a lower (C₁₋₄) alkyl group (e.g. methyl, ethyl, propyl, isopropyl, butyl, tert-butyl etc.). In addition, in the optionally substituted carbamoyl group, two substituents on a nitrogen atom may be taken together with the nitrogen atom to form a cyclic amino group, and examples of such a cyclic amino group include 1-azetidinyl, 1-pyrrolidinyl, piperidino, morpholino, 1-piperazinyl and the like. The cyclic amino group may further have a substituent.

Examples of a substituent for the "optionally substituted hydroxyl group" represented by Y include lower (C₁₋₄) alkyl (e.g. methyl, ethyl, propyl, isopropyl, butyl, tert-butyl etc.), a C₃₋₆ cycloalkyl group (e.g. cyclopropyl, cyclobutyl, cyclopentyl, cyclohexyl etc.), an optionally substituted C₆₋₁₀ aryl group (e.g. phenyl, 1-naphthyl, 2-naphthyl etc.), an optionally substituted C₇₋₁₁ aralkyl group (e.g. benzyl, phenethyl etc.) and the like. Examples of a substituent for the optionally substituted aryl group and the optionally substituted aralkyl group include a halogen atom (e.g. fluorine, chlorine, bromine, iodine), a carboxyl group optionally esterified with a lower (C₁₋₄) alkyl group (e.g. methyl, ethyl, propyl, isopropyl, butyl, tert-butyl etc.) and the like.

Examples of the "optionally substituted amino group" represented by Y include a mono-substituted or di-substituted amino group, and examples of a substituent for the amino group include lower (C₁₋₄)alkyl (e.g. methyl, ethyl, propyl, isopropyl, butyl, tert-butyl etc.), a C₃₋₆ cycloalkyl group (e.g. cyclopropyl, cyclobutyl, cyclopentyl, cyclohexyl etc.), an optionally substituted C₆₋₁₀ aryl group (e.g. phenyl, 1-naphtyl, 2-naphtyl etc.), an optionally substituted C₇₋₁₁ aralkyl group (e.g. benzyl, phenethyl etc.) and the like. Examples of a substituent for the optionally substituted aryl group and the optionally substituted aralkyl group include a halogen atom (e.g. fluorine, chlorine, bromine, iodine), and a carboxyl group optionally esterified with a lower (C₁₋₄)alkyl group (e.g. methyl, ethyl, propyl, isopropyl, butyl, tert-butyl etc.) and the like, and the optionally substituted aryl group and the optionally substituted aralkyl group may have 1 to 4, preferably 1 to 2 of these substituents. In addition, two substituents on a nitrogen atom may be taken together with the nitrogen atom to form a cyclic amino group, and examples of such a cyclic amino group include 1-azetidinyl, 1-pyrrolidinyl, piperidino, morpholino, 1-piperazinyl and the like. The cyclic amino group may further have a substituent.

Examples of the heterocyclic residue of the "optionally substituted heterocyclic residue having a hydrogen atom which can be deprotonated" represented by Y include a 5- to 7-membered (preferably 5-membered) monocyclic heterocyclic residue containing at least one of N, S and O (preferably nitrogen-containing), and the heterocyclic residue may have a hydrogen atom which is desorbed to form a proton. Examples of the heterocyclic residue include tetrazol-5-yl, a group represented by the formula: 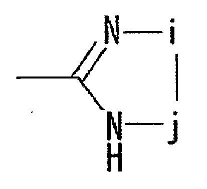 [wherein i represents -O- or -S-, and j represents >C=O, >C=S or >S(O)₂] (inter alia, 2,5-dihydro-5-oxo-1,2,4-oxadiazol-3-yl, 2,5-dihydro-5-thioxo-1,2,4-oxadiazol-3-yl, and 2,5-dihydro-5-oxo-1,2,4-thiadiazol-3-yl are preferable) and the like.

The heterocyclic residue may be protected with an optionally substituted lower alkyl group (preferably C₁₋₄ alkyl), an acyl group or the like. Examples of the optionally substituted lower alkyl group include C₁₋₄ alkyl which may be substituted with phenyl optionally substituted with C₁₋₃alkyl, nitro or C₁₋₃alkoxy, or C₁₋₃alkoxy (e.g. methyl, triphenylmethyl, methoxymethyl, ethoxymethyl, p-methoxybenzyl, p-nitrobenzyl etc.) and the like. Examples of the acyl group include lower (C₂₋₅)alkanoyl, benzoyl and the like.

Among them, X' is preferably an alkyl group substituted with an optionally esterified carboxyl group, an alkyl group substituted with an optionally substituted heterocyclic residue having a hydrogen atom which can be deprotonated, or an alkyl group substituted with an optionally substituted carbamoyl group.

In the formula (I), examples of the heterocycle represented by a ring A include the heterocyclic groups described above concerning a substituent for the hydrocarbon group represented by R₁, and inter alia, a heterocycle represented by: 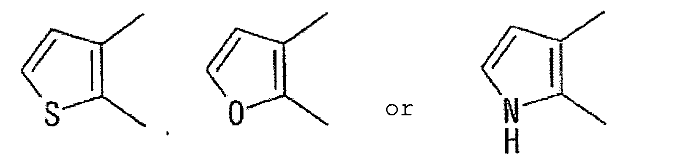 is preferable.

Examples of a substituent for the "optionally substituted benzene ring" and the "optionally substituted heterocycle" represented by a ring A include halogen (e.g. fluorine, chlorine, bromine, iodine), an optionally substituted lower alkyl group of 1 to 4 carbon atoms (e.g. methyl, ethyl, propyl, butyl, tert-butyl etc.), an optionally substituted lower alkoxy group of 1 to 4 carbon atoms (e.g. methoxy, ethoxy, propoxy, isopropoxy, butoxy, tert-butoxy etc.), a hydroxyl group, a nitro group, cyano and the like. The ring A may have 1 to 3, preferably 1 to 2 of these substituents. In addition, adjacent substituents among these substituents may be taken together to form a ring. Examples of a substituent for the optionally substituted lower alkyl group and the optionally substituted lower alkoxy group include a halogen atom (e.g. fluorine, chlorine, bromine, iodine) and the like, and the optionally substituted lower alkyl group and the optionally substituted lower alkoxy group may have 1 to 3 of these substituents at arbitrary positions. The ring A is preferably substituted with methoxy or a chlorine atom, and is particularly preferably substituted with a chlorine atom.

In the formula (Ia), examples of a substituent for the "optionally substituted benzene ring" represented by a ring B include halogen (e.g. fluorine, chlorine, bromine, iodine), an optionally substituted lower alkyl of 1 to 4 carbon atoms (e.g. methyl, ethyl, propyl, butyl, tert-butyl etc.), an optionally substituted lower alkoxy group of 1 to 4 carbon atoms (e.g. methoxy, ethoxy, propoxy, isopropoxy, butoxy, tert-butoxy etc.), a hydroxyl group, a nitro group, cyano and the like. The ring B may have 1 to 3, preferably 1 to 2 of these substituents. In addition, adjacent substituents among these substituents may be taken together to form a ring. Examples of a substituent for the optionally substituted lower alkyl group or the optionally substituted lower alkoxy group include a halogen atom (e.g. fluorine, chlorine, bromine, iodine) and the like, and the optionally substituted lower alkyl group or the optionally substituted lower alkoxy group may have 1 to 3 of these substituents at arbitrary positions. The ring B is preferably substituted with methoxy or a chlorine atom, and is particularly preferably substituted with a chlorine atom.

In the formula (I), examples of the heterocycle of the "7- or 8-membered heterocycle containing three or less heteroatoms as ring constituting atoms" represented by a ring J' include a 7- or 8-membered saturated or unsaturated heterocycle containing at least one of O, S(O)q (wherein q represents 0, 1 or 2) and N, provided that the number of heteroatoms in atoms constituting the heterocycle (ring constituting atoms) is not more than 3.

The ring J' may further have, in addition to groups represented by R₁, R₂, R₃, and X', 1 to 2 substituents at replaceable positions. Examples of such a substituent, when the substituent is bound to a nitrogen atom on the ring J', include an alkyl group (e.g. C₁₋₆ alkyl such as methyl, ethyl, n-propyl, isopropyl, n-butyl, isobutyl, tert-butyl, n-pentyl, isopentyl, neopentyl etc.), an acyl group (e.g. C₁₋₄ acyl group such as formyl, acetyl, propionyl, butyroyl etc.) and the like. The alkyl group or the acyl group may be further substituted with 1 to 5 halogen atoms (e.g. fluorine, chlorine, bromine, iodine). When the substituent is bound to a carbon atom on the ring J', examples of the substituent include oxo, thioxo, an optionally substituted hydroxyl group, an optionally substituted amino group and the like. Examples of the optionally substituted hydroxyl group and the optionally substituted amino group are the same as those of the "optionally substituted hydroxyl group" and the "optionally substituted amino group" represented by Y as defined above.

The ring J' is preferably substituted with oxo or thioxo in addition to groups represented by R₁, R₂, R₃ and X' at replaceable positions.

Examples of the fused ring consisting of the ring A and the ring J' include:

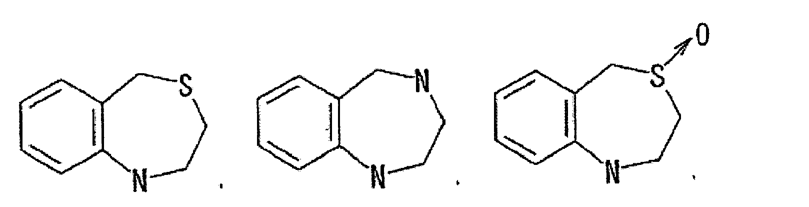

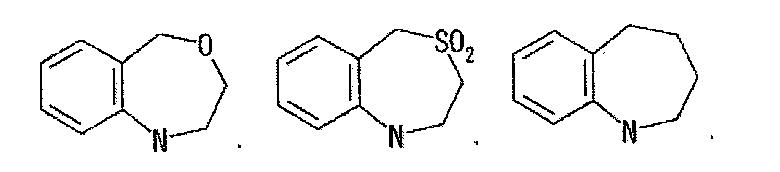

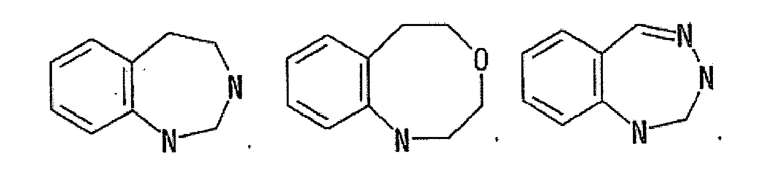

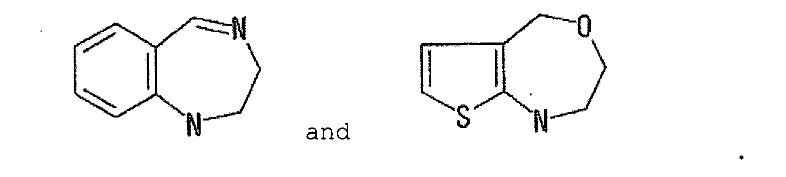

As the compound represented by the formula (I), a compound represented by the formula (I'): 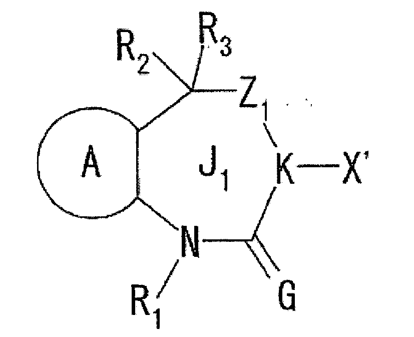 wherein R₁, R₂, R₃, X', and a ring A are as defined above, a ring J₁ represents a 7-membered heterocycle, Z₁ represents -N(R₇)-(R₇ represents a hydrogen atom, an alkyl group, or an acyl group), -S(O)_{q}- (q represents 0, 1, or 2), -CH₂- or -O-, K represents C or N, and G represents O or S, is preferable.

In the formula (I'), examples of the alkyl group represented by R₇ include a straight or branched lower alkyl group having 1 to 6 carbon atoms (e.g. methyl, ethyl, n-propyl, isopropyl, n-butyl, isobutyl, tert-butyl, n-pentyl, isopentyl, neopentyl), optionally substituted with 1 to 5 halogen atoms (e.g. fluorine, chlorine, bromine, iodine).

Examples of the acyl group represented by R₇ include a C₁₋₄ acyl group (e.g. formyl, acetyl, propionyl, butyroyl), optionally substituted with 1 to 5 halogen atoms (e.g. fluorine, chlorine, bromine, iodine).

In the formula (I'), as Z₁, S(O)_{q} (q is 0, 1 or 2), or O is preferable. In addition, as K, C is preferable and, as G, O is preferable.

As the compound represented by the formula (I'), a compound represented by the formula (I"): 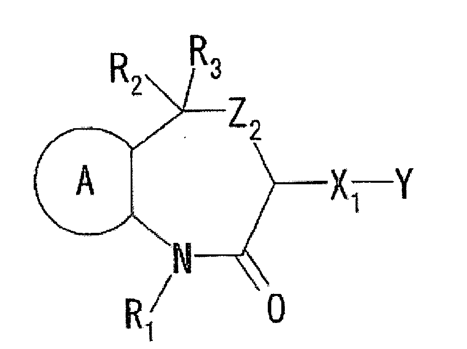 wherein, R₁, R₂, R₃, X₁, Y, and a ring A are as defined above, and Z₂ represents S(O)_{q} (q is 0, 1 or 2) or O, is preferable.

As the compound represented by the formula (I), a compound represented by the formula (Ia): 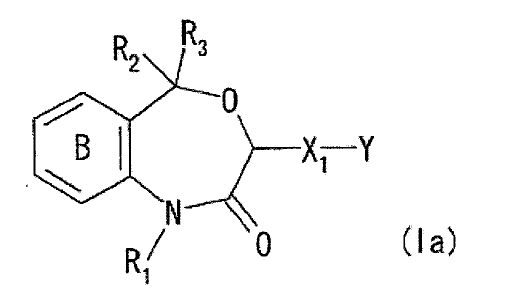 is preferable.

The compound represented by the formula (Ia) may be a compound represented by the formula (Ia'): 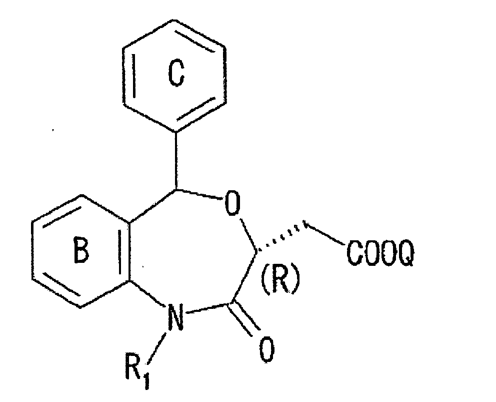 wherein R₁ and a ring B are as defined above, Q represents hydrogen or a metal ion, and a ring C represents an optionally substituted phenyl group. The formula indicates that 3- and 5-positional substituents are oriented in a reverse direction relative to a 7-membered ring plane, i.e., trans, and (R) indicates a R-configuration.

In the formula (Ia'), examples of the metal ion represented by Q include a sodium ion, a potassium ion, a calcium ion, and an aluminum ion and, inter alia, a sodium ion and a potassium ion are preferable.

Examples of a substituent for the "optionally substituted phenyl group" represented by a ring C include the same groups as those listed as the substituent of the "optionally substituted aryl group" described as an example of the "optionally substituted hydrocarbon group" as defined by R₂ and R₃.

Examples of a salt of the compound represented by the formula (I) include pharmaceutically acceptable salts such as inorganic acid salts such as hydrochloride, hydrobromide, sulfate, nitrate, phosphate and the like, organic acid salts such as acetate, tartarate, citrate, fumarate, maleate, toluenesulfonate, methanesulfonate and the like, metal salts such as a sodium salt, a potassium salt, a calcium salt, an aluminum salt and the like, base salts such as a triethylamine salt, a guanidine salt, an ammonium salt, a hydrazine salt, a quinine salt, a cinchonine salt and the like, and the like. Inter alia, a sodium salt is preferable.

Specific examples of the compound represented by the formula (I) include:
(3R,5S)-7-cyano-5-(2,3-dimethoxyphenyl)-1-neopentyl-2-oxo-1,2,3,5-tetrahydro-4,1-benzoxazepine-3-acetic acid,
(3R,5S)-7-cyano-5-(2,4-dimethoxyphenyl)-1-neopentyl-2-oxo-1,2,3,5-tetrahydro-4,1-benzoxazepine-3-acetic acid,
(3R,5S)-7-cyano-5-(2,3-methylenedioxyphenyl)-1-neopentyl-2-oxo-1,2,3,5-tetrahydro-4,1-benzoxazepine-3-acetic acid,
(3R,5S)-7-cyano-5-(2,3-ethylenedioxyphenyl)-1-neopentyl-2-oxo-1,2,3,5-tetrahydro-4,1-benzoxazepine-3-acetic acid,
(3R,5S)-7-cyano-5-(2,3-dimethoxyphenyl)-1-isobutyl-2-oxo-1,2,3,5-tetrahydro-4,1-benzoxazepine-3-acetic acid,
(3R,5S)-7-cyano-5-(2,4-dimethoxyphenyl)-1-isobutyl-2-oxo-1,2,3,5-tetrahydro-4,1-benzoxazepine-3-acetic acid,
(3R,5S)-7-cyano-5-(2,3-methylenedioxyphenyl)-1-isobutyl-2-oxo-1,2,3,5-tetrahydro-4,1-benzoxazepine-3-acetic acid,
(3R,5S)-7-cyano-5-(2,3-ethylenedioxyphenyl)-1-isobutyl-2-oxo-1,2,3,5-tetrahydro-4,1-benzoxazepine-3-acetic acid,
(3R,5S)-7-chloro-5-(2,3-dimethoxyphenyl)-1-neopentyl-2-oxo-1,2,3,5-tetrahydro-4,1-benzoxazepine-3-acetic acid,
(3R,5S)-7-chloro-5-(2,4-dimethoxyphenyl)-1-neopentyl-2-oxo-1,2,3,5-tetrahydro-4,1-benzoxazepine-3-acetic acid,
(3R,5S)-7-chloro-5-(2,4-methylenedioxyphenyl)-1-neopentyl-2-oxo-1,2,3,5-tetrahydro-4,1-benzoxazepine-3-acetic acid,
(3R,5S)-7-chloro-5-(2,4-ethylenedioxyphenyl)-1-neopentyl-2-oxo-1,2,3,5-tetrahydro-4,1-benzoxazepine-3-acetic acid,
(3R,5S)-7-chloro-5-(2,3-dimethoxyphenyl)-1-isobutyl-2-oxo-1,2,3,5-tetrahydro-4,1-benzoxazepine-3-acetic acid,
(3R,5S)-7-chloro-5-(2,4-dimethoxyphenyl)-1-isobutyl-2-oxo-1,2,3,5-tetrahydro-4,1-benzoxazepine-3-acetic acid,
(3R,5S)-7-chloro-5-(2,3-methylenedioxyphenyl)-1-isobutyl-2-oxo-1,2,3,5-tetrahydro-4,1-benzoxazepine-3-acetic acid,
(3R,5S)-7-chloro-5-(2,3-ethylenedioxyphenyl)-1-isobutyl-2-oxo-1,2,3,5-tetrahydro-4,1-benzoxazepine-3-acetic acid,
(3R,5S)-7-cyano-5-(2,3-dimethoxyphenyl)-1-neopentyl-2-oxo-1,2,3,5-tetrahydro-4,1-benzothiazepine-3-acetic acid,
(3R,5S)-7-cyano-5-(2,4-dimethoxyphenyl)-1-neopentyl-2-oxo-1,2,3,5-tetrahydro-4,1-benzothiazepine-3-acetic acid,
(3R,5S)-7-cyano-5-(2,3-methylenedioxyphenyl)-1-neopentyl-2-oxo-1,2,3,5-tetrahydro-4,1-benzothiazepine-3-acetic acid,
(3R,5S)-7-cyano-5-(2,3-ethylenedioxyphenyl)-1-neopentyl-2-oxo-1,2,3,5-tetrahydro-4,1-benzothiazepine-3-acetic acid,
(3R,5S)-7-cyano-5-(2,3-dimethoxyphenyl)-1-isobutyl-2-oxo-1,2,3,5-tetrahydro-4,1-benzothiazepine-3-acetic acid,
(3R,5S)-7-cyano-5-(2,4-dimethoxyphenyl)-1-isobutyl-2-oxo-1,2,3,5-tetrahydro-4,1-benzothiazepine-3-acetic acid,
(3R,5S)-7-cyano-5-(2,3-methylenedioxyphenyl)-1-isobutyl-2-oxo-1,2,3,5-tetrahydro-4,1-benzothiazepine-3-acetic acid,
(3R,5S)-7-cyano-5-(2,3-ethylenedioxyphenyl)-1-isobutyl-2-oxo-1,2,3,5-tetrahydro-4,1-benzothiazepine-3-acetic acid,
(3R,5S)-7-chloro-5-(2,3-dimethoxyphenyl)-1-neopentyl-2-oxo-1,2,3,5-tetrahydro-4,1-benzothiazepine-3-acetic acid,
(3R,5S)-7-chloro-5-(2,4-dimethoxyphenyl)-1-neopentyl-2-oxo-1,2,3,5-tetrahydro-4,1-benzothiazepine-3-acetic acid,
(3R,5S)-7-chloro-5-(2,3-methylenedioxyphenyl)-1-neopentyl-2-oxo-1,2,3,5-tetrahydro-4,1-benzothiazepine-3-acetic acid,
(3R,5S)-7-chloro-5-(2,3-ethylenedioxyphenyl)-1-neopentyl-2-oxo-1,2,3,5-tetrahydro-4,1-benzothiazepine-3-acetic acid,
(3R,5S)-7-chloro-5-(2,3-dimethoxyphenyl)-1-isobutyl-2-oxo-1,2,3,5-tetrahydro-4,1-benzothiazepine-3-acetic acid,
(3R,5S)-7-chloro-5-(2,4-dimethoxyphenyl)-1-isobutyl-2-oxo-1,2,3,5-tetrahydro-4,1-benzothiazepine-3-acetic acid,
(3R,5S)-7-chloro-5-(2,3-methylenedioxyphenyl)-1-isobutyl-2-oxo-1,2,3,5-tetrahydro-4,1-benzothiazepine-3-acetic acid, (3R,5S)-7-chloro-5-(2,3-ethylenedioxyphenyl)-1-isobutyl-2-oxo-1,2,3,5-tetrahydro-4,1-benzothiazepine-3-acetic acid,
(3R,5S)-7-cyano-5-(2-chlorophenyl)-1-neopenyl-2-oxo-1,2,3,5-tetrahydro-4,1-benzoxazepine-3-acetic acid,
(3R,5S)-7-cyano-5-(2-chlorophenyl)-1-isobutyl-2-oxo-1,2,3,5-tetrahydro-4,1-benzoxazepine-3-acetic acid,
(3R,5S)-7-chloro-5-(2-chlorophenyl)-1-neopentyl-2-oxo-1,2,3,5-tetrahydro-4,1-benzoxazepine-3-acetic acid,
(3R,5S)-7-chloro-5-(2-chlorophenyl)-1-isobutyl-2-oxo-1,2,3,5-tetrahydro-4,1-benzoxazepine-3-acetic acid,
(3R,5S)-7-cyano-5-(2-chlorophenyl)-1-neopentyl-2-oxo-1,2,3,5-tetrahydro-4,1-benzothiazepine-3-acetic acid,
(3R,5S)-7-cyano-5-(2-chlorophenyl)-1-isobutyl-2-oxo-1,2,3,5-tetrahydro-4,1-benzothiazepine-3-acetic acid,
(3R,5S)-7-chloro-5-(2-chlorophenyl)-1-neopentyl-2-oxo-1,2,3,5-tetrahydro-4,1-benzothiazepine-3-acetic acid,
(3R,5S)-7-chloro-5-(2-chlorophenyl)-1-isobutyl-2-oxo-1,2,3,5-tetrahydro-4,1-benzothiazepine-3-acetic acid,
(3R,5S)-7-chloro-5-(4-ethoxy-2-methoxyphenyl)-1-neopentyl-2-oxo-1,2,3,5-tetrahydro-4,1-benzoxazepine-3-acetic acid,
(3R)-7-chloro-5-(2-chlorophenyl)-2,3-dihydro-1-isobutyl-2-oxo-1H-1,4-benzodiazepine-3-acetic acid,
(3R,5S)-7-chloro-5-(2-chlorophenyl)-2,3,4,5-tetrahydro-1-isobutyl-2-oxo-1H-1,4-benzodiazepine-3-acetic acid,
N-[[(3R,5S)-7-chloro-5-(2-chlorophenyl)-1-neopentyl-2-oxo-1,2,3,5-tetrahydro-4,1-benzothiazepine-3-yl]-acetyl]glycine,
(3R,5S)-7-chloro-5-(2-chlorophenyl)-3-dimethylaminocarbonylmethyl-1-neopentyl-2-oxo-1,2,3,5-tetrahydro-4,1-benzothiazepine,
7-chloro-5-(2-chlorophenyl)-1-isobutyl-2-oxo-2,3,4,5-tetrahydro-1H-[1]-benzazepine-3-acetic acid,
(3R,5S)-7-chloro-5-(2-chlorophenyl)-1-neopentyl-1,2,3,5-tetrahydro-2-thioxo-4,1-benzoxazepine-3-acetic acid,
(3R,5S)-7-chloro-5-(2-chlorophenyl)-1-neopentyl-2-oxo-1,2,3,5-tetrahydro-4,1-thieno[2,3-e]oxazepine-3-acetic acid,
(3R,5S)-7-chloro-5-(2-methoxyphenyl)-1-neopentyl-2-oxo-1,2,3,5-tetrahydro-4,1-thieno[2,3-e]oxazepine-3-acetic acid,
(3R,5S)-7-chloro-1-isobutyl-5-(2-methoxyphenyl)-2-oxo-1,2,3,5-tetrahydro-4,1-thieno[2,3-e]oxazepine-3-acetic acid,
(3R,5S)-7-chloro-5-(3-hydroxy-2-methoxyphenyl)-1-neopentyl-2-oxo-1,2,3,5-tetrahydro-4,1-benzothiazepine-3-acetic acid,
(3R,5S)-7-chloro-5-(4-hydroxy-2-methoxyphenyl)-1-neopentyl-2-oxo-1,2,3,5-tetrahydro-4,1-benzothiazepine-3-acetic acid,
(3R,5S)-7-chloro-5-(3-hydroxy-2-methoxyphenyl)-1-isobutyl-2-oxo-1,2,3,5-tetrahydro-4,1-benzothiazepine-3-acetic acid,
(3R,5S)-7-chloro-5-(4-hydroxy-2-methoxyphenyl)-1-isobutyl-2-oxo-1,2,3,5-tetrahydro-4,1-benzothiazepine-3-acetic acid,
(3R,5S)-7-chloro-5-(3-ethoxy-2-methoxyphenyl)-1-neopentyl-2-oxo-1,2,3,5-tetrahydro-4,1-benzothiazepine-3-acetic acid,
(3R,5S)-7-chloro-5-(4-ethoxy-2-methoxyphenyl)-1-neopentyl-2-oxo-1,2,3,5-tetrahydro-4,1-benzothiazepine-3-acetic acid,
(3R,5S)-7-chloro-5-(3-ethoxy-2-methoxyphenyl)-1-isobutyl-2-oxo-1,2,3,5-tetrahydro-4,1-benzothiazepine-3-acetic acid,
(3R,5S)-7-chloro-5-(4-ethoxy-2-methoxyphenyl)-1-isobutyl-2-oxo-1,2,3,5-tetrahydro-4,1-benzothiazepine-3-acetic acid,
(3R,5S)-7-chloro-5-(2-chloro-3-methoxyphenyl)-1-neopentyl-2-oxo-1,2,3,5-tetrahydro-4,1-benzothiazepine-3-acetic acid,
(3R,5S)-7-chloro-5-(2-chloro-4-methoxyphenyl)-1-neopentyl-2-oxo-1,2,3,5-tetrahydro-4,1-benzothiazepine-3-acetic acid,
(3R,5S)-7-chloro-5-(2-chloro-3-methoxyphenyl)-1-isobutyl-2-oxo-1,2,3,5-tetrahydro-4,1-benzothiazepine-3-acetic acid,
(3R,5S)-7-chloro-5-(2-chloro-4-methoxyphenyl)-1-isobutyl-2-oxo-1,2,3,5-tetrahydro-4,1-benzothiazepine-3-acetic acid,
(3R,5S)-7-chloro-5-(2-chloro-3-hydroxyphenyl)-1-neopentyl-2-oxo-1,2,3,5-tetrahydro-4,1-benzothiazepine-3-acetic acid,
(3R,5S)-7-chloro-5-(2-chloro-4-hydroxyphenyl)-1-neopentyl-2-oxo-1,2,3,5-tetrahydro-4,1-benzothiazepine-3-acetic acid,
(3R,5S)-7-chloro-5-(2-chloro-3-hydroxyphenyl)-1-isobutyl-2-oxo-1,2,3,5-tetrahydro-4,1-benzothiazepine-3-acetic acid,
(3R,5S)-7-chloro-5-(2-chloro-4-hydroxyphenyl)-1-isobutyl-2-oxo-1,2,3,5-tetrahydro-4,1-benzothiazepine-3-acetic acid, and salts thereof.

The compound represented by the general formula (I) and a salt thereof [hereinafter, the compound and a salt thereof are simply referred to as compound (I) in some cases] are disclosed, for example, in EPA567026, WO 95/21834 (Japanese Patent Application No. 6-15531), EPA645377 (Japanese Patent Application No. 6-229159), and EPA645378 (Japanese Patent Application No. 6-229160), and can be produced according to the disclosure of these gazettes.

As the compound represented by the formula (I), a compound represented by the formula (Ib): 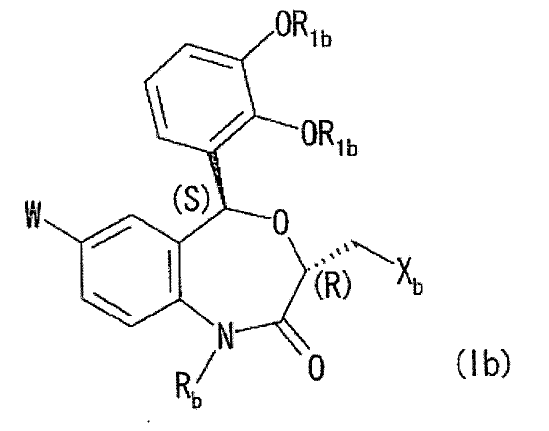 is preferable.

As the compound represented by the formula (Ib), a compound in which R_{b} is C₁₋₆alkyl optionally having 1 to 3 substituents selected from acetyloxy, propionyloxy, t-butoxycarbonyloxy, palmitoyloxy, dimethylaminoacetyloxy and 2-aminopropoionyloxy;
a compound in which R_{b} is branched C₃₋₆ alkyl optionally having 1 to 3 substituents selected from acetyloxy, propionyloxy, t-butoxycarbonyloxy, palmitoyloxy, dimethylaminoacetyloxy and 2-aminopropoionyloxy;
a compound in which R_{b} is 2,2,-dimethyl-3-hydroxypropyl, 3-hydroxy-2-hydroxymethyl-2-methylpropyl, 3-acetoxy-2,2-dimethylpropyl, 3-acetoxy-2-hydroxymethyl-2-methylpropyl or 3-acetoxy-2-acetoxymethyl-2-methylpropyl;
a compound in which R_{1b} is methyl;
a compound in which W is a chlorine atom;
a compound in which X_{b} is a group represented by the formula: 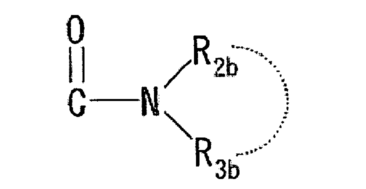 wherein R_{2b} and R_{3b} each are a hydrogen atom, an optionally substituted hydrocarbon group, an optionally substituted heterocycle or an acyl group, or R_{2b} and R_{3b} are taken together with an adjacent nitrogen atom to form an optionally substituted 5- or 6-membered nitrogen-containing heterocycle optionally containing 1 to 3 heteroatoms selected from a nitrogen atom, a sulfur atom and an oxygen atom as a ring constituting atom;
a compound in which in a group represented by X_{b},
R_{2b} represents a hydrogen atom or a C₁₋₇alkyl group,
R_{3b} represents an acyl group selected form:
(1) a hydrocarbon group selected from (a) C₁₋₇alkyl, (b) C₃₋₇ cycloalkyl, (c) C₂₋₆alkenyl, (d) C₆₋₁₀ aryl and (e) C₆₋₁₀ aryl-C₁₋₄ alkyl, wherein (a) C₁₋₇alkyl, (b) C₃₋₇ cycloalkyl, and (c) C₂₋₆ alkenyl each may have 1 to 4 substituents selected from:
   (i) a carboxyl group optionally esterified with C₁₋₆ alkyl or C₆₋₁₀ aryl-C₁₋₄ alkyl,
   (ii) a phosphoric acid group optionally mono- or di-substituted with C₁₋₆ alkyl or C₂₋₇ alkanoyloxy-C₁₋₆ alkyl,
   (iii) a sulfonic acid group,
   (iv) a sulfonamide group optionally substituted with C₁₋₆ alkyl or C₈₋₁₀ aryl-C₁₋₄ alkyl,
   (v) a hydroxy group optionally alkylated with C₁₋₃ alkyl,
   (vi) a sulfhydryl group optionally alkylated with C₁₋₃ alkyl,
   (vii) a carbamoyl group,
   (viii) a phenyl group optionally substituted with 1 to 5 substituents selected from a hydroxy group, a chlorine atom, a fluorine atom, aminosulfonyl, and an amino group optionally mono- or di-substituted with C₁₋₃ alkyl,
   (ix) an amino group optionally mono- or di-substituted with C₁₋₃ alkyl,
   (x) a cyclic amino group optionally substituted with C₁₋₃ alkyl, benzyl or phenyl, which is derived from piperidine, pyrrolidine, morpholine, thiomorpholine, piperazine, 4-methylpiperazine, 4-benzylpiperazine, 4-phenylpiperazine, 1,2,3,4-tetrahydroisoquinoline or phthalimide, and
   (xi) an aromatic 5- to 6-membered heterocyclic group derived from pyridine, imidazole, indole or tetrazole, (d) C₆₋₁₀ aryl and (e) C₆₋₁₀ aryl-C₁₋₄ alkyl each may have 1 to 4 substituents selected from:
      (i) a carboxyl group optionally esterified with C₁₋₄ alkyl,
      (ii) a phosphoric acid group optionally mono- or di-substituted with C₁₋₆ alkyl or C₂₋₇ alkanoyloxy-C₁₋₆ alkyl,
      (iii) a sulfonic acid group,
      (iv) C₁₋₄ alkylsulfonyl, C₆₋₁₀ arylsulfonyl or C₆₋₁₀ aryl-C₁₋₄ alkylsulfonyl,
      (v) a sulfonamide optionally substituted with C₁₋₆ alkyl or C₆₋₁₀ aryl-C₁₋₄ alkyl,
      (vi) a carboxyl group optionally esterified with C₁₋₄ alkyl, a phosphoric acid group optionally mono- or di-substituted with C₁₋₆ alkyl, a sulfonic acid group, C₁₋₄ alkylsulfonyl, _{C6-10} arylsulfonyl or C₆₋₁₀ aryl-C₁₋₄ alkylsulfonyl group, C₁₋₆ alkyl, or a C₁₋₃ alkyl group optionally substituted with a sulfonamido group optionally substituted with C₆₋₁₀ aryl-C₁₋₄ alkyl, and
      (vii) halogen,
(2) tetrazolyl, 4,5-dihydro-5-oxo-1,2,4-oxadiazolyl, 4,5-dihydro-5-thioxo-1,2,4-oxadiazolyl, 2,3-dihydro-3-oxo-1,2,4-oxadiazolyl, 2,3-dihydro-3-thioxo-1,2,4-oxadiazolyl, 3,5-dioxo-1,2,4-oxadiazolidinyl, 4,5-dihydro-5-oxo-isoxazolyl, 4,5-dihydro-5-thioxo-isoxazolyl, 2,3-dihydro-2-oxo-1,3,4-oxadiazolyl, 2,3-dihydro-4-oxo-1,2,4-tetrazolyl or 2,3-dihydro-3-thioxo-1,2,4-tetrazolyl group, or
(3) (i) a C₂₋₇alkanoyl group optionally substituted with 1 or 2 halogens, and (ii) a C₆₋₁₀ arylsufonyl group, a C₁₋₄ alkylsulfonyl group or a C₆₋₁₀ aryl-C₁₋₄ alkylsulfonyl group optionally substituted with 1 to 4 substituents selected from C₁₋₃ alkyl, C₁₋₃ alkoxy and halogen,
   or R_{2b} and R_{3b} are taken together with an adjacent nitrogen atom to form a 5-membered or 6-membered ring derived from piperidine, piperazine, pyrrolidine, 2-oxopiperazine, 2,6-dioxopiperazine, morpholine or thiomorpholine, wherein the 5-membered or 6-membered ring may have 1 to 4 substituents selected from:
   (A) a hydroxy group optionally substituted with C₁₋₃alkyl or C₂₋₇ alkanoyl,
   (B) a carboxyl group optionally esterified with C₁₋₆ alkyl or C₆₋₁₀ aryl-C₁₋₄ alkyl,
   (C) a phosphoric acid group optionally mono- or di-substituted with C₁₋₆ alkyl or C₂₋₇ alkanoyloxy-C₁₋₆ alkyl,
   (D) a sulfonic acid group,
   (E) a sulfonamido group optionally substituted with C₁₋₆ alkyl or C₆₋₁₀ aryl-C₁₋₄ alkyl,
   (F) a carboxyl group optionally esterified with C₁₋₆alkyl or C₆₋₁₀ aryl-C₁₋₄ alkyl, a phosphoric acid group optionally mono- or di-substituted with C₁₋₆ alkyl or C₂₋₇ alkanoyloxy-C₁₋₆ alkyl, a sulfonic acid group, a sulfonamido group optionally substituted with C₁₋₆ alkyl or C₆₋₁₀ aryl-C₁₋₄ alkyl, a hydroxy group optionally substituted with C₁₋₃ alkyl or C₂₇ alkanoyl, a sulfhydryl group optionally alkylated with C₁₃ alkyl, a carbamoyl group, and phenyl optionally substituted with 1 to 5 substituents selected from a hydroxy group, a halogen, aminosulfonyl and an amino group optionally substituted with C₁₋₃ alkyl, an amino group optionally mono- or di-substituted with C₁₋₃ alkyl, or C₁₋₆ alkyl and C₂₋₅ alkenyl optionally substituted with tetrazolyl,
   (G) an amino group optionally mono- or di-substituted with C₁₋₃ alkyl,
   (H) a cyclic amino group derived from piperidine, pyrrolidine, morpholine, thiomorpholine, 4-methylpiperazine, 4-benzylpiperazine, or 4-phenylpiperazine,
   (I) a cyano group,
   (J) a carbamoyl group,
   (K) an oxo group,
   (L) tetrazolyl or a 2,5-dihydro-5-oxo-1,2,4-oxadiazolyl group,
   (M) a carbamoyl group optionally substituted with C₆₋₁₀ arylsulfonyl, C₁₋₄ alkylsulfonyl or C₆₋₁₀ aryl-C₁₋₄ alkylsulfonyl,
   (N) a sulfhydryl group optionally alkylated with C₁₋₃ alkyl, and
   (O) a phenyl group optionally substituted with 1 to 5 substituents selected form a hydroxy group, halogen, aminosulfonyl, and an amino group optionally substituted with C₁₋₃ alkyl;
   a compound in which, in a group represented by X_{b}, R_{2b} and R_{3b} are taken together with a nitrogen atom of an adjacent carbamoyl group to form a 5-membered or 6-membered ring derived from piperidine, piperazine, pyrrolidine, 2-oxopiperazine or 2,6-dioxopiperazine, wherein the 5-membered or 6-membered ring each is a ring optionally substituted with a C₁₋₆ alkyl group optionally having 1 to 2 substituents selected from :
   (i) a carboxyl group optionally esterified with C₁₋₆ alkyl or C₆₋₁₀ aryl-C₁₋₄ alkyl,
   (ii) a phosphoric acid group optionally mono- or di-substituted with C₁₋₆ alkyl or C₂₋₇ alkanoyl-C₁₋₆ alkyl,
   (iii) a sulfonic acid group,
   (iv) a sulfonamido group optionally substituted with C₁₋₆ alkyl or C₆₋₁₀ aryl-C₁₋₄ alkyl,
   (v) a hydroxy group optionally alkylated with C₁₋₃ alkyl,
   (vi) a sulfhydryl group optionally alkylated with C₁₋₃ alkyl,
   (vii) a carbamoyl group,
   (viii) a phenyl group optionally substituted with 1 to 5 substituents selected from a hydroxy group, halogen, aminosulfonyl, and an amino group optionally substituted with C₁₋₃ alkyl,
   (ix) an amino group optionally mono- or di-substituted with C₁₋₃alkyl, and
   (x) a tetrazolyl group;
      a compound in which, in a group represented by X_{b}, R_{2b} is a hydrogen atom or C₁₋₇ alkyl, and R_{3b} is C₁₋₄ alkylsulfonyl;
      a compound in which a heterocyclic group represented by X_{b} is tetrazolyl, 4,5-dihydro-5-oxo-1,2,4-oxadiazolyl, 4,5-dihydro-5-thioxo-1,2,4-oxadiazolyl, 2,3-dihydro-3-oxo-1,2,4-oxadiazolyl, 2,3-dihydro-3-thioxo-1,2,4-oxadiazolyl, 3,5-dioxo-1,2,4-oxadiazolydium, 4,5-dihydro-5-oxo-isoxazolyl, 4,5-dihydro-5-thioxo-isoxazolyl, 2,3-dihydro-2-oxo-1,3,4-oxadiazolyl, 2,3-dihydro-3-oxo-1,2,4-tetrazolyl or 2,3-dihydro-3-thioxo-1,2,4-tetrazolyl;
      a compound in which R_{1b} is methyl,
      W is a chlorine atom,
      R_{b} is branched C₃₋₆ alkyl substituted with 1 to 3 substituents selected from a hydroxy group, acetyloxy, propionyloxy, tert-butoxycarbonyloxy, palmitoyloxy, dimethylaminoacetyloxy and 2-aminopropionyloxy,
      X_{b} is a group represented by the formula: 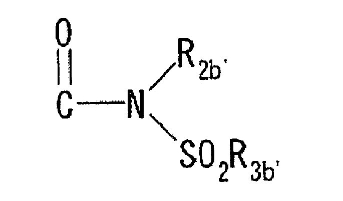
wherein R_{2b'} represents a hydrogen atom or C₁₋₇alkyl, and
R_{3b}, represents C₁₋₄ alkyl;
a compound in which R_{1b} is methyl,
W is a chlorine atom,
R_{b} is branched C₃₋₆ alkyl substituted with 1 to 3 substituents selected from a hydroxy group, acetyloxy, propionyloxy, tert-butoxycarbonyloxy, palmitoyloxy, dimethylaminoacetyloxy and 2-aminopropionyloxy,
X_{b} is a group represented by the formula: 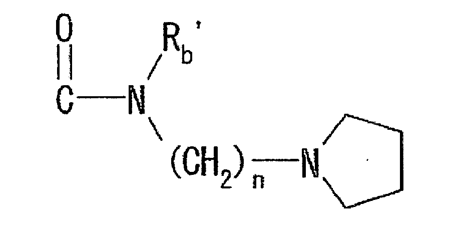 wherein R_{b}' represents a hydrogen atom or C₁₋₇alkyl, and n represents an integer of 1 to 5;
a compound in which R_{1b} is methyl,
W is a chlorine atom,
R_{b} is branched C₃₋₆ alkyl substituted with 1 to 3 substituents selected from a hydroxy group, acetyloxy, propionyloxy, tert-butoxycarbonyloxy, palmitoyloxy, dimethylaminoacetyloxy and 2-aminopropionyloxy,
X_{b} is a group represented by the formula: 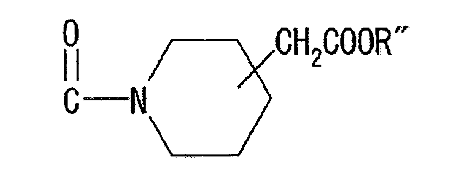 wherein R" represents a hydrogen atom or C₁₋₄ alkyl;
a compound in which R_{1b} is methyl,
W is a chlorine atom,
R_{b} is branched C₃₋₆ alkyl substituted with 1 to 3 substituents selected from a hydroxy group, acetyloxy, propionyloxy, tert-butoxycarbonyloxy, palmitoyloxy, dimethylaminoacetyloxy and 2-aminopropionyloxy,
X_{b} is tetrazolyl;
a compound in which R_{b} is lower alkyl optionally substituted with 1 or 2 hydroxy groups,
X_{b} is:
(1) a carbamoyl group optionally substituted with a hydrocarbon group selected from (a) C₁₋₇ alkyl, (b) C₃₋₇ cycloalkyl, (c) C₂₋₆ alkenyl, (d) C₆₋₁₀ aryl and (e) C₇₋₁₄ aryloxy, wherein the (a) C₁₋₇ alkyl, the (b) C₃₋₇ cycloalkyl, and the (c) C₂₋₅ alkenyl each may have 1 to 4 substituents selected from:
   (i) a carboxyl group optionally esterified with C₁₋₆ alkyl or C₇₋₁₀ arylalkyl,
   (ii) a phosphoric acid group,
   (iii) a sulfonic acid group,
   (iv) a sulfonamido optionally substituted with C₁₋₆ alkyl or C₇₋₁₀ arylalkyl,
   (v) a hydroxy group optionally alkylated with C₁₋₃alkyl,
   (vi) a sulfhydryl group optionally alkylated with C₁₋₃ alkyl,
   (vii) a carbamoyl group,
   (viii) a phenyl group optionally substituted with a substituent selected from a hydroxy group, a chlorine atom, a fluorine atom, aminosulfonyl, and an amino group optionally mono- or di-substituted with C₁₋₃ alkyl,
   (ix) an amino group optionally mono- or di-substituted with C₁₋₃alkyl,
   (x) a cyclic amino group optionally substituted with C₁₋₃ alkyl, benzyl or phenyl, which is derived from piperidine, pyrrolidine, morpholine, thiomorpholine, piperazine, 4-methylpiperazine, 4-benzylpiperazine or 4-phenylpiperazine, and
   (xi) an aromatic 5- to 6-membered heterocyclic group derived from pyridine, imidazole, indole or tetrazole, the (d) C₆₋₁₀ aryl and the (e) C₇₋₁₄ arylalkyl each may have 1 to 4 substituents selected from:
      (i) a carboxyl group optionally esterified with C₁₋₄ alkyl,
      (ii) a phosphoric acid group,
      (iii) a sulfonic acid group,
      (iv) a sulfonamido optionally substituted with C₁₋₆ alkyl or C₇₋₁₀ arylalkyl,
      (v) a C₁₋₃ alkyl group optionally substituted with a carboxyl group optionally esterified with C₁₋₄ alkyl, a phosphoric acid group, a sulfonic acid group, C₁₋₆ alkyl, or a sulfonamido group optionally substituted with C₇₋₁₀ arylalkyl, or
      (vi) a halogen atom,
(2) tetrazolyl, 4,5-dihydro-5-oxo-1,2,4-oxadiazolyl, 4,5-dihydro-5-thioxo-1,2,4-oxadiazolyl, 2,3-dihydro-3-oxo-1,2,4-oxadiazolyl, 2,3-dihydro-3-thioxo-1,2,4-oxadiazolyl, 3,5-dioxo-1,2,4-oxadiazolidinyl, 4,5-dihydro-5-oxo-isoxazolyl, 4,5-dihydro-5-thioxo-osoxazolyl, 2,3-dihydro-2-oxo-1,3,4-oxadiazolyl, 2,3-dihydro-3-oxo-1,2,4-tetrazolyl or 2,3-dihydro-3-thioxo-1,2,4-tetrazolyl group,
(3) carbamoyl optionally substituted with an acyl group selected from (i) a C₂₋₇ alkanoyl group optionally substituted with 1 or 2 halogens, and (ii) a C₆₋₁₀ arylsulfonyl group, a C₁₋₄ alkylsulfonyl group or a C₇₋₁₄ arylalkylsulfonyl group optionally substituted with 1 to 4 substituents selected from C₁₋₃ alkyl, C₁₋₃ alkoxy and halogen, or
(4) a cyclic aminocarbonyl group derived from piperidine, piperazine, pyrrolidine, 2-oxopiperazine, 2,6-dioxopiperazine, morpholine and thiomorpholine, wherein the cyclic amino group may have 1 to 4 substituents selected from:
   (A) a hydroxyl group,
   (B) a carboxyl group optionally esterified with C₁₋₄ alkyl,
   (C) a phosphoric acid group,
   (D) a sulfonic acid group,
   (E) a sulfonamido group optionally substituted with C₁₋₆ alkyl or C₇₋₁₀ arylalkyl,
   (F) C₁₋₃ alkyl or C₂₋₅alkenyl optionally substituted with the (A), (B), (C), (D) or (E),
   (G) an amino group optionally sub- or di-substituted with C₁₋₃ alkyl,
   (H) a cyclic amino group derived from piperidine, pyrrolidine, morpholine, thiomorpholine, 4-methylpiperazine, 4-benzylpiperazine or 4-phenylpiperazine,
   (I) a cyano group,
   (J) a carbamoyl group,
   (K) oxo,
   (L) C₁₋₃ alkoxy,
   (M) a heterocyclic group derived from tetrazolyl or 2,5-dihydro-5-oxo-1,2,4-oxadiazolyl,
   (N) a carbamoyl group optionally substituted with C₆₋₁₀ arylsulfonyl, C₁₋₄ alkylsulfonyl or C₇₋₁₄ arylalkylsulfonyl; a compound in which R_{b} is 2,2-dimethyl-3-hydroxypropyl group; are preferable.

In the aforementioned formulas, examples of the lower alkyl group represented by R_{b} include C₁₋₆ alkyl such as methyl, ethyl, n-propyl, isopropyl, n-butyl, isobutyl, n-pentyl, isopentyl, neopentyl, hexyl and the like. Among them, a C₃₋₆ alkyl group is preferable, and C₄₋₅ alkyl is more preferable. Inter alia, a branched C₄₋₅ alkyl group such as isobutyl, neopentyl and the like is preferable.

Examples of a substituent for the lower alkyl represented by R_{b} include a hydroxy group optionally substituted with C₂₋₂₀ alkanoyl or C₁₋₇ alkyl. Examples of such a substituent include a hydroxy group, acetyloxy, propionyloxy, tert-butoxycarbonyloxy, palmitoyloxy, dimethylaminoacetyloxy and 2-aminopropionyloxy.

The group may be substituted with 1 to 3 of such substituents at replaceable positions.

Further, examples of the lower alkyl optionally substituted with R_{b} include 2,2-dimethyl-3-hydroxypropyl, 3-hydroxy-2-hydroxymethyl-2-methylpropyl, 3-acetoxy-2,2-dimethylpropyl, 3-acetoxy-2-hydroxymethyl-2-methyl-propyl and 3-acetoxy-2-acetoxymethyl-2-methylpropyl.

Examples of the optionally substituted carbamoyl group represented by X_{b} include a group represented by the formula:

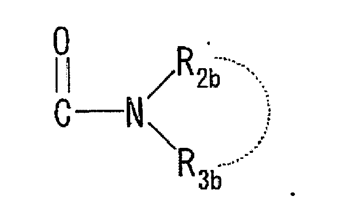

Examples of the "optionally substituted hydrocarbon" represented by R_{2b} and R_{3b} include an optionally substituted C₁₋₇ straight or branched alkyl group (e.g. methyl, ethyl, n-propyl, isopropyl, n-butyl, isobutyl, 1,1-dimethylethyl, n-pentyl, 3-methylbutyl, 2-methylbutyl, 1-methylbutyl, 1-ethylpropyl, n-hexyl, 4-methylpentyl, 3-methylpentyl, 2-methylpentyl, 2-ethylbutyl, 1-ethylbutyl, neopentyl, hexyl, heptyl), an optionally substituted C₃₋₇ cycloalkyl group (e.g. cyclopropyl, cyclobutyl, cyclopentyl, cyclohexyl, cyclohexylmethyl etc.), an optionally substituted straight or branched C₂₋₆ alkenyl group (e.g. vinyl, allyl, isopropenyl, 2-methylallyl, 1-propenyl, 2-methyl-1-propenyl, 2-methyl-2-propenyl, 1-butenyl, 2-butenyl, 3-butenyl, 2-ethyl-1-butenyl, 2-methyl-2-butenyl, 3-methyl-2-butenyl, 1-pentenyl, 2-pentenyl, 3-pentenyl, 4-pentenyl, 4-methyl-3-pentenyl, 1-hexenyl, 2-hexenyl, 3-hexenyl, 4-hexenyl, 5-hexenyl etc.), an optionally substituted C₆₋₁₀ aryl group (e.g. phenyl, naphthyl group) and an optionally substituted C₇₋₁₄ arylalkyl group (e.g. benzyl, phenethyl, naphthylmethyl).

Examples of a substituent for the "optionally substituted C₁₋₇ straight or branched alkyl group, optionally substituted C₃₋₇ cycloalkyl group, C₂₋₆ straight or branched alkenyl group" include a carboxyl group optionally esterified with a C₁₋₆ alkyl group or a C₆₋₁₀ aryl-C₁₋₄ alkyl group (e.g. methyl, ethyl, propyl, isopropyl, butyl, tert-butyl, phenyl, benzyl etc.), a phosphoric acid group optionally mono- or di-substituted with C₁₋₆alkyl (e.g. methyl, ethyl, n-propyl, isopropyl, n-butyl, isobutyl, n-pentyl, isopentyl, neopentyl, hexyl etc.) or C₂₋₇ alkanoyloxy-C₁₋₆ alkyl such as acetyloxymethyl and pivaloyloxymethyl, a sulfonamido group optionally substituted with a C₁₋₈ alkyl group or a C₆₋₁₀ aryl-C₁₋₄ alkyl group (e.g. methyl, ethyl, propyl, isopropyl, butyl, tert-butyl, benzyl etc.), a hydroxy group and a sulfhydryl group optionally alkylated with a C₁₋₃ alkyl group (e.g. methyl, ethyl, propyl etc.), a carbamoyl group, a phenyl optionally substituted with 1 to 5 substituents [e.g., a hydroxy group, chlorine, fluorine, an aminosulfonyl group, an amino group optionally substituted with a C₁₋₃ alkyl group (e.g. methyl, ethyl, propyl etc.)], an amino group optionally mono- or di-substituted with a C₁₋₃ alkyl group (e.g. methyl, ethyl, propyl etc.), a cyclic amino group (e.g. a 5- to 6-membered cyclic amino group optionally substituted with C₁₋₃ alkyl, benzyl, phenyl etc., and optionally containing an oxygen atom and a sulfur atom as a ring constituting atom, which is derived from a cyclic amine such piperidine, pyrrolidine, morpholine, thiomorpholine, piperazine, 4-methylpiperazine, 4-benzylpiperazine, 4-phenylpiperazine, 1,2,3,4-tetrahydroisoquinoline, phthalimide etc.), and an aromatic 5- to 6-membered heterocycle containing 1 to 4 heteroatoms selected from N, O and S (e.g. pyridine, imidazole, indole, tetrazole etc.).

Further, examples of a substituent for a C₆₋₁₀ aryl group and a C₆₋₁₀ aryl-C₁₋₄ alkyl group as a substituent of an optionally substituted amino group constituting the carbamoyl group of the "optionally substituted carbamoyl group" represented by X_{b} include a carboxyl group optionally esterified with a C₁₋₄ alkyl group (e.g. methyl, ethyl, propyl, tert-butyl group etc.), a phosphoric acid group optionally mono- or di-substituted with C₁₋₆ alkyl (e.g. methyl, ethyl, n-propyl, isopropyl, n-butyl, isobutyl, n-pentyl, isopentyl, neopentyl, hexyl) or a C₂₋₇ alkanoyl-C₁₆ alkyl group such as a pivaloyloxymethyl group and an acetyloxymethyl group, a sulfonic acid group, C₁₋₄ alkylsulfonyl, C₆₋₁₀ arylsulfonyl or C₆₋₁₀ aryl-C₁₋₄ alkylsulfonyl, a sulfonamido group optionally substituted with a C₁₋₆ alkyl group or a C₆₋₁₀ aryl-C₁₋₄ alkyl group (e.g. methyl, ethyl, propyl, isopropyl, butyl, tert-butyl, benzyl etc.), a carboxyl group optionally esterified with a C₁₋₄ alkyl group, a phosphoric acid group optionally mono- or di-substituted with a C₁₋₆ alkyl group such as methyl, ethyl, n-propyl, isopropyl, n-butyl, isobutyl, n-pentyl, isopentyl, neopentyl, hexyl and the like or a C₂₋₇ alkanoyloxy-C₁₋₆ alkyl group such as a pivaloyloxymethyl group, a sulfonic acid group, a C₁₋₃ alkyl group optionally substituted with a sulfonamido group optionally substituted with C₁₋₃ alkyl, C₁₆ alkyl, C₆₋₁₀ aryl-C₁₋₃ alkyl, halogen (e.g. fluorine, chlorine) and the like.

The "hydrocarbon group" may have 1 to 5 substituents at replaceable positions.

As the "optionally substituted heterocyclic group" represented by R_{2b} and R_{3b}, a heterocyclic group optionally having 1 to 2 (preferably 1) substituents such as an oxo group, a thioxo group and the like, and having a hydrogen atom which can be deprotonated is preferable. Such heterocyclic group is preferably a 5 - to 6- membered heterocyclic group containing 1 to 4, preferably 2 to 3 heteroatoms selected from S, O and N. Specifically, the examples thereof include tetrazolyl, 4,5-dihydro-5-oxo-1,2,4-oxadiazolyl, 4,5-dihydro-5-thioxo-1,2,4-oxadiazolyl, 2,3-dihydro-3-oxo-1,2,4-oxadiazolyl, 2,3-dihydro-3-thioxo-1,2,4-oxadiazolyl, 3,5-dioxo-1,2,4-oxadiazolidinyl, 4,5-dihydro-5-oxo-isoxazolyl, 4,5-dihydro-5-thioxo-isoxazolyl, 2,3-dihydro-2-oxo-1,3,4-oxadiazolyl, 2,3-dihydro-3-oxo-1,2,4-tetorazolyl and 2,3-dihydro-3-thioxo-1,24-tetrazolyl. Inter alia, a tetrazolyl group is preferable.

Examples of the "acyl group" represented R_{2b} and R_{3b} include a carboxylic acid acyl group derived from carboxylic acid (e.g. a C₂₋₇ carboxylic acid acyl group such as acetyl, propionyl, butyryl, benzoyl etc.), and a C₆₋₁₀ arylsulfonyl group, a C₁₋₄ alkylsulfonyl group and a C₆₋₁₀ aryl-C₁₋₄ alkylsulfonyl group (e.g. methylsulfonyl, ethylsulfonyl, phenylsulfonyl, naphthylsulfonyl, phenylmethylsulfonyl, phenylethylsulfonyl, naphthylmethylsulfonyl, naphthylethysulfonyl, etc.). Examples of a substituent for aryl, and alkyl- and arylalkyl-sulfonyl groups include C₁₋₃ alkyl (e.g. methyl, ethyl, propyl etc.), C₁₋₃ alkoxy (e.g. methoxy, ethoxy, propoxy, etc.), halogen (e.g. chlorine, fluorine, bromine) and the like, and the groups may be substituted 1 to 4 preferably 1 to 2 of them at replaceable positions.

The above carboxylic acid acyl group may have 1 to 2 halogens (e.g. chlorine, fluorine, bromine) as substituents.

Examples of the cyclic amino group optionally substituted with C₁₋₃ alkyl or C₂₋₇ alkanoyl, which is formed by R_{2b} and R_{3b} being taken together with the nitrogen atom of the adjacent carbamoyl, include a group derived from a 5- or 6- membered cyclic amine, which is a cyclic amine such as piperazine, piperidine, pyrrolidine, piperazine-2-one, piperazine-2,6-dione, morpholine, and thiomorpholine, and may further contain 1 to 3 heteroatoms selected from a nitrogen atom, a sulfur atom and an oxygen atom as a ring constituting atom. These cyclic amino groups may have 1 to 4, preferably 1 to 2 substituents. Examples of the substituent include a hydroxy group optionally substituted with C₁₋₃ alkyl or C₂₋₇ alkanoyl, a carboxyl group optionally esterified with a C₁₋₄ alkyl group (e.g. methyl, ethyl, propyl, tert-butyl group etc.) or C₇₋₁₀ arylalkyl, a phosphoric acid group optionally mono- or di-substituted with a C₁₋₆ alkyl or C₂₋₇ alkanoyloxy-C₁₋₆ alkyl group (e.g. an acetyloxymethyl group, a pivaloyloxymethyl group), a sulfonic acid group, and a sulfonamido group optionally substituted with a C₁₋₆ alkyl group or a C₆₋₁₀ aryl-C₁₋₄ alkyl group (e.g. methyl, ethyl, propyl, isopropyl, butyl, tert-butyl, benzyl etc.), C₁₋₆ alkyl and C₂₋₅ alkenyl optionally substituted with "a carboxyl group optionally esterified with C₁₋₆ alkyl or C₆₋₁₀ aryl-C₁₋₄ alkyl, a phosphoric acid group optionally mono- or di-substituted with a C₁₋₆ alkyl or a C₂₋₆ alkanoyloxy-C₁₋₆ alkyl group (e.g. an acetyloxymethyl group, a pivaloyloxymethyl group), a sulfonic acid group, a sulfonamido group optionally substituted with C₁₋₆ alkyl or C₆₋₁₀ aryl-C₁₋₄ alkyl, a hydroxy group optionally substituted with C₁₋₃ alkyl or C₂₋₇ alkanoyl, a sulfhydryl group optionally alkylated with C₁₋₃ alkyl, a carbamoyl group, phenyl optionally substituted with 1 to 5 substituents (e.g. a hydroxy group, halogen, aminosulfonyl, an amino group optionally substituted with C₁₋₃ alkyl), an amino group optionally mono- or di-substituted with C₁₋₃ alkyl, or tetrazolyl", an amino group optionally mono- or di-substituted with a C₁₋₃ alkyl group (e.g. methyl, ethyl, propyl etc.), a cyclic amino group (e.g. a group derived from a 5- or 6- membered cyclic amine optionally substituted with C₁₋₃alkyl, benzyl, or phenyl, and optionally containing a heteroatom selected from a nitrogen atom, a sulfur atom, and an oxygen atom, such as piperidine, pyrrolidine, morpholine, thiomorpholine, 4-methylpiperazine, 4-benzylpiperazine, 4-phenylpiperazine, etc.), a cyano group, a carbamoyl group, an oxo group, C₁₋₃ alkoxy (e.g. methoxy, ethoxy, ethylenedioxy etc.), a heterocyclic group optionally substituted with an oxo group or a thioxo group having the aforementioned hydrogen atom which can be deprotonated (e.g. tetrazolyl, 2,5-dihydro-5-oxo-1,2,4-oxadiazolyl etc.), C₆₋₁₀ arylsulfonyl, C₆₋₁₀ aryl-C₁₋₄ alkylsulfonyl and C₁₋₄ alkylsulfonyl exemplified as the substituents of the optionally substituted amino group forming carbamoyl of the "optionally substituted carbamoyl group" represented by X (e.g. methylsulfonyl, ethylsulfonyl, propylsulfonyl, butylsulfonyl, isopropylsulfonyl, tert-butylsulfonyl, phenyl, sulfonyl, benzylsulfonyl, etc.), a sulfhydryl group optionally alkylated with C₁₋₃ alkyl, or a carbamoyl group substituted with phenyl optionally substituted with 1 to 5 substituents (e.g. a hydroxy group, halogen, aminosulfonyl, and an amino group optionally substituted with C₁₋₃ alkyl).

Examples of the optionally substituted carbamoyl group represented by X_{b} include: 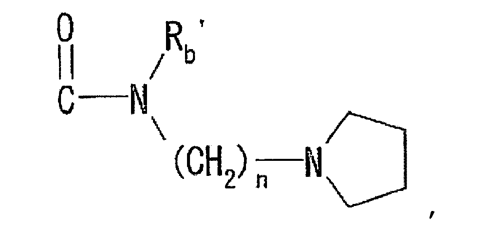 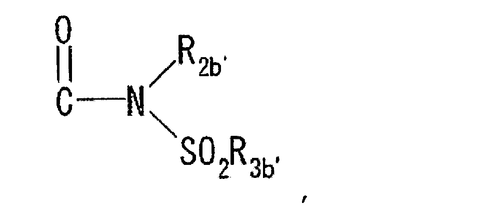 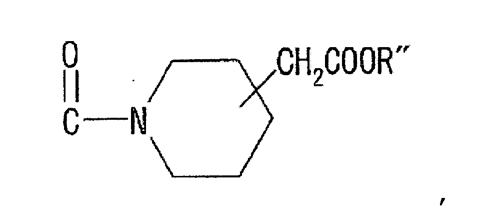 and the like.

Examples of R_{2b}, and R_{b}' include a hydrogen atom and C₁₋₇ alkyl. Inter alia, the hydrogen atom is preferable.

Examples of the C₁₋₇ alkyl represented by R_{2b}, R_{2b'} and R_{b}' include the same C₁₋₇ alkyl as that of the "hydrocarbon group".

Examples of R" include a hydrogen atom and C₁₋₄ alkyl. Inter alia, a hydrogen atom is preferable.

Examples of the C₁₋₄ alkyl represented by R_{3b}, and R" include methyl, ethyl, propyl, isopropyl, n-butyl, tert-butyl and the like.

As the optionally substituted heterocyclic group having a hydrogen atom which can be deprotonated, presented by X_{b}, a nitrogen containing (preferably, 1 to 4 nitrogen atoms-containing) 5- to 6- membered heterocycle having a Brönsted acid active proton is preferable, and it is preferable that the heterocycle contains 1 to 4, preferably 2 to 3 of a nitrogen atom, a sulfur atom, and an oxygen atom. Examples of the substituent thereof include an oxo group, a thioxo group and the like, and the heterocycle may have 1 to 2, particularly 1 of these substituents. Examples of the "optionally substituted heterocyclic group having a hydrogen atom which can be deprotonated" represented by X include the groups exemplified as the "optionally substituted heterocyclic group" as a substituent of the "optionally substituted carbamoyl group" represented by X, such as tetrazolyl, 2,5-dihydro-5-oxo-1,2,4-oxadiazolyl and the like.

Examples of the "lower alkyl group" represented by R_{1b} include a C₁₋₆ alkyl group such as methyl, ethyl, n-propyl, isopropyl, n-butyl, tert-butyl, pentyl, hexyl and the like. Inter alia, a C₁₋₃ alkyl group is preferable. As R_{1b}, particularly, a methyl group is preferable from a viewpoint of the pharmacological activity.

Examples of the "halogen atom" represented by W include a chlorine atom, a fluorine atom, a bromine atom, and an iodine atom. Inter alia, a chlorine atom is preferable.

Examples of a salt of the compound represented by the formula (Ib) include pharmaceutically acceptable salts such as inorganic acid salts such as hydrochloride, hydrobromide, sulfate, nitrate, phosphate and the like, organic acid salts such as acetate, tartarate, citrate, fumarate, maleate, toluenesulfonate, methanesulfonate and the like, metal salts such as a sodium salt, a potassium salt, a calcium salt, an aluminum salt and the like, base salts such as a triethylamine salt, a guanidine salt, an ammonium salt, a hydrazine salt, a quinine salt, a cinchonine salt and the like, and the like.

In addition, a hydrate and a non-hydrate of the compound represented by the formula (Ib) are also included in the present invention.

In the compound represented by the formula (Ib) or a salt thereof, there are an asymmetric carbon at a 3-position and a 5-position, an trans isomer, i.e., an isomer whose substituents at 3-position and 5-position are oriented in a reverse direction relative to a 7-membered ring plane is preferable and, particularly, an isomer whose absolute configuration of the substituent at 3-position is R configuration, and absolute configuration of the substituent at 5-position is S configuration is preferable.

As the compound represented by the formula (Ib) or a salt thereof, specifically, the following are preferable. N-methanesulfonyl-[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetamide, N-methanesulfonyl-[(3R,5S)-7-chloro-5-(2,3-dimethoxyphenyl)-1-(3-hydroxy-2-hydroxymethyl-2-methylpropyl)-2-oxo-1,2,3,5-tetrahydro-4,1-benzoxazepin-3-yl]acetamide,
N-[2-(pyrrolidin-1-yl)ethyl]-[(3R,5S)-7-chloro-5-(2,3-dimethoxyphenyl)-1-(3-hydroxy-2-hydroxymethyl-2-methylpropyl)-2-oxo-1,2,3,5-tetrahydro-4,1-benzoxazepin-3-yl]acetamide,
N-[2-(pyrrolidin-1-yl)ethyl]-[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetamide, N-methanesulfonyl-[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetamide, N-methanesulfonyl-[(3R,5S)-1-(3-acetoxy-2-acetoxymethyl-2-methylpropyl)-7-chloro-5-(2,3-dimethoxyphenyl)-2-oxo-1,2,3,5-tetrahydro-4,1-benzoxazepin-3-yl]acetamide,
N-[[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl]piperidine-4-acetic acid,
N-[[(3R,5S)-1-(3-hydroxy-2,2-dimethylpropyl)-7-chloro-5-(2,3-dimethoxyphenyl)-2-oxo-1,2,3,5-tetrahydro-4,1-benzoxazepin-3-yl]acetyl]piperidine-4-acetic acid,
N-[[(3R,5S)-1-(2,2-dimethylpropyl)-7-chloro-5-(2,3-dimethoxyphenyl)-2-oxo-1,2,3,5-tetrahydro-4,1-benzoxazepin-3-yl]acetyl]piperidine-4-acetic acid,
N-[[(3R,5S)-1-(3-acetoxy-2-acetoxymethyl-2-methylpropyl)-7-chloro-5-(2,3-dimethoxyphenyl)-2-oxo-1,2,3,5-tetrahydro-4,1-benzoxazepin-3-yl]acetyl]piperidine-4-acetic acid,
N-[[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl]piperidine-4-acetic acid ethyl ester,
N-[[(3R,5S)-1-(3-acetoxy-2-acetoxymethyl-2-methylpropyl)-7-chloro-5-(2,3-dimethoxyphenyl)-2-oxo-1,2,3,5-tetrahydro-4,1-benzoxazepin-3-yl]acetyl]piperidine-4-acetic acid ethyl ester,
(3R,5S)-7-chloro-5-(2,3-dimethoxyphenyl)-1-(3-hydroxy-2,2-dimethylpropyl)-1,2,3,5-tetrahydro-3-[1H (or 3H)-tetrazol-5-yl]methyl-4,1-benzoxazepine-2-one,
(3R,5S)-7-chloro-5-(2,3-dimethoxyphenyl)-1-(3-hydroxy-2-hydroxymethyl-2-methylpropyl)-1,2,3,5-tetrahydro-3-[1H (or 3H)-tetrazol-5-yl]methyl-4,1-benzoxazepine-2-one,
(3R,5S)-1-(3-acetoxy-2,2-dimethylpropyl-7-chloro-5-(2,3-dimethoxyphenyl)-1,2,3,5-tetrahydro-3-[1H (or 3H)-tetrazol-5-yl]methyl-4,1-benzoxazepine-2-one,
(3R,5S)-1-(3-acetoxy-2-acetoxymethyl-2-methylpropyl)-7-chloro-5-(2,3-dimethoxyphenyl)-1,2,3,5-tetrahydro-3-[1H (or 3H)-tetrazol-5-yl]methyl-4,1-benzoxazepine-2-one,
N-[2-pyrrolidin-1-yl]ethyl]-[(3R,5S)-7-chloro-5-(2,3-dimethoxyphenyl)-1-neopentyl-2-oxo-1,2,3,5-tetrhydro-4,1-benoxazepin-3-yl]acetamide; etc.

The compound represented by the formula (Ib) or a salt thereof can be produced by the method described in gazettes such as EPA567026, WO95/21834 (PCT application based on Japanese Patent Application No.6-15531), EPA645377 (application based on Japanese Patent Application No.6-229159), EPA645378 (application based on Japanese Patent Application No.6-229160), and WO9710224, or a similar method.

As the compound represented by the formula (I), a compound represented by the formula (Ic): 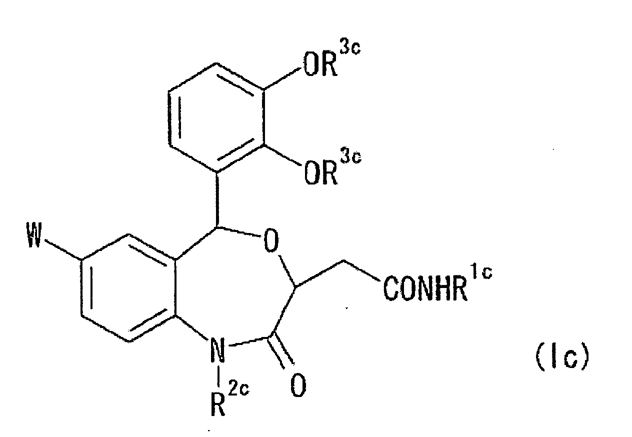 is preferable.

As the compound represented by the formula (Ic), a compound in which R^{1c} is a 3-carboxypropyl group, or a 1-carboxyethyl group, or a C₃₋₆ straight alkyl-sulfonyl group, a (carboxy-C₅₋₇ cycloalkyl)-C₁₋₃ alkyl group, a (carboxyfuryl)-alkyl group, a carboxy-C₆₋₁₀ aryl group, a (carboxy-C₂₋₃ alkyl) -C₆₋₁₀ aryl group or a (carboxy-C₁₋₃ alkyl)-C₇₋₁₄ aralkyl group, each optionally having a substituent;
a compound in which R^{1c} is a (carboxy-C₁₋₄ alkyl)-C₆₋₁₀ aryl group optionally having a substituent;
a compound in which R^{1c} is a (carboxy-C₂₋₃ alkyl) -C₆₋₁₀ aryl group optionally having a substituent;
a compound in which R^{1c} is a (carboxy-C₂₋₃ alkyl)-phenyl group optionally having a substituent;
a compound in which R^{1c} is a (carboxyfuryl)-alkyl group optionally having a substituent;
a compound in which R^{2c} is a C₃₋₆alkyl group having an alkanoyloxy group and/or a hydroxy group;
a compound in which R^{2c} is a C₃₋₆alkyl group optionally having 1 to 3 substituents selected from acetoxy, propionyloxy, tert-butoxycarbonyl and palmitoyl;
a compound in which R^{2c} is 2,2-dimethylpropyl, 3-hydroxy-2,2-dimethylpropyl or 3-acetoxy-2,2-dimethypropyl;
a compound in which R^{3c} is a methyl group;
a compound in which W is a chlorine atom;
a compound in which a 3-position is R-configuration, and a 5-position is S-configuration; are preferable.

In the above formulas, R^{1c} represents a 1-carboxyethyl group optionally having a substituent, a carboxy-C₃₋₆ straight alkyl group optionally having a substituent, a C₃₋₆ straight alkyl-sulfonyl group optionally having a substituent, a (carboxy-C₅₋₇ cycloalkyl) -C₁₋₃ alkyl group optionally having a substituent, or a group represented by the formula -X^{1c}-X^{2c}-Ar-X^{3c}-X^{4c}-COOH (wherein X^{1c} and X^{4c} each represent a bond or a C₁₋₄alkylene group optionally having a substituent, X^{2c} and X^{3c} each represent a bond, -O- or -S-, Ar represents a divalent aromatic cyclic group optionally having a substituent, provided that when X_{1c} is a bond, X^{2c} represents a bond and, when X4c is a bond, X^{3c} represents a bond).

Examples of the C₃₋₆ straight alkyl group in the carboxy-C₃₋₆ straight alkyl group optionally having a substituent represented by R^{1c} include n-propyl, n-butyl, n-pentyl, and n-hexyl. Among them, n-propyl, and n-butyl are preferable, and n-propyl is more preferable.

Examples of the C₃₋₆ straight alkyl group in the C₃₋₆ straight alkyl-sulfonyl group optionally having a substituent represented by R^{1c} include n-propyl, n-butyl, n-pentyl, and n-hexyl. Among them, n-propyl, and n-butyl are preferable, and n-propyl is more preferable.

Examples of the C₅₋₇ cycloalkyl group in the (caboxy-C₅₇ cycloalkyl)-C₁₋₃ alkyl group optionally having a substituent represented by R^{1c} include cyclopentyl, cyclohexyl, and cyclobutyl. Among them, cyclopentyl, and cyclohexyl are preferable, and cyclohexyl is more preferable.

Examples of the C₁₋₃ alkyl group in the (carboxy-C₅₋₇ cycloalkyl)-C₁₋₃ alkyl group optionally having a substituent represented by R^{1c} include methyl, ethyl, n-propyl, and isopropyl. Among them, methyl, and ethyl are preferable, and methyl is more preferable.

Examples of the "C₁₋₄ alkylene group" in the "C₁₋₄ alkylene group optionally having a substituent" represented by X^{1c} and X^{4c}, in a group represented by the formula-X^{1c}-X^{2c}-Ar-X^{3c}-X^{4c}-COOH as R^{1c}, include methylene, dimethylene, trimethylene, and tetramethylene, a C₁₋₃ alkylene group is preferable and, inter alia, a straight group is preferably used.

Examples of the "divalent aromatic cyclic group" in the "divalent aromatic cyclic group optionally having a substituent" represented by Ar include a divalent aromatic hydrocarbon group, and a divalent aromatic heterocyclic group.

Herein, examples of the divalent aromatic hydrocarbon group include a group formed by removing one hydrogen atom from a C₆₋₁₀ aryl group (e.g. phenyl, naphthyl) and, as the divalent aromatic hydrocarbon group, phenylene is preferably used.

Examples of the divalent aromatic heterocyclic group include a group formed by removing one hydrogen atom from an aromatic heterocyclic group containing 1 to 3 kinds (at least 1 to 2 kinds) of at least one (preferably 1 to 4, further preferably 1 to 2) heteroatoms selected from an oxygen atom, a sulfur atom and a nitrogen atom as an atom constituting a ring system (ring atom).

Herein, examples of the aromatic heterocyclic group include a 5- to 6-membered aromatic monocyclic heterocyclic group such as furyl, thienyl, pyrrolyl, oxazolyl, isoxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyridazinyl, pyrimidinyl, pyrazinyl, triazinyl and the like (preferably, furyl, thienyl, pyrrolyl, imidazolyl, thiazolyl, pyridyl), and a 8- to 12-membered aromatic fused heterocyclic group such as benzofuranyl, isobenzofuranyl, benzo[b]thienyl, indolyl, isoindolyl, 1H-indazolyl, benzimidazolyl, benzoxazolyl, 1,2-benzisoxazolyl, benzothiazolyl, benzopyranyl, 1,2-benzisothiazolyl, 1H-benzotriazolyl, quinolyl, isoquinolyl, cinnolinyl, quinazolinyl, quinoxalinyl, phthalazinyl, naphthyridinyl, purinyl, pteridinyl, carbazolyl, α-carbolinyl, β-carbolinyl, γ-carbolinyl, acridinyl, phenoxazinyl, phenothiazinyl, phenazinyl, phenoxathiyl, thianthrenyl, phenathrydinyl, phenanthrolinyl, indolizinyl, pyrrolo[1,2-b]pyridazinyl, pyrazolo[1,5-a]pyridyl, imidazo[1,2-a]pyridyl, imidazo[1,5-a]pyridyl, imidazo[1,2-b]pyridazinyl, imidazo[1,2-a]pyrimidinyl, 1,2,4-triazolo[4,3-a]pyridyl, 1,2,4-triazolo[4,3-b]pyridazinyl and the like (preferably, a heterocyclc ring in which the 5- to 6-membered aromatic monocyclic heterocyclic group is fused with a benzene ring, or a heterocyclc ring in which the same or different two 5-to 6-membered aromatic monocyclic heterocyclic groups are fused, more preferably a heterocyclc ring in which the 5-to 6-membered aromatic monocyclic heterocyclic group is fused with a benzene ring) and the like.

Examples of a substituent optionally possessed by the "C₁₋₄alkylene group" in the "C₁₋₄alkylene group optionally having a substituent" represented by X^{1c} and X^{4c} ; as well as the "divalent aromatic cyclic group" in the "divalent aromatic ring group optionally having a substituent" represented by Ar, respectively, include (i) a carboxyl group optionally esterified with a C₁₋₆alkyl group or a C₆₁₀aryl-C₁₋₄alkyl group (e.g. methyl, ethyl, propyl, isopropyl, butyl, tert-butyl, phenyl, benzyl, etc.), (ii) a phosphoric acid group optionally mono- or di-substituted with C₁₋₆alkyl (e.g. methyl, ethyl, n-propyl, isopropyl, n-butyl, isobutyl, n-pentyl, isopentyl, neopentyl, hexyl, etc.), or C₂₋₇alkanoyloxy-C₁₋₆alkyl such as an acetoxymethyl group and a pivaloyloxymethyl group, (iii) a sulfonic acid group, (iv) a sulfonamide group optionally substituted with a C₁₋₆alkyl group or a C₆₋₁₀aryl-C₁₋₄alkyl group (e.g. methyl, ethyl, propyl, isopropyl, butyl, tert-butyl, benzyl, etc.), (v) a hydroxyl group and a sulfhydryl group optionally alkylated with a C₁₋₃alkyl group (e.g. methyl, ethyl, propyl, etc.), (vi) a carbamoyl group, (vii) a phenyl group which may be substituted with 1 to 5 substituents [e.g. a hydroxyl group, chlorine, fluorine, an aminosulfonyl group, an amino group optionally substituted with a C₁₋₃alkyl group (e.g. methyl, ethyl, propyl, etc.)], and which may be bound via O or S, (viii) an amino group optionally mono- or di-substituted with a C₁₋₃alkyl group (e.g. methyl, ethyl, propyl, etc.), (ix) a cyclic amino group optionally substituted with 1 to 3 of C₁₋₃alkyl (e.g. methyl, ethyl, etc.), benzyl, phenyl and the like (e.g. a 5- to 6-membered cyclic amino group optionally containing an oxygen atom or a sulfur atom as a ring constituting atom in addition to a nitrogen atom, such as a cyclic amino group derived (by removing one hydrogen atom) from cyclic amine, such as piperidine, pyrrolidine, morpholine, thiomorpholine, piperazine, 4-methylpiperazine, 4-benzylpiperazine, 4-phenylpiperazine, 1,2,3,4-tetrahydroisoquinoline, phthalimide, etc.), (x) a 5- to 6-membered aromatic heterocyclic group which contains 1 to 4 heteroatoms selected from N, O and S, and which may be bound via O or S (e.g. pyridyl, imidazolyl, indolyl, tetrazolyl, etc.), (xi) a halogen atom (e.g. chlorine, fluorine, bromine, iodine), (xii) a C₁₋₄alkyl group (e.g. methyl, ethyl, propyl, isopropyl, butyl, tert-butyl, etc.), a C₁₋₄alkoxy group (e.g. methoxy, ethoxy, propoxy, isopropoxy, butoxy, t-butoxy, etc.) or a C₁₋₄alkylthio group (e.g. methylthio, ethylthio, propylthio, isopropylthio, butylthio, tert-butylthio, etc.), each being optionally substituted with a substituent selected from a C₁₋₄alkoxy group, a C₁₋₄alkylthio group, carboxyl and phenyl, (xiii) a C₅₋₇cycloalkyl group (e.g. cyclopentyl, cyclohexyl, cycloheptyl, etc.), (xiv) a C₁₇alkanoyloxy (e.g. formyloxy, acetoxy, propionyloxy, butyryloxy, t-butoxycarbonyloxy, isobutyryloxy, valeryloxy, pivaloyloxy, etc.). One to six, preferably one to three of such substituents can be present at replaceable positions. Alternatively, two substituents may be taken together to form C₃₋₆alkylene, C₃₋₆alkyleneoxy, C₃₋₆alkylenedioxy, etc. For example, when two adjacent substituents on a phenyl group are taken together to form C₄alkylene, leading to formation of a tetrahydronaphthalene group.

Examples of the group represented by the formula -X^{1c}-X^{2c}-Ar-X^{3c}-X^{4d}-COOH as R^{1c} include a (carboxy-heteroaryl)-C₁₄alkyl group optionally having a substituent [preferably, a (carboxy-furyl)-C₁₋₄alkyl group optionally having a substituent], a (carboxy-C₆₋₁₀aryl)-C₁₋₄alkyl group optionally having a substituent, a carboxy-heteroaryl group optionally having a substituent, a carboxy-C₆₋₁₀aryl group optionally having a substituent, a (carboxy-C₁₋₄alkyl)-heteroaryl group optionally having a substituent, a (carboxy-C₁₋₄alkyl)-C₆₋₁₀aryl group optionally having a substituent [preferably, a (carboxy-C₂₋₃alkyl)-C₆₋₁₀aryl group], a (carboxy-C₁₋₄alkyl)-heteroaryl-C₁₋₄alkyl group optionally having a substituent, a (carboxy-C₁₋₄alkyl)-C₇₁₄aralkyl group optionally having a substituent [preferably, a (carboxy-C₁₋₃alkyl)-C₇₋₁₄aralkyl group optionally having a substituent], a (carboxy-C₁₋₄alkoxy) -C₆₋₁₀aryl group optionally having a substituent, a (carboxy-C₁₋₄alkoxy)-C₆₁₀aryl-C₁₋₄alkyl group optionally having a substituent, a (carboxy-C₁₋₄alkyl)-C₆₋₁₀aryloxy-C₁₋₄alkyl group optionally having a substituent, a (carboxy-C₆₋₁₀aryloxy)-C₁₋₄alkyl group optionally having a substituent, and a (carboxy-C₁₄alkylthio)-heteroaryl group.

Herein, examples of the heteroaryl include the same groups as those described above for the "aromatic heterocyclic group", and the heteroaryl may have the same substituents as the substituents which may be possessed by the "aromatic heterocyclic group" as described above. In addition, examples of the C₆₋₁₀aryl include phenyl, naphthyl, and azulenyl, and phenyl is preferable. The C₆₋₁₀aryl may have the same substituents as the substituents which may be possessed by the "aromatic heterocyclic group" as described above.

Examples of the alkyl group in the (carboxyfuryl)-C₁₄alkyl group optionally having a substituent represented by R¹ include a C₁₋₄ straight or branched alkyl group such as methyl, ethyl, n-propyl, isopropyl, n-butyl, isobutyl, 1,1-dimethylethyl and the like. Among them, a C₁₋₄alkyl group such as methyl, ethyl, n-propyl, isopropyl, n-butyl and the like is preferable, and methyl, ethyl, and n-propyl are more preferable. Examples of the carboxyfuryl group include 3-carboxy-2-furyl, 4-carboxy-2-furyl, 2-carboxy-3-furyl, 2-carboxy-5-furyl and the like. Among them, 3-carboxy-2-furyl, and 4-carboxy-2-furyl are preferable, and 3-carboxy-2-furyl is more preferable.

Examples of the C₂₋₃alkyl group in the (carboxy-C₂₃alkyl)-C₆₋₁₀aryl group optionally having a substituent represented by R^{1c} include ethyl, n-propyl, and isopropyl, and ethyl and n-propyl are preferable. Examples of the C₆₁₀aryl group include phenyl, naphthyl, and azulenyl, and phenyl is preferable.

Examples of the C₁₋₃alkyl group in the (carboxy-C₁-3alkyl)-C₇₋₁₄aralkyl group optionally having a substituent represented by R^{1c} include methyl, ethyl, n-propyl, and isopropyl. Methyl and ethyl are preferable, and ethyl is particularly preferable. Examples of the C₇₋₁₄aralkyl group (a C₆₋₁₀aryl-C₁₋₄alkyl group) include phenylmethyl, 1-phenylethyl, 2-phenylethyl, 3-phenylpropyl, 2-phenylpropyl, 4-phenylbutyl, (1-naphthyl)methyl, (2-naphthyl)methyl, 1-(1-naphthyl)ethyl, 1-(2-naphthyl)ethyl, 3-(1-naphthyl)propyl, 3-(1-naphthyl)propyl, 4-(1-naphthyl)butyl, and 4-(2-naphthyl)butyl. Phenylmethyl, 1-phenylethyl, 3-phenylpropyl, (1-naphthyl)methyl, (2-naphthyl)methyl, (1-naphthyl)ethyl, and (2-naphthyl)ethyl are preferable, and phenylmethyl, and 2-phenylethyl are particularly preferable.

When each group represented by R^{1c} has a substituent, examples of the substituent include the same substituents as the substituents which may be possessed by the "divalent aromatic cyclic group" in the "divalent aromatic cyclic group optionally having a substituent" represented by Ar, and 1 to 6, preferable 1 to 3 of such substituents can be present at replaceable positions. In addition, in each group represented by R^{1c}, it is preferable that a carboxyl moiety is unsubstituted, but any moieties other than the carboxyl moiety may have a replaceable substituent at a replaceable position.

For R^{1c}, a 3-carboxypropyl group, and a 1-carboxyethyl group, as well as a C₃₋₆ straight alkyl-sulfonyl group, a (carboxy-C₅₋₇cycloalkyl)-C₁₋₃alkyl group, a (carboxyfuryl)-alkyl group, a carboxy-C₆₋₁₀aryl group, a (carboxy-C₁₄alkyl)-C₆₋₁₀aryl group [preferably, a (carboxy-C₂₋₃alkyl)-C₆₋₁₀aryl group], a (carboxy-C₁₋₃alkyl)-C-₇₋₁₄aralkyl, each optionally having a substituent, is preferable, a (carboxy-C₁₋₄alkyl)-C₆₋₁₀aryl group optionally having a substituent is more preferable, a (carboxy-C₂₋₃alykl)-C₆₋₁₀aryl group optionally having a substituent is further preferable and, inter alia, a (carboxy-C₂₋₃alkyl)-phenyl group optionally having a substituent is particularly preferable.

Examples of the C₃₋₆alkyl group in the C₃₋₆alkyl group optionally substituted with an alkanoyloxy group or a hydroxyl group represented by R^{2c} include, for example, n-propyl, isopropyl, 1,1-dimethylethyl, n-butyl, isobutyl, n-pentyl, 2,2-dimethylpropyl, isopentyl, n-hexyl, isohexyl and the like. Among them, isopropyl, 1,1-dimethylethyl, n-butyl, isobutyl, 2,2-dimethylpropyl, and isohexyl are preferable, and 2,2-dimethylpropyl is particularly preferable.

Examples of the alkanoyloxy group in the C₃₋₆alkyl group optionally substituted with an alkanoyloxy group or a hydroxyl group represented by R^{2c} include, for example, a C₁₋₂₀alkanoyloxy group (preferably, a C₁₋₇alkanoyloxy group) such as formyloxy, acetoxy, propionyloxy, butyryloxy, tert-butoxycarbonyloxy, isobutyryloxy, valeryloxy, pivaloyloxy, lauryloxy, palmitoyloxy, strearoyloxy and the like. Among them, acetoxy, propionyloxy, tert-butoxycarbonyloxy, and palmitoyloxy are preferable, and acetoxy is particularly preferable. It may be substituted with one to three alkanoyloxy or hydroxyl groups at replaceable positions.

Preferable examples of the C₃₋₆alkyl group optionally substituted with an alkanoyloxy group or a hydroxyl group represented by R^{2c} include 2,2-dimethylpropyl, 3-hydroxy-2,2-dimethylpropyl, 3-hydroxy-2-hydroxymethyl-2-methylpropyl, 2-acetoxy-2,2-dimethylpropyl, 3-acetoxy-2-hydroxymethyl-2-methylpropyl and 3-acetoxy-2-acetoxymethyl-2-methylpropyl. Among them, 2,2-dimethylpropyl, 3-hydroxy-2,2-dimethylpropyl, and 3-acetoxy-2,2-dimethylpropyl are particularly preferable.

In addition, For R^{2c}, a C₃₋₆alkyl group having an alkanoyloxy group and/or a hydroxyl group is preferable.

Examples of the lower alkyl group represented by R^{3c} include a C₁₋₆alkyl group such as methyl, ethyl, n-propyl, isopropyl, n-butyl, tert-butyl, pentyl, hexyl and the like. Inter alia, a C₁₋₃alkyl group is preferable. For R^{3c}, a methyl group is particularly preferable from a viewpoint of the pharmacological activity.

Examples of the halogen atom represented by W include chlorine, fluorine, bromine and iodine atoms. Inter alia, a chlorine atom is preferable.

The compound represented by the formula (Ic), whether it is a free compound or a pharmacologically acceptable salt, is included in the present invention. For such salts, when the compound represented by the formula (Ic) has an acidic group such as a carboxyl group and the like, the compound may form salts with inorganic bases (e.g. alkali metals such as sodium, potassium, etc., alkaline earth metals such as calcium, magnesium, etc., transition metals such as zinc, iron, copper, etc.), or organic bases (e.g. organic amines such as trimethylamine, triethylamine, pyridine, picoline, ethanolamine, diethanolamine, triethanolamine, dicyclohexylamine, N,N'-dibenzylethylenediamine, etc., basic amino acids such as arginine, lysine, ornithine).

When the compound represented by the formula (Ic) of the present invention has a basic group such as an amino group and the like, the compound may form salts with inorganic or organic acids (e.g. hydrochloric acid, nitric acid, sulfuric acid, phosphoric acid, carbonic acid, bicarbonic acid, formic acid, acetic acid, propionic acid, trifluoroacetic acid, fumaric acid, oxalic acid, tartaric acid, maleic acid, citric acid, succinic acid, malic acid, methanesulfonic acid, benzenesulfinic acid, p-toluenesulfonic acid, etc.), or acidic amino acids such as aspartic acid, and glutamic acid.

The compound represented by the formula (Ic) or a salt thereof has an asymmetric carbon atom at a 3-position and a 5-position, and may be a mixture of stereoisomers, and isomers may be separated by the known means. A trans-entity being an isomer in which substituents at a 3-position and a 5-position are oriented in a reverse direction of each other to the plane of a 7-membered ring is preferable and, particularly, an isomer in which the absolute configuration at a 3-position is the R configuration, and the absolute configuration at a 5-position is the S configuration is preferable. In addition, the compound may be a recemate or an optically active isomer. The optically active isomer can be separated from a racemate by the known optical resolution means.

As the compound represented by the formula (Ic) of the present invention or a salt thereof, specifically, the following are preferable:
N-propanesulfonyl-[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetamide, or a salt thereof,
(2R)-2-[[(3R,5S)-7-chloro-5-(2,3-dimethoxyphenyl)-1-(2,2-dimethylpropyl)-2-oxo-1,2,3,5-tetrahydro-4,1-benzoxazepin-3-yl]acetyl]aminopropionic acid, or a salt thereof,
3-[3-[[(3R,5S)-7-chloro-5-(2,3-dimethoxyphenyl)-1-(2,2-dimethylpropyl)-2-oxo-1,2,3,5-tetrahydro-4,1-benzoxazepin-3-yl]acetyl]aminophenyl]propionic acid, or a salt thereof,
4-[[(3R,5S)-7-chloro-5-(2,3-dimethoxyphenyl)-1-(2,2-dimethylpropyl)-2-oxo-1,2,3,5-tetrahydro-4,1-benzoxazepin-3-yl]acetyl]aminobutanoic acid, or a salt thereof,
trans-4-[[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl]-aminomethyl-1-cyclohexanecarboxylic acid, or a salt thereof,
trans-4-[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl]-aminomethyl-1-cyclohexanecarboxylic acid, or a salt thereof,
3-[3-[[[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl]amino]-4-fluorophenyl]propionic acid, or a salt thereof,
3-[3-[[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl]amino]-4-methylphenyl]propionic acid, or a salt thereof,
3-[3-[[[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl]amino]-4-methylphenyl]propionic acid, or a salt thereof,
3-[3-[[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl]aminomethyl]phenyl]propionic acid, or a salt thereof,
3-[3-[[[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl]aminomethyl]phenyl]propionic acid, or a salt thereof,
3-[3-[[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl]amino]-4-methoxyphenyl]propionic acid, or a salt thereof,
2-[2-[[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl]amino]ethyl]furan-3-carboxylic acid, or a salt thereof,
3-[3-[[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl]amino]-4-fluorophenyl]propionic acid, or a salt thereof,
3-[3-[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl]aminophenyl]propionic acid, or a salt thereof,
4-[3-[[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl]amino]-4-methoxyphenyl]butanoic acid, or a salt thereof,
5-[3-[[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl]amino]-4-methoxyphenyl]pentanoic acid, or a salt thereof,
5-[3-[[[(3R, 5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl]amino]-4-fluorophenyl]pentanoic acid, or a salt thereof, and the like.

The compound represented by the formula (Ic) or a salt thereof can be produced according to the method disclosed, for example, in gazettes such as EPA 567026, WO95/21834 (International Application based on Japanese Patent Application No.6-15531), EPA645377 (Application based on Japanese Patent Application No.6-229159), and EPA 645378 (Application based on Japanese Patent Application No.6-229160), and WO 01/98282 (International Application based on Japanese Patent Application No.2000-190253), or a similar method.

Also as a raw material compound for the compound represented by the formula (I) of the present invention, the same salts as those described above are used, and they are not particularly limited as far as a reaction is not affected.

Preferable examples of each definition in the compound represented by the formula (II) are as follows. Examples of the substituent for the "optionally substituted benzene ring" represented by a ring A include halogen (e.g. fluorine, chlorine, bromine, iodine), an optionally substituted lower alkyl group of a carbon number of 1 to 4 (e.g. methyl, ethyl, propyl, butyl, tert-butyl, etc.), an optionally substituted lower alkoxy group of a carbon number of 1 to 4 (e.g. methoxy, ethoxy, propoxy, isopropoxy, butoxy, tert-butoxy, etc.), a hydroxyl group, a nitro group, cyano and the like. The ring A may have 1 to 3, preferably 1 to 2 of these substituents. Alternatively, these substituents adjacent to each other may form a ring. Examples of the substituent in the optionally substituted lower alkyl group of a carbon number of 1 to 4 and in the optionally substituted lower alkoxy group of a carbon number of 1 to 4 include a halogen atom (e.g. fluorine, chlorine, bromine, iodine) and the like, and such alky or alkyl group may be substituted with 1 to 3 of the substituents at any replaceable positions. As the ring A, a benzene ring substituted with a halogen atom is preferable, and a benzene ring substituted with a chlorine atom is particularly preferable. As the ring A, a benzene ring represented by the formula: 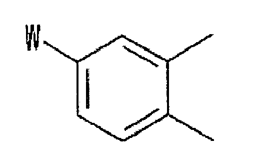 wherein W represents a halogen atom (e.g. fluorine, chlorine, bromine, iodine), is preferable and, inter alia, it is preferable that W is a chlorine atom.

As the substituent in the "optionally substituted benzene ring" represented by the ring B, the same number of the same groups as the substituents which may be possessed by the benzene ring in the "optionally substituted benzene ring" represented by the ring A as described above are used. As the ring B, a benzene ring substituted with a lower alkoxy group of a carbon number of 1 to 4 is preferable and, inter alia, a benzene ring represented by the formula: 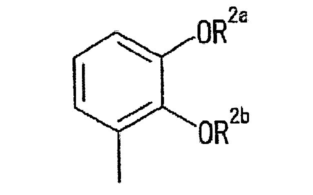 wherein R^{2a} and R^{2b} each independently represent a hydrogen atom or a lower alkyl group of a carbon number of 1 to 4 (e.g. methyl, ethyl, propyl, butyl), is preferable, and it is particularly preferable that both of R^{2a} and R^{2b} are a methyl group.

Examples of the aromatic ring in the "optionally further substituted aromatic ring" represented by the ring C include an aromatic hydrocarbon ring and an aromatic heterocycle. Examples of the aromatic hydrocarbon ring include a benzene ring, a naphthalene ring and the like, preferably a benzene ring. Examples of the aromatic heterocycle (aromatic heterocycle in the "optionally further substituted aromatic heterocycle" represented by the ring C") include an aromatic heterocycle contained at least one (preferably 1 to 4, more preferably 1 to 2) of 1 to 3 (preferably, 1 to 2) different heteroatoms selected from an oxygen atom, a sulfur atom and a nitrogen atom as atoms constituting a ring system (ring atoms).

Examples of the aromatic heterocycle include a 5- to 6-membered monocyclic aromatic heterocycle such as furan, thiophene, pyrrole, oxazole, isoxazole, thiazole, isothiazole, imidazole, pyrazole, 1,2,3-oxadiazole, 1,2,4-oxadiazole, 1,3,4-oxadiazole, furazan, 1,2,3-thiadiazole, 1,2,4-thiadiazole, 1,3,4-thiadiazole, 1,2,3-triazole, 1,2,4-triazole, tetrazole, pyridine, pyridazine, pyrimidine, pyrazine, triazine and the like, and a 8- to 12-membered fused aromatic heterocycle such as benzofuran, isobenzofuran, benzo[b]thiophene, indole, isoindole, 1H-indazole, benzimidazole, benzoxazole, 1,2-benzisoxazole, benzothiazole, benzopyran, 1,2-benzisothiazole, 1H-benzotriazole, quinoline, isoquinoline, cinnoline, quinazoline, quinoxaline, phthalazine, naphthyridine, purine, pteridine, carbazole, α-carboline, β-carboline, γ-carboline, acridine, phenoxazine, phenothiazine, phenazine, phenoxathine, thianthrene, phenanthridine, phenathroline, indolizine, pyrrolo[1,2-b]pyridazine, pyrazolo[1,5-a]pyridine, imidazo[1,2-a]pyridine, imidazo[1,5-a]pyridine, imidazo[1,2-b]pyridazine, imidazo[1,2-a]pyrimidine, 1,2,4-triazolo[4,3-a]pyridine, 1,2,4-triazolo[4,3-b]pyridazine and the like (preferably, a heterocycle in which the 5- to 6-membered monocyclic aromatic heterocycle is fused with a benzene ring, or a heterocycle in which the same or different two 5- to 6-membered monocyclic aromatic heterocycles are fused, more preferably a heterocycle in which the 5- to 6-membered monocyclic aromatic heterocycle is fused with a benzene ring).

As the ring C, a monocyclic aromatic heterocycle and a benzene ring are preferable and, inter alia, a 5-membered monocyclic aromatic heterocycle such as pyrazole, imidazole, thiazole, oxazole, isoxazole, 1,2,4-oxadiazole, and 1,3,4-oxadiazole is preferable.

In addition, the ring C may be any of an aromatic ring having a hydrogen atom which can be deprotonated, and an aromatic ring not having a hydrogen atom which can be deprotonated, and an aromatic ring not having a hydrogen atom which can be deprotonated is preferable. Examples of the aromatic ring not having a hydrogen atom which can be deprotonated include, in addition to an aromatic ring originally not having a hydrogen atom which can be deprotonated (e.g. a benzene ring, thiazole, oxazole, isoxazole, 1,2,4-oxadiazole, 1,3,4-oxadiazole), an aromatic ring in which a hydrogen atom which can be deprotonated is substituted (e.g. pyrrole, pyrazole and imidazole in which a hydrogen atom on a ring constituting nitrogen atom is substituted, or which are bound to X^{1a} and/or X^{1b} via a ring constituting nitrogen atom).

Examples of the substituent which may be possessed by the aromatic ring in the "optionally further substituted aromatic ring" represented by the ring C include (i) a carboxyl group optionally esterified with an optionally halogenated C₁₋₆alkyl group or an optionally halogenated C₆₁₀aryl-C₁₋₄alkyl group (e.g. methyl, ethyl, propyl, isopropyl, butyl, tert-butyl, phenyl, benzyl, etc.), (ii) a phosphoric acid group optionally mono- or di-substituted with an optionally halogenated C₁₋₆alkyl (e.g. methyl, ethyl, n-propyl, isopropyl, n-butyl, isobutyl, n-pentyl, isopentyl, neopentyl, hexyl, etc.), or C₂₋₇alkanoyloxy-C₁₋₆alkyl such as an acetoxymethyl and a pivaloyloxymethyl group, (iii) a sulfonic acid group, (iv) a sulfonamide group optionally substituted with an optionally halogenated C₁₋₆alkyl group or an optionally halogenated C₆₋₁₀aryl-C₁₋₄alkyl group (e.g. methyl, ethyl, propyl, isopropyl, butyl, tert-butyl, benzyl, etc.), (v) a hydroxyl group and a sulfhydryl group optionally substituted with an optionally halogenated C₁₃alkyl group (e.g. methyl, ethyl, propyl, etc.), (vi) a carbamoyl group, (vii) a phenyl group which may be substituted with 1 to 5 substituents [e.g. a hydroxyl group, chlorine, fluorine, an aminosulfonyl group, an amino group optionally substituted with a C₁₋₃alkyl group (e.g. methyl, ethyl, propyl, etc.)], and which may be bound via O or S, (viii) an amino group optionally mono- or di-substituted with an optionally halogenated C₁₋₃alkyl group (e.g. methyl, ethyl, propyl, etc.), (ix) a cyclic amino group optionally substituted with 1 to 3 of C₁₋₃alkyl (e.g. methyl, ethyl, etc.), benzyl, phenyl and the like (e.g. a 5- to 6-membered cyclic amino group optionally containing an oxygen atom, or a sulfur atom as a ring constituting atom in addition to a nitrogen atom, such as a cyclic amino group derived (by removing one hydrogen atom) from a cyclic amine such as piperidine, pyrrolidine, morpholine, thiomorpholine, piperazine, 4-methylpiperazine, 4-benzylpiperazine, 4-phenylpiperazine, 1,2,3,4-tetrahydroisoquinoline, phthalimide and the like), (x) a 5- to 6-membered aromatic heterocyclic group which contains 1 to 4 heteroatoms selected from N, O and S, and which may be bound via O or S (e.g. pyridyl, imidazolyl, indolyl, tetrazolyl, etc.), (xi) a halogen atom (e.g. chlorine, fluorine, bromine, iodine), (xii) a C₁₋₄alkyl group (e.g. methyl, ethyl, propyl, isopropyl, butyl, tert-butyl, etc.), a C₁₋₄alkoxy group (e.g. methoxy, ethoxy, propoxy, isopropoxy, butoxy, tert-butoxy, etc.) or a C₁₋₄alkylthio group (e.g. methylthio, ethylthio, propylthio, isopropylthio, butylthio, tert-butylthio, etc.), each being optionally substituted with a substituent selected from a halogen atom, a C₁₋₄alkoxy group, a C₁₄alkylthio group, carboxyl and phenyl, (xiii) a C₅₇cycloalkyl group (e.g. cyclopentyl, cyclohexyl, cycloheptyl, etc.), (xiv) an optionally halogenated C₁₇alkanoyloxy (e.g. formyloxy, acetoxy, propionyloxy, butyryloxy, tert-butoxycarbonyloxy, isobutyryloxy, valeryloxy, pivaloyloxy etc). One to six, preferably one to three of these substituents can be present at replaceable positions. Alternatively, two substituents may be taken together to form C₃₋₆alkylene, C₃₋₆alkyleneoxy, C₃₋₆ alkylenedioxy or the like. For example, when two adjacent substituents on a phenyl group are taken together to form C₄alkylene, a tetrahydronaphthyl group is formed.

Examples of the lower alkyl group in the "lower alkyl group optionally substituted with an optionally substituted hydroxyl group" represented by R¹ include C₁₋₆alkyl such as methyl, ethyl, n-propyl, isopropyl, n-butyl, isobutyl, n-pentyl, isopentyl, neopentyl, hexyl and the like. Among them, a C₃₋₆alkyl group is preferable, and a C₄₋₅alkyl group is more preferable. Inter alia, a branched C₄₋₅alkyl group such as isobutyl, neopentyl and the like is preferable.

Examples of the substituent which may be possessed by the lower alkyl group in the "lower alkyl group optionally substituted with an optionally substituted hydroxyl group" represented by R¹ include a hydroxyl group optionally substituted with C₂₋₂₀alkanoyl or C₁₋₇alkyl. Examples of such substituents include, for example, a hydroxyl group, acetyloxy (acetoxy), propionyloxy, tert-butoxycarbonyloxy, palmitoyloxy, dimethylaminoacetyloxy, 2-aminopropionyloxy and the like. It may be substituted with 1 to 3 of such substituents at replaceable positions.

Examples of R¹ include 1-propyl, 1-isopropyl, 1-isobutyl, 1-neopentyl, 2,2-dimethyl-3-hydroxypropyl, 3-hydroxy-2-hydroxymethyl-2-methylpropyl, 3-acetoxy-2,2-dimethylpropyl, 3-acetoxy-2-hydroxymethyl-2-methyl-propyl, 3-acetoxy-2-acetoxymethyl-2-methylpropyl, [1-(hydroxymethyl)cyclobutyl]methyl and the like and, among them, 2,2-dimethyl-3-hydroxypropyl, 3-hydroxy-2-hydroxymethyl-2-methylpropyl, 3-acetoxy-2,2-dimethylpropyl, 3-acetoxy-2-hydroxymethyl-2-methyl-propyl, and 3-acetoxy-2-acetoxymethyl-2-methylpropyl are preferable.

Examples of the lower alkylene in the "optionally substituted lower alkylene" represented by X^{1a} include C₁₆alkylene such as methylene, dimethylene, trimethylene, tetramethylene, pentamethylene, hexamethylene and the like and, among them, straight C₁₋₄alkylene such as methylene, dimethylene, trimethylene, tetramethylene and the like is preferable, and straight C₁₋₃alkylene is more preferable.

As the substituent which may be possessed by the lower alkylene in the "optionally substituted lower alkylene" represented by X^{1a}, the same groups as the substituents which may be possessed by the aromatic ring in the "optionally further substituted aromatic ring" represented by the ring C as described above, and an oxo group are used, and 1 to 6, preferable 1 to 3 of such substituents can be present at replaceable positions.

As X^{1a}, a bond, and straight C₁₋₃alkylene are preferable, and methylene is particularly preferable.

Examples of the lower alkylene in the "optionally substituted lower alkylene" represented by X^{1b} include the same groups as examples of the lower alkylene in the "optionally substituted lower alkylene" represented by X^{1a} and, as the substituent which may be possessed by the lower alkylene in the "optionally substituted lower alkylene" represented by X^{1b}, the same number of the same groups as the substituents which may be possessed by the lower alkylene in the "optionally substituted lower alkylene" represented by X^{1a} are used.

As X^{1b}, a bond, and straight C₁₋₃alkylene are preferable, and a bond is particularly preferable.

As X², a bond is preferable.

Examples of the "divalent hydrocarbon group" in the "optionally substituted divalent hydrocarbon group" represented by X³ include a group formed by removing one hydrogen atom from a hydrocarbon group. Examples of the hydrocarbon group include a C₁₋₇ straight or branched alkyl group (e.g. methyl, ethyl, n-propyl, isopropyl, n-butyl, isobutyl, 1,1-dimethyethyl, n-pentyl, 3-methylbutyl, 2-methylbutyl, 1-methylbutyl, 1-ethylpropyl, n-hexyl, 4-methylpentyl, 3-methylpentyl, 2-methylpentyl, 2-ethylbutyl, 1-ethylbutyl, neopentyl, hexyl, heptyl), a C₃₋₇cycloalkyl group (cyclopropyl, cyclobutyl, cyclopentyl, cyclohexyl, cyclohexylmethyl, etc.), a C₂₋₆ straight or branched alkenyl group (e.g. vinyl, allyl, isopropenyl, 2-methylallyl, 1-propenyl, 2-methyl-1-propenyl, 2-methyl-2-propenyl, 1-butenyl, 2-butenyl, 3-butenyl, 2-ethyl-1-butenyl, 2-methyl-2-butenyl, 3-methyl-2-butenyl, 1-pentenyl, 2-pentenyl, 3-pentenyl, 4-pentenyl, 4-methyl-3-pentenyl, 1-hexenyl, 2-hexenyl, 3-hexenyl, 4-hexenyl, 5-hexenyl, etc.), a C₆₋₁₀aryl group (e.g. a phenyl or naphthyl group) and a C₇₋₁₄arylalkyl group (e.g. benzyl, phenethyl, naphthylmethyl) and the like.

As the substituent which may be possessed by the "divalent hydrocarbon group" in the "optionally substituted divalent hydrocarbon group" represented by X³, the same substituents as the substituents which may be possessed by the lower alkylene in the "optionally substituted lower alkylene" represented by X^{1a}, and optionally halogenated C₁₆alkylidene (e.g. methylidene, ethylidene, propylidene, isopropylidene, butenylidene), vinylidene, cyclohexylidene, benzylidene and the like are used, and 1 to 6, preferably 1 to 3 of these substituents can be present at replaceable positions.

As the "divalent hydrocarbon group" in the "optionally substituted divalent hydrocarbon group" represented by X³, (1) straight or branched alkylene in which a carbon number constituting a straight-chain portion is 1 to 7 (preferably 1 to 4) (e.g. methylene, ethylene, trimethylene, tetramethylene, pentamethylene, hexamethylene, heptamethylene, propylene, ethylmethylene, ethylethylene, propylethylene, butylethylene, methyltetramethylene, methyltrimethylene), (2) a carbon chain containing a double bond, in which a carbon number constituting a straight-chain portion is 2 to 7 (preferably 2 to 4) (e.g. vinylene, propenylene, butenylene, butadienylene, methylpropenylene, ethylpropenylene, propylpropenylene, methylbutenylene, ethylbutenylene, propylbutenylene, methylbutadienylene, ethylbutadienylene, propylbutadienylene, pentenylene, hexenylene, heptenylene, pentadienylene, hexadienylene, heptadienylene), (3) phenylene (e.g. 1,2-phenylene, 1,3-phenylene, 1,4-phenylene), and (4) a divalent group obtained by combining phenylene and alkylene and/or alkenylene (e.g. -CH₂-C₆H₄-, -CH₂CH₂-C₆H₄-, -CH₂-C₆H₄-CH₂-) are preferable.

As X³, C₁₋₄alkylene such as methylene, ethylene, trimethylene, tetramethylene and the like, vinylene, propenylene, and phenylene are preferable.

Examples of the "optionally esterified or amidated carboxyl group" represented by Y include carboxyl, lower alkoxycarbonyl of a carbon number of 2 to 7 (e.g. methoxycarbonyl, ethoxycarbonyl, propoxycarbonyl, isopropoxycarbonyl, butoxycarbonyl, tert-butoxycarbonyl, sec-butoxycarbonyl, pentyloxycarbonyl, isopentyloxycarbonyl, neopentyloxycarbonyl), C₇₋₁₄aryloxycarbonyl (e.g. phenoxycarbonyl, 1-naphthoxycarbonyl), C₈₁₂aralkyloxycarbonyl (e.g. benzyloxycarbonyl), carbamoyl, N-C₁₋₆alkylcarbamoyl, N,N-diC₁₋₆alkylcarbamoyl, N-C₈₁₂aralkylcarbamoyl, N,N-diC₈₋₁₂aralkylcarbamoyl, 1-pyrrolidinylcarbonyl, piperidinocarbonyl, and morpholinocarbonyl. Among them, as Y, carboxyl, methoxycarbonyl, ethoxycarbonyl and the like are preferable, and carboxyl is particularly preferable.

The compound represented by the formula (II), whether it is a free compound or a pharmacologically acceptable salt, is included in the present invention. When the compound represented by the formula (II) has an acidic group such as a carboxyl group and the like, the compound may form salts with inorganic bases (e.g. alkali metals such as sodium, potassium, etc., alkaline earth metals such as calcium, magnesium, etc., transition metals such as zinc, iron, copper, etc.), or organic bases (e.g. organic amines such as trimethylamine, triethylamine, pyridine, picoline, ethanolamine, diethanolamine, triethanolamine, tris(hydroxymethyl)methylamine, dicyclohexylamine, N,N'-dibenzylethylenediamine, and t-butylamine, and basic amino acids such as arginine, lysine, ornithine).

When the compound represented by the formula (ii) of the present invention has a basic group such as an amino group and the like, the compound may form salts with inorganic acids or organic acids (e.g. hydrochloric acid, nitric acid, sulfuric acid, phosphoric acid, carbonic acid, bicarbonic acid, formic acid, acetic acid, propionic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid, etc.), or acidic amino acids such as aspartic acid and glutamic acid.

In the compound represented by the formula (II) or a salt thereof, asymmetric carbons are present at 3- and 5-positions, the compound may be a mixture of stereoisomers, and the isomers may be separated by the known means. A trans-entity being an isomer in which substituents at a 3-position and a 5-position are oriented in a reverse direction of each other to the plane of a 7-membered ring is preferable and, particularly, the absolute configuration represented by the formula [IIa] is preferable. In addition, the compound represented by the formula (II) or a salt thereof may be any of a racemate and an optically active isomer, and the optically active isomer can be separated from a racemate by the known optional resolution means.

As the compound represented by the formula (II) of the present invention or a salt thereof, specifically, the following are particularly preferable:
(1) a compound in which X^{1b} is a bond, and Y is an optionally esterified carboxyl group;
(2) a compound in which a ring A is a benzene ring substituted with a halogen atom;
(3) a compound in which a ring B is a benzene ring substituted with a lower alkoxy group;
(4) a compound in which a ring C is an optionally further substituted monocyclic aromatic heterocycle;
(5) a compound in which a ring C is an optionally further substituted benzene ring;
(6) a compound in which a ring C is an optionally further substituted aromatic ring not having a hydrogen atom which can be deprotonated;
(7) a compound in which X^{1a} is C₁₋₃alkylene;
(8) a compound in which X² is a bond;
(9) a compound in which X³ is C₁₋₄alkylene;
(10) a compound in which the formula (II) is the formula (IIa) : 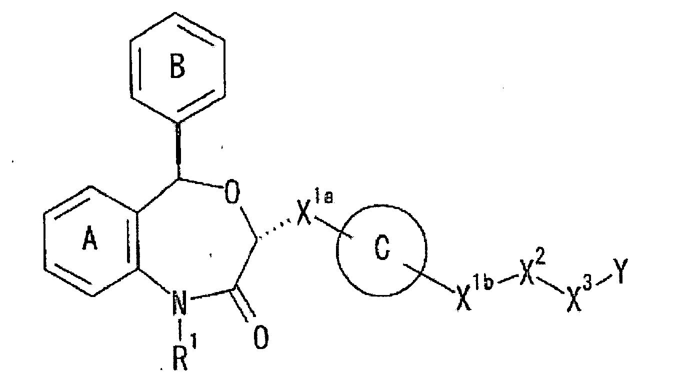 wherein each of symbols is as defined in the formula (II);
(11) 3-(2-{3-[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]propyl}-1,3-thiazol-5-yl)propionic acid, 3-(2-{2-[(3R,5S)-7-chloro-5-(2,3-dimethoxyphenyl)-1-(2,2-dimethylpropyl)-2-oxo-1,2,3,5-tetrahydro-4,1-benzoxazepin-3-yl]thyl}-1,3-thiazol-4-yl)propionic acid, or a salt thereof;
(12) (2-{[(3R,5S)-7-chloro-5-(2,3-dimethoxyphenyl)-1-(3-hydroxy-2,3-dimethylpropyl)-2-oxo-1,2,3,5-tetrahydro-4,1-benzoxazepin-3-yl]methyl}-1,3-oxazol-5-yl)propionic acid, (2-{[(3R,5S)-7-chloro-5-(2,3-dimethoxyphenyl)-1-isobutyl-2-oxo-1,2,3,5-tetrahydro-4,1-benzoxazepin-3-yl]methyl}-1,3-oxazol-5-yl)acetic acid, or a salt thereof;
(13) 5-(3-{[(3R,5S)-7-chloro-5-(2,3-dimethoxyphenyl)-1-(2,2-dimethylpropyl)-2-oxo-1,2,3,5-tetrahydro-4,1-benzoxazepin-3-yl]methyl}-1,2,4-oxadiazol-5-yl)pentanoic acid, 5-(3-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-1,2,4-oxadiazol-5-yl)pentanoic acid, 5-(3-{[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]methyl}-1,2,4-oxadiazol-5-yl)pentanoic acid, (4-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl}phenyl)acetic acid, or salts thereof.

The compound represented by the formula (II) can be produced by the method described, for example, in WO2005/012272 gazette.

The SSI compound represented by the formulas (I) and (II) used in the present invention has low toxicity (e.g. more excellent as a drug from a viewpoint of acute toxicity, chronic toxicity, genotoxicity, reproductive toxicity, cardiotoxicity, drug interaction, and carcinogenicity), and can be safely used as it is as a drug, or as a pharmaceutical composition by mixing with the known per se pharmaceutically acceptable carrier, in a mammal (e.g. human, monkey, cow, horse, pig, mouse, rat, hamster, rabbit, cat, dog, sheep, gout etc.),

For implementation of the present invention, use of the SSI compound represented by the formulas (Ib) and (II) is preferable.

In the agent of the present invention, a compound having an inhibitory effect on squalene synthase, or a prodrug thereof, or salt thereof which is an active ingredient (hereinafter, referred to as "SSI compound or prodrug thereof" in some cases) may be administered as bulk, but usually administered in a form prepared according to a conventional method using an appropriate amount of an appropriate carrier for formulation, for example, an excipient (e.g. calcium carbonate, kaolin, sodium hydrogencarbonate, lactose, starches, crystalline cellulose, talc, granulated sugar, porous substance etc.), a binder (e.g. dextrin, gums, alcoholized starch, gelatin, hydroxypropylcellulose, hydroxypropylmethylcellulose, pullulan etc.), a disintegrating agent (e.g. carboxymethylcellulose calcium, croscarmellose sodium, crospovidone, low-substituted hydroxypropylcellulose, partially pregelatinized starch etc.), a lubricant (e.g. magnesium stearate, calcium stearate, talc, starch, sodium benzoate etc.), a coloring agent (e.g. tar dye, caramel, iron sesquioxide, titanium oxide, riboflavin etc.), a corrigent (e.g. sweetener, perfume etc.), a stabilizer (e.g. sodium sulfite etc.) and a preservative (e.g. parabens, sorbic acid etc.). The agent of the present invention including the above preparation appropriately contains the SSI compound or a prodrug thereof at an effective amount for treating and preventing a disease. A content of the SSI compound or a prodrug thereof in the preparation of the present invention is usually 0.1 to 100% by weight of a whole preparation. In addition, the preparation used in the present invention may contain a pharmaceutical component other than the SSI compound or a prodrug thereof as an active ingredient, and the component is not particularly limited as far as an object of the present invention is attained, and the component can be used at an appropriate blending ratio. As specific examples of the dosage form, for example, tablets (including sugar-coated tablets, film-coated tablets), pills, capsules, granules, fine granules, powders, syrups, emulsions, suspensions, injectables, sustained-release injectables, inhalants, ointments and the like are used. These preparations are prepared according to a conventional method (e.g. the method described in Japanese Pharmacopoeia).

Specifically, for producing tablets, the SSI compound or a prodrug thereof, as it is, is mixed homogeneously after adding an excipient, a binder, a disintegrating agent or other additive, the mixture is converted into granules by an appropriate method, a lubricant or the like is added, and this is compression-molded. Alternatively, the SSI compound or a prodrug, as it is or after homogenously mixing following addition of an excipient, a binder, a disintegrating agent or other appropriate additive, is directly compression-molded. Alternatively, granules which have been prepared in advance, as it is or after homogenously mixing following addition of an appropriate additive, may be compression-molded. In addition, if necessary, a coloring agent, a corrigent and the like can be added to the agent of the present invention. Further, the agent of the present invention can be coated with an appropriate coating agent. For producing injectables, an amount of the SSI compound or a prodrug thereof is dissolved, suspended or emulsified in water for injection, a physiological saline, Ringer's solution or the like in the case of an aqueous solvent, or usually in a vegetable oil in the case of a non-aqueous solvent, to a given amount, or an amount of the SSI compound or a prodrug thereof is taken, and sealed into a container for injection, thereby, injectables can be prepared.

As a carrier for an oral preparation, substances which are usually used in the pharmacy field such as starch, mannitol, crystalline cellulose, carboxymethylcellulose sodium and the like are used. As the carrier for injection, for example, distilled water, a physiological saline, a glucose solution, an infusion or the like is used. Additionally, additives which are generally used in preparations may be appropriately added.

In addition, the preparation of the present invention can be also used as a sustained release preparation. As the sustained release preparation, a microcapsule (e.g. microsphere microcapsule, microparticle etc.) prepared, for example, by an drying-in-water method (o/w method, w/o/w method etc.), a phase separation method, a spray drying method or a similar method can be administered as it is, or after this microcapsule or a spherical, needle-like pellet-like, film-like or cream-like pharmaceutical composition as a raw material substance is formulated into various dosage forms. Examples of the dosage forms include parenteral preparations (e.g. intramuscular, subcutaneous, intraorgan injectables, or implant; transmucosal preparation into nasal cavity, rectum, uterine etc.), and oral preparations (e.g. hard capsules, soft capsules, granules, powders, suspensions etc.).

In the case where the sustained release preparation is an injectable, the microcapsule together with a dispersant (e.g. surfactant such as Tween 80, HCO-60 etc.; polysaccharides such as carboxymethylcellulose, sodium alginate, sodium hyaluronate etc.); protamine sulfate, polyethylene glycol etc.), a preservative (e.g. methylparaben, propylparaben etc.), an isotonic (e.g. sodium chloride, mannitol, sorbitol, glucose etc.), a local anesthetic (e.g. xylocaine hydrochloride, chlorobutanol etc.) and the like is formulated into an aqueous suspension. Alternatively, the microcapsule together with a vegetable oil (e.g. sesame oil, corn oil etc.) or the oil mixed with a phospholipid (e.g. lecithin etc.), or with a medium chain fatty acid triglyceride (e.g. Myglyol 812 etc.) is dispersed into an oily suspension, to obtain a sustained release injectable.

When the sustained release preparation is a microcapsule, an average particle diameter thereof is about 0.1 to about 300 µm, preferably about 1 to about 150 µm, further preferably about 2 to about 100 µm.

For formulating the microcapsule into a sterile preparation, there are a method of rendering all preparation steps sterile, a method of sterilizing with a gamma-ray, a method of adding an antiseptic and the like, being not limiting.

A dose of the agent of the present invention is different depending on an administration route, a symptom, an age or a weight of a patient and the like and, for example, when orally administered to an adult patient as a preventive or a remedy for arteriosclerosis, it is desirable that the agent is administered at a daily dose of 1 to 400 mg/day, preferably 6 to 120mg/day as expressed by the SSI compound, by dividing into one or several portions. An administration route may be either oral or parenteral.

In addition, a dose of the sustained release preparation as an example of the agent of the present invention varies depending on an administration route, a symptom, an age or a weight of a patient and the like, as well as a lasting time of release, but is not particularly limited as far as it is such an amount that an effective concentration of an active ingredient is retained in a body. An administration time can be appropriately selected depending on the circumstances, such as once every 1 to 3 days or every 1 week to 3 months.

The SSI compound can be used in combination with other drug and a surgical procedure. Therefore, the present invention also provides a joint use agent comprising a combination of the SSI compound and other drug.

Examples of a drug which can be used in combination with the SSI compound in the joint use agent of the present invention (hereinafter, abbreviated as combinantion drug in some cases) include a drug having the xanthoma treating effect other than the SSI compound, and a drug exhibiting the preventing or treating effect on any of various diseases which promote formation of xanthoma.

Examples of the drug having the xanthoma treating effect other than the SSI compound include a hyperlipemia treating drug such as a HMG-CoA reductase inhibitor (see, for example, US No.4,444,784 gazette), probucol, a fibrate drug, cholesterol absorption inhibitor (e.g. ezetimibe) and the like; a compound which is disclosed as the general formula (I) in US Application Publication 2003/0171251 gazette; a benzofuran derivative disclosed as the general formula (I) in US No.6,653,346 gazette; a nicotinic acid preparation; an anion exchange resin such as cholestyramin; and the like, being not limiting.

Examples of the drug having the xanthoma treating effect other than the SSI compound include a HMG-CoA reductase inhibitor (see, for example, US 4,444,784 gazette), a compound disclosed as the general formula (I) in US Application Publication 2003/0171251 gazette, a benzofuran derivative disclosed as the general formula (I) in US No.6,653,346 gazette, a fibrate drug, a nicotinic acid preparation, an anion exchange resin such as cholestyramin, cholesterol absorption inhibitor ezetimibe and the like, being not limiting.

On the other hand, as various diseases and factors of promoting xanthoma formation, autoimmune hyperlipemia, biliary obstructive hepatic disease, inflammatory disease, and extraneous stimulation (ultraviolet ray, physical load) are known, and examples of a drug exhibiting the preventing or treating effect on any one of them include anti-inflammatory drug, anti-rheumatoid drug, antibacterial drug, anti-fungal drug, anti-viral drug, anti-allergic drug, anti-angiopathic drug, anti-cancer drug, and bile acid preparation, being not limiting.

More specifically, examples of the anti-inflammatory drug include non-steroidal anti-inflammatory agent which is a cyclooxygenase (COX) inhibitor (e.g. salicylic acid drug such as various aspirins, anthranilic drug such as mefenamic acid, and flufenamic acid, indolacetic acid drug such as indometacin, sulindac, and acemetacin, phenylacetic acid drug such as diclofenac, and fenbufen, propionic drug such as ibuprofen, ketoprofen, roxoprofen, naproxen, and tiaprofen, oxicam drug such as piroxicam, tenoxicam, and ampiroxicam, pyrazolone drug such as ketophenylbutazone.), an anti-cytokine drug (e.g. anti-cytokine antibody such as anti-TNF-α antibody, and anti-IL-6 antibody, antisense oligonucleotide for cytokine gene, cytokine-binding protein etc.).

Examples of the anti-rheumatoid drug include gold preparation such as sodium aurothiomalate, and auranofin, penicillamine drug such as bucillamine and penicillamine, lobenzarit drug such as lobenzarit disodium, acritat, salazosulfapyridine, methotrexate, mizoribine, cyclosporine, azathioprine, cyclophosphamide, and prednisolone farnesylate.

Examples of the antibacterial agent include a penicillin antibiotic (e.g. sawacillin, pasetocin, yamacillin, bacacil, viccillin, pentrex etc), a cephem antibiotic (e.g. keflex, kefral, cefzon, tomiron, cefspan, pansporin etc.), a macrolide antibiotic (e.g. erythrocin, clarith, klaricid, rulid, josamycin etc). a tetracyclin antibiotic (e.g. minomycin, vibraromycin, hydramycin, ledermycin etc.), a phosphomycin antibiotic (e.g. fosmicin, eukocin etc.), an aminoglycoside antibiotic (e.g. kanamycin etc.), a new quinolone antibacterial agent (e.g. cravit, tarivid, baccidal, tosuxacin, ozex etc.). Examples of the anti-fungal drug include a polyene anti-fungal drug (e.g. trichomycin, amphotericin B, nystatin etc.), an imidazole anti-fungal drug (e.g., econazole, miconazole, clotrimazole etc.), a triazole anti-fungal drug (e.g. fluconazole, itraconazole, fluconzole etc.), an allylamine anti-fungal drug (e.g. butenafine, terbinafine hydrochloride etc.), a flucytosine (5-FC) anti-fungal drug (e.g. flucytosine etc.). Examples of the anti-viral drug include a nucleic acid synthesis inhabiting anti-viral drug (e.g. acyclovir, ganciclovir, vidarabine, foscarnet, zidovudine, lamivudine, didanosine etc.), an intracellular entry inhibiting anti-viral drug (e.g. amantadine, zanamivir, oseltamivir etc.), a host infection defending ability enhancing anti-viral drug (e.g. interferon, isoprinosine etc.).

Examples of the anti-allergic drug include an anti-histamic anti-allergic drug (e.g. ketotifen, azelastine, oxatomide, mequitazine, epinastine hydrochloride, terfenadine etc.), a non-anti-histamic anti-allergic drug (e.g. ozagrel hydrochloride, sodium cromoglicate, tranilast, repirinast, amlexanox etc.).

Examples of the anti-angiopathic drug include cilostazol, abciximab and the like.

Examples of the anti-cancer drug include a molecule targeting drug (e.g. trastuzumab, rituximab, imatinib, gefitinib etc.), an alkylating drug (e.g. cyclophosphamide, cisplastin, etc.), a metabolism antagonist (e.g. methotrexate, 6-mercaptopurine, 5-FU etc.), an antibiotic (e.g. bleomycin, adriamycin, actinomycin D etc.), a plant alkaloid (e.g. vincristine, vinblastine, paclitaxel etc.), and a hormone (e.g. prednisolone, tamoxifen etc.).

Examples of the bile acid preparation include chenodeoxycholic acid preparations, ursodeoxycholic acid preparation and the like.

An administration form of the SSI compound and the concomitant drug used in the present invention is not particularly limited. The SSI compound and the concomitant drug may be combined upon administration. Examples of such administration form include (1) administration of a single preparation obtained by formulating the SSI compound and the concomitant drug into a preparation simultaneously (so-cold combined preparation), (2) simultaneous administration of two kinds of preparations obtained by formulating the SSI compound and the concomitant drug into preparations separately through the same administration route, (3) administration of two kinds of preparations obtained by formulating the SSI compound and the concomitant drug into preparations separately through the same administration route with a time lag, (4) simultaneous administrations of two kinds of preparations obtained by formulating the SSI compound and the concomitant drug into preparations separately through different administration routes, and (5) administration of two kinds of preparations obtained by formulating the SSI compound and the concomitant drug into preparations separately through different administration routes with a time lag (e.g. administration in an order of SSI compound → concomitant drug, or administration in reverse order).

In the present invention, for examples, when referred to the "combination use of the SSI compound and the concomitant drug", combination use of both drugs in any form of the aforementioned administration forms is meant and, when referred to the "agent obtained by combining the SSI compound and the concomitant drug", an agent prepared for combination use of both drugs in any form of the aforementioned administration forms is meant.

In addition, upon combination use with the concomitant drug, it can be grasped that the SSI compound enhances the treating or preventing effect of other joint use drug on xanthoma.

A dose of the concomitant drug can be appropriately selected based on a dose which is clinically used. In addition, a ratio of blending the SSI compound and the concomitant drug can be appropriately selected depending on a kind of the concomitant drug, an administration subject, an administration route, a subject disease, a symptom, and a combination. For example, when the HMG-CoA reductase inhibitor is administered as the concomitant drug to a human, the SSI compound may be used at 0.01 to 100 parts by weight relative to 1 part by weight of the HMG-CoA reductase inhibitor.

Since the compound having the xanthoma improving effect exhibits the preventing or treating effect on damage of a tissue, or dysfunction as described above, the SSI compound, as it is or together with an additive such as an appropriate excipient and the like, can be used for preventing damage or degeneration of a skin, as a skin external agent other than a drug (quasi-drug, cosmetic etc.; hereinafter, simply abbreviated as "external agent of the present invention" in some cases).

The external agent of the present invention can take a form of aqueous solutions, oily solutions, other solutions, emulsions, creams, gels, suspensions, microcapsules, powders, granules or the like. After formulated into these forms by the known per se method, the external agent of the present invention can be applied, stuck or sprayed to a body, as lotion preparations, emulsion preparations, cream preparations, ointment preparations, plasters preparations, cataplasm preparations, aerosol preparations or the like.

Into the external agent of the present invention, in addition to excipients, perfumes and the like which are usually used, fats or oils, surfactants, antiseptics, metal ion sequesting agents, water-soluble polymers, thickening agents, powder components, ultraviolet-ray defending agents, humectants, other drug efficacy components, antioxidants, pH adjusting agents, detergents, drying agents, emulsifiers and the like can be appropriately blended.

Examples of the fats or oils include liquid fat or oil (e.g. avocado oil, camellia oil etc.), solid fat or oil (e.g. cacao butter, palm oil, horse butter, hardened palm oil etc.), waxes (e.g. beewax, candelilla wax, cotton wax, carnauba wax), hydrocarbon oil (e.g. liquid paraffin, paraffin etc.), higher fatty acid (e.g. lauric acid, myristic acid, palmitic acid, stearic acid, oleic acid, etc.), higher alcohol (e.g. linear alcohol such as lauryl alcohol etc.), branched alcohol such as monostearylglycerin ether (batyl alcohol ) etc.), synthetic ester oil (e.g. isopropyl myristate, cetyl octanotate etc.), silicones (e.g. chain-like polysiloxane such as dimethylpolysiloxane etc., cyclic polysiloxane such as decamethylpolysiloxane etc., silicone resin forming 3-dimentional network structure, silicone gum etc.).

Examples of the surfactant include anionic surfactant (e.g. sodium laurate, sodium lauryl sulfate, lauroylsarcosine sodium, hardened palm oil fatty acid glycerin sodium sulfate, turkey red oil etc.), cationic surfactant (e.g. stearyltrimethylammonium chloride, polyamine fatty acid derivative, amyl alcohol fatty acid derivative, benzalkonium chloride etc.), amphoteric surfactant (e.g. imdazolin amphoteric surfactant such as 2-undecyl-N,N,N-(hydroxyethylcarboxymethyl)-2-imidazolin sodium etc., betaine surfactant such as 2-heptadecyl-N-carboxymethyl-N-hydroxyethylimidazoliumbetaine etc.), noionic surfactant (e.g. sorbitan fatty acid esters such as sorbitan monooleate etc., glycerin polyglycerin fatty acids such as monocottonseed oil fatty acid glycerin etc., propylene glycol fatty acid esters such as monostearic acid propylene glycol etc., hardened castor oil derivative, polyoxyethylene-methylpolysiloxane copolymer etc.)

Examples of the antiseptic agent include methylparaben, ethylparaben, butylparaben and the like.

Examples of the methyl ion sequesting agent include sodium edatate salt, EDTA and the like.

Examples of the water-soluble polymer include natural polymers (e.g. plant polymers such as gum arabic, tragacanth gum, starch, glycyrrhizic acid etc., microorganism polymers such as xanthan gum, dextrin, pullulan etc., animal polymers such as collagen, casein, albumin, gelatin etc.), semisynthetic polymers (starch polymers such as dextrin, methylhydroxypropylstarch etc., cellulose polymers such as methylcellulose, nitrocellulose, methylhyroxypropylcellulose, hydroxypropylcellulose, carboxymethylcellulose sodium (CMC), crystalline cellulose etc., aliginate polymers such as sodium aliginate, alginic acid propylene glycol ester etc.), synthetic polymers (e.g. vinyl polymers such as polyvinyl alcohol, polyvinylpyrrolidone, carboxyvinyl polymer etc., polyoxyethylene polymers such as polyethylene glycol 2000, 4000, 6000 etc., polyoxyethylene polyoxypropylene copolymer polymers, acryl polymers such as polysodium acrylate, polyacrylamide etc., polyethyleneimine, cation polymer etc.), inorganic polymers (e.g. bentonite, magnesium aluminum silicate, anhydrous silicic acid etc.).

Examples of the powder component include inorganic powders such as talc, kaolin, mica, magnesium carbonate, aluminum silicate, metal tungstate, silica, zeolite, barium sulfate, calcinated calcium sulfate (calcinated terra alba), calcium phosphate, hydroxyapatite, metal soap (zinc myristate, calcium palmitate, aluminum stearate), boron nitride, etc., organic powders such as polyamide resin powder (nylon powder), polyethylene powder, styrene-acrylic acid copolymer resin powder, cellulose powder, etc., inorganic white pigments such as titanium dioxide, zinc oxide etc., inorganic red pigments such as iron oxide (colcothar), iron titanate, etc., inorganic brown pigments such as γ-iron oxide, etc., inorganic yellow pigments such as yellow iron oxide, etc., inorganic black pigments such as black iron oxide, etc., inorganic purple pigments such as Mango Violet, etc., inorganic green pigments such as chromium oxide, etc., inorganic blue pigments such as ultramarine, etc., pearlescent pigments such as titanium oxide-coated mica, etc., metal powder pigments such as aluminum powder, etc., organic pigments such as zirconium, barium or aluminum lake such as Red No. 201, Red No. 202, Orange No. 203, Orange No. 204, Yellow No. 205, Yellow No. 401, Blue No. 404, Red No. 3, Red No. 104, Orange No. 205, Yellow No. 4, Yellow No. 5, Green No. 3, and Blue No. 1, natural colorants such as chlorophyll, β-carotene, etc., coloring materials such as Titan Yellow, Safflower Red, etc.

Examples of the ultraviolet-defending agent include ultraviolet-absorbing agents which chemically absorb ultraviolet (long wavelength ultraviolet light (UVA) absorbing agents such as 4-methoxy-4'-tert-butyldibenzoylmethane, 2-hydroxy-4-methoxybenzophenone, 2-hydroxy-4-methoxybenzophenone derivatives, etc.; benzoic acid ultraviolet-absorbing agents such as paraaminobenzoic acid (PABA), etc., salicylic acid ultraviolet-absorbing agents such as dipropylene glycol salicylate, etc., cinnamic acid ultraviolet-absorbing agents such as octyl cinnamate, etc., medium wavelength ultraviolet light (UVB) absorbing agents such as camphor derivatives such as 3-(4'-methylbenzylidene)-d,l-camphor, etc.), and ultraviolet shielding agents which scatter and reflect ultraviolet light by the physical action (e.g. titanium oxide, talc, carmine, bentonite, kaolin, zinc oxide, etc.).

Examples of the moisturizer include polyethylene glycol, propylene glycol, glycerin, xylitol, sorbitol, maltitol, chondroitin sulfate, hyaluronic acid, mucoitinsulfuric acid, atelocollagen, cholesteryl-12-hydroxystearate, sodium lactate, bile salts, Chinquapin Rose extract, Yarrow extract and the like.

Examples of the other drugs efficacy component include skin-whitening agents such as arbutin, vitamin C and derivative thereof, kojic acid placenta extract, glutathione, saxifrage extract etc.; antiphlogistics such as glycyrrhizic acid derivatives, glycyrrhetic acid derivatives, salicylic acid derivatives, hinokitiol etc.; activators such as royal jelly, photosensitier, cholesterol derivatives etc., blood circulation promoting agents such as nonylic acid vanillylamide, nicotinic acid benzyl ester capsaicin, caffeine, tannic acid, nicotinic acid tocopherol, acetylcholine etc.; anti-seborrhea agents such as sulfur, thianthol etc.; for diverse purposes, phellodendri cortex extract, gold thread extract, lithospermum root extract, peony extract, swertia japonica extract, sage extract, Japanese Medlar extract, carrot extract, aloe extract, Loofah extract, lily extract, saffron extract, hypericum extract, ononis spinosa root extract, rosemary extract, garlic extract; vitamins such as vitamins A, vitamins B2, vitamins C, pantothenic acid, nicotinic acid, vitamins E, vitamin P, biotin, etc.

A amount of the SSI compound contained in the present external agent is not particularly limited as far as it is a sufficient amount to exert the effect of preventing or treating tissue damage and in such a range that a living body is not adversely effected, but, for example, the compound can be blended in a range from about 0.01 to about 20% by weight.

### Examples

The agent of the present invention can be produced, for example, by the following formulation.

In the following formulation, as components (additives) other than an active ingredient, products listed in Japanese Pharmacopoeia, Japanese Standards for Pharmaceuticals or Standards for Pharmaceutical Additives can be used.

### Preparation Example 1

### Capsule

(1) N-{[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl}piperidine-4-acetic acid

| | | |
|---|---|---|
| | | 10 mg |
| (2) | Lactose | 90 mg |
| (3) | Microcrystalline cellulose | 70 mg |
| (4) | Magnesium stearate | 10 mg |
| | One capsule | 180 mg |

(1), (2) and (3), and 1/2 of (4) are mixed, and granulated. To this is added the remaining amount of (4), and the whole is encapsulated into a gelatin capsule.

### Preparation Example 2

### Tablet

(1) N-{[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yllacetyllpiperidine-4-acetic acid

| | | |
|---|---|---|
| | | 10 mg |
| (2) | Lactose | 35 mg |
| (3) | Corn starch | 150 mg |
| (4) | Microcrystalline cellulose | 30 mg |
| (5) | Magnesium stearate | 5 mg |
| | One tablet | 230 mg |

(1), (2) and (3), 2/3 of (4), and 1/2 of (5) are mixed, and granulated. The remaining amounts of (4) and (5) are added to this granule, and it is pressure-molded into a tablet.

### Preparation Example 3

### Injectable

(1) N-{[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl}piperidine-4-acetic acid

| | | |
|---|---|---|
| | | 10 mg |
| (2) | Inosit | 100 mg |
| (3) | Benzyl alcohol | 20 mg |
| | One ampule | 130 mg |

(1), (2) and (3) are dissolved in distilled water for injection to a total amount of 2 ml, and the solution is sealed in an ampule. All steps are performed under the sterile conditions.

In order to demonstrate the "xanthomatosis-improving activity" of the present invention, the results of a pharmacological test regarding the "xanthomatosis-inhibiting activity in a rabbit model" using one example of the agent of the present invention are shown below. This Example is merely illustrative, and does not limit the scope of the present invention at all.

### Test compound 1: N-{[(3R, 5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl}acetyl}piperidine-4-acetic acid

The test compound 1 is a compound described as Example 36 in JP-A 9-136880 gazette, and can be synthesized by the method described in the gazette, or the like.

### Test Example 1 Xanthomatosis-inhibiting activity in rabbit model

### Method:

A vehicle or the test compound 1 was orally administered to a 2 months old male WHHLMI rabbit (10 to 11 animals/group) in an amount of 100, 200mg/kg in a mixed diet for 32 weeks. After the administration for 32 weeks, xanthoma was evaluated at the digital joints of forelegs and hind legs (Table 1). The extent of limb xanthoma was assessed by classifying into four stages: no occurrence (-), dotting of granule (+), scattering (++), mass (+++).

### Results:

**[Table 1]**

| Distribution of Severity | - | + | ++ | +++ | |
|---|---|---|---|---|---|
| Control | 2 | 8 | 26 | 4 | |
| Test compound 1 (100 mg/kg) | 4 | 13 | 27 | 0 | P=0.032 |
| Test compound 1 (200 mg/kg) | 0 | 29 | 15 | 0 | P<0.025 |

| | | | | | |
|---|---|---|---|---|---|
| *P<0.025 vs. the control values by one-tailed Shirley-Williams' test | | | | | |

### Industrial applicability

The present invention revealed that the SSI compound has the excellent xanthomatosis-improving activity. A pharmaceuticals comprising the compound according to the present invention is useful as an agent for preventing or treating xanthomatosis and an accompanying symptom, and has an extremely high utility value, for example, in the field of the pharmaceutical industry.

## Claims

1. An agent for preventing and/or treating xanthomatosis; or physical dysfunction or cosmetic disorder resulting from xanthoma formation, which comprises a compound having an inhibitory effect on squalene synthase, its prodrug or its salt.

2. The agent according to claim 1, which is a preventing and/or treating agent for xanthomatosis.

3. The agent according to claim 1, wherein the compound having an inhibitory effect on squalene synthase is a compound represented by the formula: 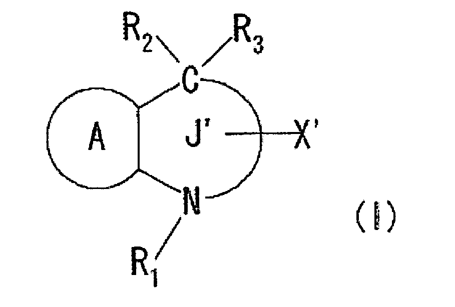 wherein R₁ represents a hydrogen atom or an optionally substituted hydrocarbon group; R₂ and R₃ are the same or different, and represent a hydrogen atom, an optionally substituted hydrocarbon group or an optionally substituted heterocyclic group; X' represents an optionally esterified carboxyl group, an optionally substituted carbamoyl group, an optionally substituted hydroxyl group, an optionally substituted amino group, or a group composed of an optionally substituted heterocyclic residue having a hydrogen atom which can be deprotonated; a ring A represents an optionally substituted benzene ring or an optionally substituted heterocycle; a ring J' represents a 7- or 8-membered heterocycle containing three or less heteroatoms as ring constituting atoms, and a ring J' may have a further substituent in addition to R₁, R₂, R₃ and X'.

4. The agent according to claim 1, wherein the compound having an inhibitory effect on squalene synthase is a compound represented by the formula: 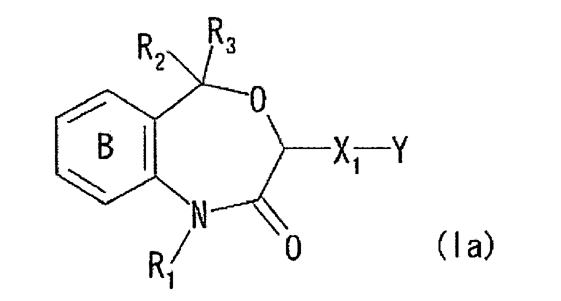 wherein R₁ represents an optionally substituted hydrocarbon group; R₂ and R₃ are the same or different, and represent a hydrogen atom or an optionally substituted hydrocarbon group; X₁ represents a divalent atom chain; Y represents an optionally substituted carbamoyl group; and a ring B represents an optionally substituted benzene ring.

5. The agent according to claim 1, wherein the compound having an inhibitory effect on squalene synthase is N-[[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl]piperidine-4-acetic acid.

6. The agent according to claim 1, wherein the compound having an inhibitory effect on squalene synthase is a compound represented by the formula: 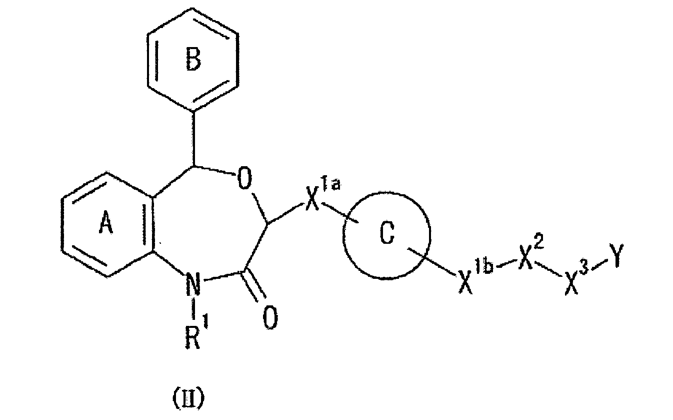 wherein a ring A and a ring B each represent an optionally substituted benzene ring; a ring C represents an optionally further substituted aromatic ring; R¹ represents a lower alkyl group optionally substituted with an optionally substituted hydroxyl group; X^{1a} represents a bond or optionally substituted lower alkylene; X^{1b} represents a bond or optionally substituted lower alkylene; X² represents a bond, -O- or -S-; X³ represents a bond or an optionally substituted divalent hydrocarbon group; and Y represents an optionally esterified or amidated carboxyl group.

7. The agent according to claim 1, wherein the compound having an inhibitory effect on squalene synthase is 3-(2-{3-[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]propyl}-1,3-thiazol-5-yl)propionic acid, 3-(2-{2-[(3R,5S)-7-chloro-5-(2,3-dimethoxyphenyl)-1-(2,2-dimethylpropyl)-2-oxo-1,2,3,5-tetrahydro-4,1-benzoxazepin-3-yl]ethyl}-1,3-thiazol-4-yl)propionic acid, (2-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-1,3-oxazol-5-yl)propionic acid, or (2-{[(3R,5S)-7-chloro-5-(2,3-dimethoxyphenyl)-1-isobutyl-2-oxo-1,2,3,5-tetrahydro-4,1-benzoxazepin-3-yl]methyl}-1,3-oxazol-5-yl)acetic acid.

8. The agent according to claim 1, wherein the compound having an inhibitory effect on squalene synthase is 5-(3-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]methyl}-1,2,4-oxadiazol-5-yl)pentanoic acid, 5-(3-{[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]methyl}-1,2,4-oxadiazol-5-yl)pentanoic acid, 5-(3-{[(3R,5S)-7-chloro-5-(2,3-dimethoxyphenyl)-1-(2,2-dimethylpropyl)-2-oxo-1,2,3,5-tetrahydro-4,1-benzoxazepin-3-yl]methyl}-1,2,4-oxadiazol-5-yl)pentanoic acid, or (4-{[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl}phenyl)acetic acid.

9. A method for preventing and/or treating xanthomatosis; or physical dysfunction or cosmetic disorder resulting from xanthoma formation, which comprises administering an effective amount of a compound having an inhibitory effect on squalene synthase, its prodrug or its salt to a mammal.

10. Use of a compound having an inhibitory effect on squalene synthase, its prodrug or its salt for production of a preventing and/or treating agent for xanthomatosis; or physical dysfunction or cosmetic disorder resulting from xanthoma formation.
